# EUROPEAN PATENT SPECIFICATION

(11) **EP 3 983 400 B1**
(45) Date of publication and mention of the grant of the patent: **28.05.2025**
(21) Application number: 20735729.4
(22) Date of filing: 11.06.2020
(51) Int. Cl.: C07D 401/12, C07D 401/04, C07D 239/84, C07D 401/14, C07D 405/12, A61K 31/517, A61P 35/00

(54) **QUINAZOLINYL COMPOUNDS AND METHODS OF USE**
CHINAZOLINYLINVERBINDUNGEN UND VERFAHREN ZUR VERWENDUNG
COMPOSÉS QUINAZOLINYLES ET PROCÉDÉS D'UTILISATION

(30) Priority: 11.06.2019 WO PCT/CN2019/090714
(43) Date of publication of application: 20.04.2022
(73) Proprietor: Genentech, Inc., South San Francisco, CA 94080 (US)
(72) Inventor: BRAUN, Marie-Gabrielle, South San Francisco, California 94080 (US); CASTANEDO, Georgette, South San Francisco, California 94080 (US); GIBBONS, Paul, South San Francisco, California 94080 (US); RUDOLPH, Joachim, South San Francisco, California 94080 (US); WU, Yao, Beijing 100176 (CN); WU, Guosheng, Beijing 100176 (CN)
(74) Representative: J A Kemp LLP
(86) International application number: PCT/US2020/037233
(87) International publication number: WO 2020/252165

(56) References cited:
- WO-A1-2018/102751
- WO-A1-2018/222917
- WO-A1-2018/222918
- WO-A1-2020/117635
- WO-A2-2006/039718
- US-A1- 2018 346 447

## Description

### FIELD OF THE INVENTION

Provided herein are compounds or pharmaceutically acceptable salts thereof, pharmaceutic compositions comprising them, and the compounds or salts thereof for use in methods for treating a cancer.

### BACKGROUND

The kinase/endoribonuclease inositol requiring enzyme 1 (IRE1α), one of the key sensors of misfolded protein accumulation in the endoplasmic reticulum that triggers the unfolded protein response (UPR), is a potential therapeutic target for diverse diseases including cancer for inhibitors that bind to the ATP-binding site on the kinase moiety of IRE1α and block its endoribonuclease activity. IRE1α is a transmembrane, bifunctional protein with a luminal domain that binds to misfolded proteins, a transmembrane segment, and a cytoplasmic portion consisting of a kinase moiety and a tandem endoribonuclease domain. Structure-activity relationship (SAR) studies led to compounds selective in recombinant IRE1α kinase screens and potent against endoribonuclease activity of recombinant IRE1α as well as cellular IRE1α. IRE1α activity mediates certain cytoprotective and pro-survival functions of the UPR, increases viability and growth in certain tumor cell lines, and can be an effective therapeutic target for specific small molecule inhibitors that block malignant tumor growth, contrary to an earlier report (Harrington, P.E. et al (2015) ACS Med. Chem. Lett. 6:68-72). In addition, inhibitors of IRE1α can be therapeutically useful for other types of diseases besides cancer including certain autoimmune, neurodegenerative, fibrotic and metabolic disorders (Wang M. and Kaufman, R.J. (2016) Nature 529:326-335).

Homeostatic regulation of protein folding in the endoplasmic reticulum (ER) is under the control of three key intracellular signaling pathways: IRE1α, PERK, and ATF6, which together orchestrate the unfolded protein response (UPR) (Schroder, et al (2005) Mutat Res-Fund Mol Mech Metagenesis 569:29 - 63). An increase in demand for protein folding in the ER or certain types of cellular injury or stress lead to the accumulation of unfolded proteins in the ER - a condition called ER stress. Cells respond to ER stress by activating the UPR to help adjust or maintain their high-fidelity protein synthetic capacity (Walter, P. and Ron, D. (2011) Science, 334:1081-1086). IRE1α is the most evolutionarily conserved of the three branches of the UPR. Importantly, the UPR makes life/ death decisions for the cell, depending on the severity and duration of ER stress, and the final outcome is either cell survival and recovery or programmed cell death (apoptosis) (Sovolyova et al, (2014) Biol Chem 395: 1-13). All three pathways of the UPR form a coordinated reaction to the accumulation of unfolded proteins; and several studies have demonstrated that there is cross talk between the different pathways (Yamamoto et al, J. Biochem. (2004) 136:343-350); Arai et al, FEBS Letts. (2006) 580:184-190; Adachi et al, Cell Struct. Func. (2008) 33:75-89). ER stress and activation of the UPR can be caused by mechanical injury, inflammation, genetic mutations, infections, oxidative stress, metabolic stress, and other types of cellular stress associated with malignancy. ER stress has also been implicated in diseases that result in fibrotic remodeling of internal organs, such as chronic liver diseases (Galligan et al, J. Toxicol. (2012) Vol. 2012, Article ID 207594, 12 pgs.; Shin et al, Cell Reports (2013) 5:654-665; Ji, Int. J. Hepatol. (2014) Vol. 2014, Article ID 513787, 11 pages), pulmonary fibrosis (Baek et al, Am. J. Resp. Cell Mol. Bio. (2012) 46:731-739); Tanjore et al, Biochim Biophys Acta (2012, online), (2013) 1832:940-947), kidney fibrosis (Chiang et al, Mol. Med. (2011) 17:1295-1305), cardiovascular disease (Spitler & Webb, Hypertension (2014) 63:e40-e45), and inflammatory bowel disease (Bogaert et al, PLoS One (2011) 6(10) e25589; Cao et al, Gastroent (2013) 144:989-1000).

Activation of the UPR has been shown to be an important survival pathway for tumors of secretory cell origin like multiple myeloma that have a very high protein synthesis burden. Therefore, efforts to disrupt the UPR by blocking the IRE1α endoribonuclease cleavage and activation of XBP1 have been an active area of cancer research. As a specific IRE1α RNase product, XBP1s is a direct indicator of functional IRE1 inhibition. A potent and selective IRE1α inhibitor would serve as an important tool to test the hypothesis that, without full UPR activation, tumor cells would be driven to apoptosis. IRE1α inhibitors and activating compounds have been reported (Harrington, P.E. et al (2015) ACS Med. Chem. Lett. 6:68-72; Volkmann, K., et al (2011) J. Biol. Chem., 286:12743-12755; Cross, B.C.S., et al (2012) Proc. Natl. Acad. Sci. U.S.A., 109:E869-E878; Wang, L., et al (2012) Nat. Chem. Biol., 8:982-989; Ghosh, R., et al (2014) Cell, 158:534-548; Ranatunga, S., et al (2014) J. Med. Chem., 57, 4289-4301; US 9382230; US 8815885).

Accordingly, there is a need for potent and selective inhibitors having suitable pharmacological properties for the treatment of IRE1-related diseases or disorders in patients.

WO 2018/222918, WO 2018/102751, WO 2018/222917, US 2018/346447, and WO 2020/117635 are each directed to small molecule IRE1 inhibitors. WO 2006/039718 is concerned with a class of compounds said to be useful for prophylaxis and treatment of protein kinase mediated diseases.

### SUMMARY

Provided herein are solutions to the problems above and other problems in the art.

The invention is set out in the appended set of claims. In addition, any reference to methods of treatment in the subsequent paragraphs of this description is to be interpreted as references to the compounds and pharmaceutical compositions of the present invention for use in a method for treatment of the human or animal body by therapy.

Disclosed herein are compounds of Formula (A) or a stereoisomer, tautomer, or pharmaceutically acceptable salt thereof as described herein, including pharmaceutical compositions of the same that are inhibitors of IRE1α. The compounds described herein are useful in treating diseases and disorders mediated by IRE1α, in particular cancer.

In one aspect provided herein are compounds having formula (A) as described herein.

In another aspect provided herein are compounds having formula (B) as described herein.

In another aspect provided herein are compounds having formula (D), (E), or (F) as described herein.

In one embodiment is a compound or pharmaceutically acceptable salt thereof of Table 1 or Table 2 as described herein (with the exception of compounds 628, 629, 630, 637, and 638, or pharmaceutically acceptable salts thereof).

In one aspect provided herein is a pharmaceutical composition comprising a compound or pharmaceutically acceptable salt thereof as described herein.

In still another aspect provided herein is a compound or pharmaceutically acceptable salt thereof or a pharmaceutical composition as described herein for use in a method for treating an IRE1-related disease or disorder, wherein the IRE1-related disease or disorder is cancer.

The present embodiments can be understood more fully by reference to the detailed description and examples, which are intended to exemplify non-limiting embodiments.

### DETAILED DESCRIPTION

Unless defined otherwise, all technical and scientific terms used herein have the same meaning as commonly understood by those of ordinary skill in the art to which the invention belongs. See, e.g., Singleton et al., DICTIONARY OF MICROBIOLOGY AND MOLECULAR BIOLOGY 2nd ed., J. Wiley & Sons (New York, NY 1994); Sambrook et al., MOLECULAR CLONING, A LABORATORY MANUAL, Cold Springs Harbor Press (Cold Springs Harbor, NY 1989). Any methods, devices and materials similar or equivalent to those described herein can be used in the practice of this invention.

The following definitions are provided to facilitate understanding of certain terms used frequently herein and are not meant to limit the scope of the present disclosure.

As used herein, and unless otherwise specified, the terms "about" and "approximately," when referring to doses, amounts, or weight percents of ingredients of a composition or a dosage form, mean a dose, amount, or weight percent that is recognized by one of ordinary skill in the art to provide a pharmacological effect equivalent to that obtained from the specified dose, amount, or weight percent. The equivalent dose, amount, or weight percent can be within 30%, 20%, 15%, 10%, 5%, 1%, or less of the specified dose, amount, or weight percent.

"Alkyl" as used herein refers to a saturated linear (i.e. unbranched) or branched univalent hydrocarbon chain or combination thereof, having the number of carbon atoms designated (*i.e.,* C₁₋₁₀ means one to ten carbon atoms). Particular alkyl groups are those having 1 to 20 carbon atoms (a "C₁₋₂₀ alkyl"), having a 1 to 8 carbon atoms (a "C₁₋₈ alkyl"), having 1 to 6 carbon atoms (a "C₁₋₆ alkyl"), having 2 to 6 carbon atoms (a "C₂₋₆ alkyl"), having 1 to 4 carbon atoms (a "C₁₋₄ alkyl") or having 1 to 3 carbon atoms (a "C₁₋₃ alkyl"). Examples of alkyl group include, but are not limited to, groups such as methyl, ethyl, n-propyl, isopropyl, n-butyl, t-butyl, isobutyl, sec-butyl, homologs and isomers of, for example, n-pentyl, n-hexyl, n-heptyl, n-octyl, and the like.

"Cycloalkyl" as used herein refers to non-aromatic, saturated or unsaturated cyclic univalent hydrocarbon structures having the number of carbon atoms designated (*i.e.,* (C₃₋₁₀ means three to ten carbon atoms). Cycloalkyl can consist of one ring, such as cyclohexyl, or multiple rings, such as adamantly, but excludes aryl groups. A cycloalkyl comprising more than one ring can be fused, spiro, or bridged, or combinations thereof. Particular cycloalkyl groups are those having from 3 to 7 annular carbon atoms (a "C₃₋₇ cycloalkyl"), or having 3 to 6 carbon atoms (a "C₃₋₆ cycloalkyl"). Examples of cycloalkyl include, but are not limited to, cyclopropyl, cyclobutyl, cyclopentyl, cyclohexyl, 1-cyclohexenyl, 3-cyclohexenyl, cycloheptyl, norbornyl, and the like.

"Heterocycloalkyl" as used herein refers to a cycloalkyl as defined herein where one or more of the ring carbon atoms have been replaced by a heteroatom such as, for example, nitrogen, oxygen, or sulfur. Representative examples of a heterocycloalkyl group include, but are not limited to, aziridinyl, azetidinyl, azepanyl, oxetanyl, pyrrolidyl, imidazolidinyl (*e.g*., imidazolidin-4-onyl or imidazolidin-2,4-dionyl), pyrazolidinyl, thiazolidinyl, tetrahydrofuranyl, dioxolyl, pyrrolinyl, imidazolinyl, pyrazolinyl, thiazolinyl, piperidyl, piperidinyl, piperazinyl, piperazin-2-onyl, morpholinyl, thiomorpholinyl, tetrahydropyranyl (*e.g*., tetrahydro-2H-pyranyl), tetrahydrothiopyranyl, oxathianyl, dioxyl, dithianyl, pyranyl, dihydrodithiinyl, 1,4-dioxaspiro[4.5]decanyl, homopiperazinyl, quinuclidyl, and tetrahydropyrimidin-2(1H)-one groups.

"Aryl" as used herein refers to an unsaturated aromatic carbocyclic group having a single ring (*e.g*., phenyl) or multiple condensed rings (e.g., naphthyl or anthryl) which condensed rings can or can not be aromatic. Particular aryl groups are those having from 6 to 14 annular (i.e., ring) carbon atoms (a "C₆₋₁₄ aryl"). Preferred aryl groups include those having 5 to 6 ring carbons. An aryl group having more than one ring where at least one ring is non-aromatic can be connected to the parent structure at either an aromatic ring position or at a non-aromatic ring position. In one variation, an aryl group having more than one ring where at least one ring is non-aromatic is connected to the parent structure at an aromatic ring position.

"Heteroaryl" as used herein refers to an unsaturated aromatic cyclic group having from 1 to 14 annular (i.e., ring) carbon atoms and at least one annular heteroatom, including but not limited to heteroatoms such as nitrogen, phosphorus, oxygen and sulfur. A heteroaryl group can have a single ring (e.g., pyridyl, furyl) or multiple condensed rings (e.g., indolizinyl, benzothienyl) which condensed rings can or can not be aromatic. Particular heteroaryl groups are 5- to 14-membered rings having 1 to 12 annular (i.e., ring) carbon atoms and 1 to 6 annular (i.e., ring) heteroatoms independently selected from nitrogen, phosphorus, oxygen and sulfur; 5- to 10-membered rings having 1 to 8 annular carbon atoms and 1 to 4 annular heteroatoms independently selected from nitrogen, phosphorus, oxygen and sulfur; and 5-, 6- or 7-membered rings having 1 to 5 annular carbon atoms and 1 to 4 annular heteroatoms independently selected from nitrogen, oxygen and sulfur In one variation, heteroaryl include monocyclic aromatic 5-, 6- or 7-membered rings having from 1 to 6 annular carbon atoms and 1 to 4 annular heteroatoms independently selected from nitrogen, oxygen and sulfur. In another variation, heteroaryl includes polycyclic aromatic rings having from 1 to 12 annular carbon atoms and 1 to 6 annular heteroatoms independently selected from nitrogen, phosphorus, oxygen and sulfur. Still further, a heteroaryl as described herein can include rings have 5 or 6 members. In preferred embodiments herein, the heteroatoms are independently selected from nitrogen and oxygen. A heteroaryl group having more than one ring where at least one ring is non-aromatic can be connected to the parent structure at either an aromatic ring position or at a non-aromatic ring position. In one variation, a heteroaryl group having more than one ring where at least one ring is non-aromatic is connected to the parent structure at an aromatic ring position.

"Halo" or Halogen" refers to fluoro, chloro, bromo and/or iodo. Where a residue is substituted with more than one halogen, it can be referred to by using a prefix corresponding to the number of halogen moieties attached, e.g., dihaloaryl, dihaloalkyl, trihaloaryl etc. refer to aryl and alkyl substituted with two ("di") or three ("tri") halo groups, which can be but are not necessarily the same halo; thus 4-chloro-3-fluorophenyl is within the scope of dihaloaryl.

An alkyl group in which one or more hydrogen is replaced with a halo group is referred to as a "haloalkyl", for example, "C₁₋₆ haloalkyl." An exemplary haloalkyl group is trifluoroalkyl (-CF₃). Similarly, "haloalkoxy" refers to an alkoxy group in which a halogen takes the place of one or more hydrogens in the hydrocarbon making up the alkyl moiety of the alkoxy group. An exemplary haloalkoxy group is trifluoromethoxy (-OCF₃).

"Carbonyl" refers to the group C=O.

"Oxo" refers to the moiety =O.

The term "chiral" refers to molecules which have the property of non-superimposability of the mirror image partner, while the term "achiral" refers to molecules which are superimposable on their mirror image partner.

The term "stereoisomers" refers to compounds which have identical chemical constitution, but differ with regard to the arrangement of the atoms or groups in space.

"Diastereomer" refers to a stereoisomer with two or more centers of chirality and whose molecules are not mirror images of one another. Diastereomers have different physical properties, e.g. melting points, boiling points, spectral properties, and reactivities. Mixtures of diastereomers can separate under high resolution analytical procedures such as electrophoresis and chromatography.

"Enantiomers" refer to two stereoisomers of a compound which are non-superimposable mirror images of one another.

Stereochemical definitions and conventions used herein generally follow S. P. Parker, Ed., McGraw-Hill Dictionary of Chemical Terms (1984) McGraw-Hill Book Company, New York; and Eliel, E. and Wilen, S., "Stereochemistry of Organic Compounds", John Wiley & Sons, Inc., New York, 1994. The compounds or pharmaceutically acceptable salts thereof as described herein can contain asymmetric or chiral centers, and therefore exist in different stereoisomeric forms. It is intended that all stereoisomeric forms of the compounds or pharmaceutically acceptable salts thereof as described herein, including but not limited to, diastereomers, enantiomers and atropisomers, as well as mixtures thereof such as racemic mixtures, are included herein. Many organic compounds exist in optically active forms, i.e., they have the ability to rotate the plane of plane-polarized light. In describing an optically active compound, the prefixes D and L, or R and S, are used to denote the absolute configuration of the molecule about its chiral center(s). The prefixes d and 1 or (+) and (-) are employed to designate the sign of rotation of plane-polarized light by the compound, with (-) or 1 meaning that the compound is levorotatory. A compound prefixed with (+) or d is dextrorotatory. For a given chemical structure, these stereoisomers are identical except that they are mirror images of one another. A specific stereoisomer can also be referred to as an enantiomer, and a mixture of such isomers is often called an enantiomeric mixture. A 50:50 mixture of enantiomers is referred to as a racemic mixture or a racemate, which can occur where there has been no stereoselection or stereospecificity in a chemical reaction or process. The terms "racemic mixture" and "racemate" refer to an equimolar mixture of two enantiomeric species, devoid of optical activity. Enantiomers can be separated from a racemic mixture by a chiral separation method, such as supercritical fluid chromatography (SFC). Assignment of configuration at chiral centers in separated stereoisomers can be tentative, and depicted in Table 1 structures for illustrative purposes, before stereochemistry is definitively established, such as from x-ray crystallographic data.

The term "tautomer" or "tautomeric form" refers to structural isomers of different energies which are interconvertible via a low energy barrier. For example, proton tautomers (also known as prototropic tautomers) include interconversions via migration of a proton, such as keto-enol and imine-enamine isomerizations. Valence tautomers include interconversions by reorganization of some of the bonding electrons.

A "solvate" refers to an association or complex of one or more solvent molecules and a compound or pharmaceutically acceptable salt thereof as described herein. Examples of solvents that form solvates include, but are not limited to, water (i.e., "hydrate"), isopropanol, ethanol, methanol, DMSO, ethylacetate (EtOAc), acetic acid (AcOH), and ethanolamine.

The term "administering" refers to the act of delivering a compound, pharmaceutically acceptable salt thereof, or pharmaceutical composition described herein into a patient by such routes as, for example, oral, mucosal, topical, suppository, intravenous, parenteral, intraperitoneal, intramuscular, intralesional, intrathecal, intranasal or subcutaneous administration. Parenteral administration includes intravenous, intramuscular, intra-arterial, intradermal, subcutaneous, intraperitoneal, intraventricular, and intracranial administration. Administration generally occurs after the onset of the cancer described herein, or its symptoms but. The term includes administering a chemotherapeutic agent or a therapy as described herein.

The term "coadministration" refers to administration of two or more agents (*e.g.,* a compound, pharmaceutically acceptable salt thereof, or pharmaceutical composition with another active agent such as a chemotherapeutic agent described herein). The timing of coadministration depends in part of the compositions administered and can include administration at the same time, just prior to, or just after the administration of one or more additional therapies, for example cancer therapies such as chemotherapy, hormonal therapy, radiotherapy, or immunotherapy. Compounds, pharmaceutically acceptable salts thereof, and pharmaceutical compositions described herein may be administered alone or may be coadministered to the patient. Coadministration is meant to include simultaneous or sequential administration of the compound individually or in combination (more than one compound or agent). Thus, the preparations can also be combined, when desired, with other active substances (*e.g*., to reduce metabolic degradation). The compounds described herein may be used in combination with one another, with other active agents known to be useful in treating a cancer described herein or associated with cells expressing a particular kinase as described herein, or with adjunctive agents that cannot be effective alone, but can contribute to the efficacy of the active agent.

A "1L therapy" refers to the first line therapy administered to a treatment naïve cancer patient. Likewise, a 2L, 3L, and the like refer to subsequent therapies administered to a patient.

As used herein, and unless otherwise specified, the terms "about" and "approximately," when referring to doses, amounts, or weight percents of ingredients of a composition or a dosage form, mean a dose, amount, or weight percent that is recognized by one of ordinary skill in the art to provide a pharmacological effect equivalent to that obtained from the specified dose, amount, or weight percent. The equivalent dose, amount, or weight percent can be within 30%, 20%, 15%, 10%, 5%, 1%, or less of the specified dose, amount, or weight percent.

"Metastatic" refers to cancer that has spread to tissues beyond the local tissue and regional lymph nodes. "Locally Advanced" refers to cancer that has spread from the immediate tissue only to surrounding tissue.

The term "clinical response" refers to inhibition of disease progression, inhibition of tumor growth, reduction of primary tumor, relief of tumor-related symptoms, inhibition of tumor secreted factors (including tumor secreted hormones, such as those that contribute to carcinoid syndrome), delayed appearance of primary or secondary tumors, slowed development of primary or secondary tumors, decreased occurrence of primary or secondary tumors, slowed or decreased severity of secondary effects of disease, arrested tumor growth and regression of tumors, increased Time To Progression (TTP), increased Progression Free Survival (PFS), increased Overall Survival (OS), among others. OS as used herein means the time from treatment onset until death from any cause. In general, clinical response refers to primary or secondary measures of efficacy known and understood in the art. Treatment and clinical response as described herein can be assessed using international standards for a given condition.

"Overall survival" or "OS" refers to the time from enrollment to death from any cause.

"Objective response rate" or "ORR" refers the proportion of patients with a confirmed complete response or partial response on two consecutive occasions ≥ 4 weeks apart, as determined by the investigator according to RECIST v1.1

"Time to progression" or "TTP" refers to the time from randomization until objective tumor progression.

"Duration of response" or "DOR" refers to the time from the first occurrence of a documented objective response to disease progression, as determined by the investigator according to RECIST v1.1, or death from any cause, whichever occurs first.

"Progression free survival" or "PFS" refers to the time from enrollment to the date of the first recorded occurrence of disease progression, as determined by the investigator using RECIST v1.1 or death from any cause, whichever occurs first.

"Clinical benefit rate" or "CBR" refers to the proportion of patients with stable disease for at least 24 weeks or with confirmed complete or partial response, as determined by the investigator according to RECIST v1.1.

"Complete response" or "CR" refers to the disappearance of all target lesions and non-target lesions and (if applicable) normalization of tumor marker level.

"Partial response" or "non-CR/Non-PD" refers to persistence of one or more non-target lesions and/or (if applicable) maintenance of tumor marker level above the normal limits. A PR can also refer to ≥ 30% decrease in sum of diameters of target lesions, in the absence of CR, new lesions, and unequivocal progression in non-target lesions.

"Progressive disease" or "PD" refers to ≥ 20% increase in sum of diameters of target lesions, unequivocal progression in non-target lesions, and/or appearance of new lesions.

"Stable disease" or "SD" refers to neither sufficient shrinkage to qualify for CR or PR nor sufficient increase growth of tumor to qualify for PD.

The term "treatment" refers to clinical intervention designed to alter the natural course of the patient or cell being treated during the course of clinical pathology. Desirable effects of treatment include decreasing the rate of disease progression, ameliorating or palliating the disease state, and remission or improved prognosis. For example, a patient is "treated" if one or more symptoms associated with the disease described herein are mitigated or eliminated, including, but are not limited to, reducing the proliferation of (or destroying) cancerous cells, decreasing symptoms resulting from the disease, increasing the quality of life of those suffering from the disease, decreasing the dose of other medications required to treat the disease, and/or prolonging survival of patients. In certain embodiments, treatment can refer to a measured clinical outcome (e.g. increased OS, ORR, TTP, DOR, PFS, CBR, PR, CR, or SD).

The term "delaying progression" of a disease refers to deferring, hindering, slowing, retarding, stabilizing, and/or postponing development of a disease described herein. This delay can be of varying lengths of time, depending on the history of the cancer and/or patient being treated. As is evident to one skilled in the art, a sufficient or significant delay can, in effect, encompass prevention, in that the patient does not develop cancer.

An "effective amount" is at least the minimum amount required to effect a measurable improvement or prevention of a disease described herein. An effective amount herein may vary according to factors such as the disease state, age, sex, and weight of the patient, and the ability of the agent to elicit a desired response in the patient. An effective amount is also one in which any toxic or detrimental effects of the treatment are outweighed by the therapeutically beneficial effects. Beneficial or desired results include results such as eliminating or reducing the risk, lessening the severity, delaying the onset of the disease (including biochemical, histological and/or behavioral symptoms of the disease, its complications and intermediate pathological phenotypes presenting during development of the disease), decreasing one or more symptoms resulting from the disease, increasing the quality of life of those suffering from the disease, decreasing the dose of other medications required to treat the disease, enhancing effect of another medication such as via targeting, delaying the progression of the disease, and/or prolonging survival. In some embodiments, an effective amount of the drug may have the effect in reducing the number of cancer cells; reducing the tumor size; inhibiting (i.e., slow or stop) cancer cell infiltration into peripheral organs; inhibit (i.e., slow or stop) tumor metastasis; inhibiting (i.e., slow or stop) tumor growth; and/or relieving one or more of the symptoms associated with the disorder. An effective amount can be administered in one or more administrations. An effective amount of drug, compound, pharmaceutical composition, or combination therapy described herein can be an amount sufficient to accomplish therapeutic treatment either directly or indirectly. As is understood in the clinical context, an effective amount of a drug, compound, or pharmaceutical composition may or may not be achieved in conjunction with another drug, compound, or pharmaceutical composition, or combination therapy. An "effective amount" may be considered in the context of administering one or more therapeutic agents, and a single agent may be considered to be given in an effective amount if, in conjunction with one or more other agents, a desirable result may be or is achieved.

An "administration period" or "cycle" refers to a period of time comprising administration of a compound or pharmaceutically acceptable salt thereof described herein and an optional period of time comprising no administration of the compound or pharmaceutically acceptable salt thereof described herein. For example, a cycle can be 28 days in total length and include administration for 21 days and a rest period of 7 days. A "rest period" refers to a period of time where the compound or pharmaceutically acceptable salt thereof described herein is not administered. A rest period as provided herein can in some instances include administration of another agent that is not compound or pharmaceutically acceptable salt thereof described herein (e.g. an anticancer agent described herein). In such instances, administration of another agent during a rest period should not interfere or detriment administration of a compound or pharmaceutically acceptable salt thereof described herein.

A "dosing regimen" refers to a period of administration of a compound or pharmaceutically acceptable salt thereof described herein comprising one or more cycles, where each cycle can include administration of the compound or pharmaceutically acceptable salt thereof described herein at different times or in different amounts.

"QD" refers to administration of a compound or pharmaceutically acceptable salt thereof described herein once daily.

"BID", "TID", "and "QID" refer to administration of a compound or pharmaceutically acceptable salt thereof described herein 2, 3, and 4 times daily.

QW refers to administration of a compound or pharmaceutically acceptable salt thereof described herein once weekly.

"Q2W", "Q3W", and "Q4W" refer to administration of a compound or pharmaceutically acceptable salt thereof described herein once every 2, 3, and 4 weeks, respectively.

The terms "cancer" refers to or describe the physiological condition in mammals that is typically characterized by unregulated cell growth. A "tumor" comprises one or more cancerous cells. Examples of cancer include, but are not limited to, carcinoma, lymphoma, blastoma, sarcoma, and leukemia or lymphoid malignancies. More particular examples of such cancers include squamous cell cancer (*e.g*., epithelial squamous cell cancer), lung cancer including small- cell lung cancer, non-small cell lung cancer ("NSCLC"), small-cell lung cancer, non-small cell lung cancer (NSCLC), lung adenocarcinoma, squamous cell lung cancer, peritoneum cancer, hepatocellular cancer, stomach cancer, gastrointestinal cancer, esophageal cancer, pancreatic cancer, glioblastoma, cervical cancer, ovarian cancer, liver cancer, bladder cancer, breast cancer, colon cancer, rectal cancer, colorectal cancer, endometrial cancer, uterine cancer, salivary gland carcinoma, renal cancer, prostate cancer, vulval cancer, thyroid cancer, hepatocellular carcinoma (HCC), anal carcinoma, penile carcinoma, or head and neck cancer.

"Hematological malignancies" (British spelling "Haematological" malignancies) are the types of cancer that affect blood, bone marrow, and lymph nodes. As the three are intimately connected through the immune system, a disease affecting one of the three will often affect the others as well: although lymphoma is a disease of the lymph nodes, it often spreads to the bone marrow, affecting the blood. Hematological malignancies are malignant neoplasms (i.e. cancer), and they are generally treated by specialists in hematology and/or oncology. Hematological malignancies can derive from either of the two major blood cell lineages: myeloid and lymphoid cell lines. Lymphomas, lymphocytic leukemias, and myeloma are from the lymphoid line, while acute and chronic myelogenous leukemia, myelodysplastic syndromes and myeloproliferative diseases are myeloid in origin. Exemplary leukemias include acute lymphoblastic leukemia (ALL), acute myelogenous leukemia (AML), chronic lymphocytic leukemia (CLL), chronic myelogenous leukemia (CML), acute monocytic leukemia (AMOL) and small lymphocytic lymphoma (SLL). Exemplary lymphomas include Hodgkin's lymphomas (all four subtypes) and Non-Hodgkin's lymphomas (NHL, all subtypes).

An "IRE1-related disease" and the like refer to a disease described herein (e.g. a cancer described herein) having symptoms or requiring treatment as set forth herein that is/are wholly or partly associated with, a result of, a function of, or otherwise correlated to IRE1 activity as described herein. In the compound, salt, or composition for use of the present invention, the IRE1-related disease or disorder is cancer.

A "anti-cancer agent" is a chemical compound useful in the treatment of cancer, regardless of mechanism of action. Classes of anti-cancer agents include, but are not limited to: alkylating agents, antimetabolites, anti-hormone therapies, endocrine therapies, immunomodulatory agents, spindle poison plant alkaloids, cytotoxic/antitumor antibiotics, topoisomerase inhibitors, antibodies, photosensitizers, and kinase inhibitors. Anti-cancer agents include compounds used in targeted therapy and conventional chemotherapy.

Examplary anti-cancer agents include proteasome inhibitors such as bortezomib (VELCADE), carfilzomib (KYPROLIS) and ixazomib (NINLARO). Other examples include immunomodulatory agents such as lenalidomide (REVLIMID) and pomalidomide (POMALYST).

Other exemplary anti-cancer agents include inhibitors of B-cell receptor targets such as BTK, Bcl-2 and JAK inhibitors and include, for example, venetoclax (VENCLEXTA) and ibrutinib (IMBRUVICA).

Additional anti-cancer agents include, for example, Abemaciclib (VERZENIO); abiraterone (ZYTIGA, YONSA); aclarubicin; acivicin; acodazole; acronine; actinomycin; acylfulvene; adecypenol; adozelesin; adriamycin; aldesleukin; altretamine; ambamustine; ambomycin; ametantrone; amidox; amifostine; aminoglutethimide; aminolevulinic acid; amrubicin; amsacrine; anagrelide; anastrozole; andrographolide; antarelix; anthramycin; aphidicolin glycinate; apurinic acid; ARRY-300; arabinoside; asperlin; asulacrine; atamestane; atrimustine; azasetron; azatoxin; azatyrosine; azacitidine; AZD6244; AZD8330; azetepa; azotomycin; balanol; batimastat; bendamustine; benzochlorins; benzodopa; benzoylstaurosporine; beta-alethine; betaclamycin B; betulinic acid; bicalutamide; binimetinib; bisantrene; bisaziridinylspermine; bisnafide; bistratene; bleomycin; busulfan; bizelesin; breflate; bortezomib; brequinar; bropirimine; budotitane; buthionine; bryostatin; cactinomycin; calusterone; calcipotriol; calphostin C; camptothecin; capecitabine (XELODA); caracemide; carbetimer; carboplatin; carboquone; carmustine; carubicin; carzelesin; castanospermine; celecoxib; cetrorelix; cetuximab (ERBITUX); chloroquinoxaline; cicaprost; chlorambucil; chlorofusin; cisplatin; cladribine; clomifene; clotrimazole; crisnatol; crisnatol; cypemycin; cyclophosphamide; cytarabine; cytostatin; dacarbazine; dactinomycin; daratumamab; daunorubicin; decarbazine; dacliximab; dasatinib; decitabine; deslorelin; dexamethasone; dexifosfamide; dexrazoxane; dexverapamil; dexormaplatin; dezaguanine; diaziquone; dihydrotaxol; docosanol; dolasetron; docetaxel; doxorubicin; doxifluridine; droloxifene; dromostanolone; dronabinol; duazomycin; ebselen; ecomustine; edelfosine; edrecolomab; edatrexate; eflornithine; elemene; emitefur; elsamitrucin; enloplatin; enpromate; epipropidine; epirubicin; epristeride; erbulozole; erlotinib (TARCEVA); esorubicin; estramustine; etanidazole; etoposide; etoprine; exemestane; fadrozole; fazarabine; fenretinide; filgrastim; finasteride; flavopiridol; flezelastine; fluasterone; floxuridine; fludarabine; fludarabine; fluorodaunorubicin; forfenimex; formestane; fluorouracil; floxouridine; flurocitabine; fosquidone; fostriecin; fotemustine; fulvestrant (FASLODEX); gadolinium; gallium; galocitabine; ganirelix; gemcitabine; geldanamycin; gefitinib; gossyphol; hydroxyurea; hepsulfam; heregulin; ibandronate; ibrutinib; idarubicin; idelalisib (ZYDELIG), ifosfamide; canfosfamide; ilmofosine; iproplatin; idoxifene; idramantone; ilmofosine; ilomastat; imatinib mesylate (GLEEVEC); imiquimod; iobenguane; iododoxorubicin; ipomeanol; irinotecan; itasetron; iimofosine; lanreotide; lapatinib (TYKERB); leinamycin; lenograstim; lentinan; leptolstatin; letrozole; leuprorelin; levamisole; liarozole; lobaplatin; lombricine; lometrexol; lonidamine; lonafarnib (SARASAR); losoxantrone; lovastatin; loxoribine; lurtotecan; lapatinib; leucovorin; lometrexol; lomustine; maitansine; marimastat; masoprocol; maspin; menogaril; merbarone; meterelin; methioninase; metoclopramide; mifepristone; miltefosine; mirimostim; mitoguazone; mitolactol; mitonafide; mitoxantrone; mofarotene; molgramostim; mopidamol; maytansine; megestrol acetate; melengestrol acetate; melphalan; mercaptopurine; methotrexate; methotrexate sodium; metoprine; meturedepa; mitinmitomycin; mitosper; mitotane; mitoxantrone; mycophenolic acid; nafarelin; nagrestip; napavin; nedaplatin; nemorubicin; neridronic acid; nilutamide; nisamycin; oblimersen (GENASENSE); octreotide; okicenone; onapristone; ondansetron; ormaplatin; oxisuran; oxaloplatin; osaterone; oxaliplatin; oxaunomycin; palauamine; palbociclib (IBRANCE); panitumumab (VECTIBIX); panomifene; pegaspargase; picibanil; pirarubicin; piritrexim; prednisone; prednisolone, paclitaxel; nab-paclitaxel (ABRAXANE); prednimustine; procarbazine; puromycin; raltitrexed; ramosetron; rapamycin (RAPAMUNE); rhizoxin; ribociclib (KISQALI), rituximab; rogletimide; rohitukine; romurtide; roquinimex; romidepsin; safingol; saintopin; sargramostim; semustine; sizofiran; sobuzoxane; sorafenib (NEXAVAR); sunitinib; spiromustine; squalamine; suradista; suramin; swainsonine; spiroplatin; streptonigrin; streptozocin; sulofenur; tallimustine; tamoxifen; tauromustine; tazarotene; tellurapyrylium; temoporfin; temozolomide; teniposide; tetrachlorodecaoxide; tetrazomine; thrombopoietin; thymalfasin; thymotrinan; tirapazamine; toremifene; tretinoin; trimetrexate; triptorelin; tropisetron; talisomycin; taxotere; teroxirone; testolactone; thiamiprine; thiotepa; tirapazamine; toremifene; trastuzumab; trastuzumab emtansine; trestolone acetate; triciribine phosphate; trimetrexate; uracil mustard; vandetanib (CAPRELSA); variolin B; velaresol; veramine; verteporfin; vemurafenib; vinorelbine; vinxaltine; vitaxin; vinblastine; vincristine; vindesine; vinepidine; vinglycinate; vinleurosine; vinorelbine; vinrosidine; vinzolidine; vorozole; wortmannin; zanoterone; zeniplatin; zilascorb; zinostatin stimalamer; zinostatin; and zorubicin.

In some embodiments, an anti-cancer agent includes, for example, idelalisib (ZYDELIG), docetaxel, fluorouracil, gemcitabine (GEMZAR), cisplatin, cis-diamine, carboplatin, paclitaxel, nab-paclitaxel, trastuzumab (HERCEPTIN), temozolomide, tamoxifen, 4-hydroxytamoxifen, and doxorubicin.

Also included in the definition of anti-cancer agent are: (i) anti-estrogens and selective estrogen receptor modulators (SERMs), including, for example, tamoxifen, raloxifene, droloxifene, 4-hydroxytamoxifen, trioxifene, ketoxifene, LY117018, onapristone, and toremifine citrate; (ii) selective estrogen receptor modulators (SERDs) such as brilanestrant, GDC-0927, GDC-9545, AZ9496, AZ9833, GNE-274, and fulvestrant (FASLODEX); (iii) aromatase inhibitors such as, for example, 4(5)-imidazoles, aminoglutethimide, megestrol acetate, exemestane, formestanie, fadrozole, vorozole, letrozole, and anastrozole; (iv) anti-androgens such as apalutamide, abiraterone, enzalutamide, flutamide, nilutamide, bicalutamide, leuprolide, and goserelin.

Further included in the definition of anti-cancer agents are: (iv) MEK inhibitors such as cobimetinib; (v) lipid kinase inhibitors, such as taselisib; (vi) antisense oligonucleotides such as oblimersen; (vii) ribozymes such as VEGF expression inhibitors such as angiozyme; (viii) vaccines such as gene therapy vaccines, for example, ALLOVECTIN, LEUVECTIN, and VAXID; (ix) topoisomerase 1 inhibitors such as LURTOTECAN; ABARELIX rmRH; and (x) anti-angiogenic agents such as bevacizumab.

In some embodiments herein, the anti-cancer agent is a therapeutic antibody such as atezolizumab, nivolumab, daratumumab, pembrolizumab, alemtuzumab, bevacizumab; cetuximab; panitumumab, rituximab, pertuzumab, trastuzumab, trastuzumab emtansine, or tositumomab.

A "metabolite" is a product produced through metabolism in the body of a specified compound or salt thereof. Metabolites of a compound can be identified using routine techniques and their activities determined using tests such as those described herein. Such products can result for example from the oxidation, reduction, hydrolysis, amidation, deamidation, esterification, deesterification, enzymatic cleavage, and the like, of the administered compound. Accordingly, disclosed herein are metabolites of compounds or pharmaceutically acceptable salts thereof described herein, including compounds produced by a process comprising contacting a compound of pharmaceutically acceptable salt thereof described herein with a mammal for a period of time sufficient to yield a metabolic product thereof.

The term "package insert" is used to refer to instructions customarily included in commercial packages of therapeutic products, that contain information about the indications, usage, dosage, administration, contraindications and/or warnings concerning the use of such therapeutic products.

The term "pharmaceutically acceptable salts" denotes salts which are not biologically or otherwise undesirable. Pharmaceutically acceptable salts include both acid and base addition salts. The phrase "pharmaceutically acceptable" indicates that the substance or composition must be compatible chemically and/or toxicologically, with the other ingredients comprising a formulation, and/or the mammal being treated therewith.

The term "pharmaceutically acceptable acid addition salt" denotes those pharmaceutically acceptable salts formed with inorganic acids such as hydrochloric acid, hydrobromic acid, sulfuric acid, nitric acid, carbonic acid, phosphoric acid, and organic acids selected from aliphatic, cycloaliphatic, aromatic, aryl-aliphatic, heterocyclic, carboxylic, and sulfonic classes of organic acids such as formic acid, acetic acid, propionic acid, glycolic acid, gluconic acid, lactic acid, pyruvic acid, oxalic acid, malic acid, maleic acid, malonic acid, succinic acid, fumaric acid, tartaric acid, citric acid, aspartic acid, ascorbic acid, glutamic acid, anthranilic acid, benzoic acid, cinnamic acid, mandelic acid, embonic acid, phenylacetic acid, methanesulfonic acid "mesylate", ethanesulfonic acid, p-toluenesulfonic acid, and salicyclic acid.

The term "pharmaceutically acceptable base addition salt" denotes those pharmaceutically acceptable salts formed with an organic or inorganic base. Examples of acceptable inorganic bases include sodium, potassium, ammonium, calcium, magnesium, iron, zinc, copper, manganese, and aluminum salts. Salts derived from pharmaceutically acceptable organic nontoxic bases includes salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, 2-diethylaminoethanol, trimethamine, dicyclohexylamine, lysine, arginine, histidine, caffeine, procaine, hydrabamine, choline, betaine, ethylenediamine, glucosamine, methylglucamine, theobromine, purines, piperazine, piperidine, N-ethylpiperidine, and polyamine resins.

The term "EC₅₀" is the half maximal effective concentration" and denotes the plasma concentration of a particular compound required for obtaining 50% of the maximum of a particular effect in vivo.

The term "Ki" is the inhibition constant and denotes the absolute binding affinity of a particular inhibitor to a receptor. It is measured using competition binding assays and is equal to the concentration where the particular inhibitor would occupy 50% of the receptors if no competing ligand (e.g. a radioligand) was present. Ki values can be converted logarithmically to pKi values (-log Ki), in which higher values indicate exponentially greater potency.

The term "IC₅₀" is the half maximal inhibitory concentration and denotes the concentration of a particular compound required for obtaining 50% inhibition of a biological process in vitro. IC₅₀ values can be converted logarithmically to pIC₅₀ values (-log IC₅₀), in which higher values indicate exponentially greater potency. The IC₅₀ value is not an absolute value but depends on experimental conditions e.g. concentrations employed, and can be converted to an absolute inhibition constant (Ki) using the Cheng-Prusoff equation (Biochem. Pharmacol. (1973) 22:3099). Other percent inhibition parameters, such as IC₇₀, IC₉₀, etc., can be calculated.

A graded adverse event refers to the severity grading scale as established for by NCI CTCAE. In one embodiment, the adverse event is graded in accordance with the table below.

| Grade | Severity |
|---|---|
| 1 | Mild; asymptomatic or mild symptoms; clinical or diagnostic observations only; or intervention not indicated |
| 2 | Moderate; minimal, local, or non-invasive intervention indicated; or limiting age-appropriate instrumental activities of daily living^{a} |
| 3 | Severe or medically significant, but not immediately life-threatening; hospitalization or prolongation of hospitalization indicated; disabling; or limiting self-care activities of daily living ^{b, c} |
| 4 | Life-threatening consequences or urgent intervention indicated ^{d} |
| 5 | Death related to adverse event ^{d} |

Any formula or structure given herein, including those described herein, is intended to represent unlabeled forms as well as isotopically labeled forms of the compounds. Isotopically labeled compounds have structures depicted by the formulas given herein except that one or more atoms are replaced by an atom having a selected atomic mass or mass number. Examples of isotopes that can be incorporated into compounds or pharmaceutically acceptable salts thereof as described herein include isotopes of hydrogen, carbon, nitrogen, oxygen, phosphorous, fluorine, and chlorine, such as, but not limited to ²H (deuterium, D), ³H (tritium), ¹¹C, ¹³C, ¹⁴C, ¹⁵N, ¹⁸F, ³¹P, ³²P, ³⁵S, ³⁶Cl, and ¹²⁵I. Various isotopically labeled compounds or pharmaceutically acceptable salts thereof as described herein, for example those into which radioactive isotopes such as ³H and ¹⁴C are incorporated. Such isotopically labeled compounds can be useful in metabolic studies, reaction kinetic studies, detection or imaging techniques, such as positron emission tomography (PET) or single-photon emission computed tomography (SPECT) including drug or substrate tissue distribution assays, or in radioactive treatment of patients. Deuterium labeled or substituted therapeutic compounds or pharmaceutically acceptable salts thereof as described herein can have improved DMPK (drug metabolism and pharmacokinetics) properties, relating to distribution, metabolism, and excretion (ADME). Substitution with heavier isotopes such as deuterium can afford certain therapeutic advantages resulting from greater metabolic stability, for example increased in vivo half-life or reduced dosage requirements. An 18F labeled compound can be useful for PET or SPECT studies. Isotopically labeled compounds or pharmaceutically acceptable salts thereof as described herein can generally be prepared by carrying out the procedures disclosed in the schemes or in the examples and preparations described below by substituting a readily available isotopically labeled reagent for a non-isotopically labeled reagent. Further, substitution with heavier isotopes, particularly deuterium (i.e., ²H or D) can afford certain therapeutic advantages resulting from greater metabolic stability, for example increased in vivo half-life or reduced dosage requirements or an improvement in therapeutic index. It is understood that deuterium in this context is regarded as a substituent in the compound of the formula (A). The concentration of such a heavier isotope, specifically deuterium, can be defined by an isotopic enrichment factor. In the compounds or pharmaceutically acceptable salts thereof as described herein any atom not specifically designated as a particular isotope is meant to represent any stable isotope of that atom. Unless otherwise stated, when a position is designated specifically as "H" or "hydrogen", the position is understood to have hydrogen at its natural abundance isotopic composition. Accordingly, in the compounds or pharmaceutically acceptable salts thereof as described herein any atom specifically designated as a deuterium (D) is meant to represent deuterium.

### Compounds

Provided herein are compounds having the formula: 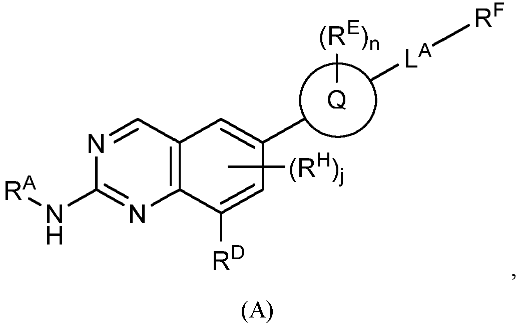 or a stereoisomer, tautomer, or pharmaceutically acceptable salt thereof, wherein:
Ring Q is R^{E}-substituted or unsubstituted C₅₋₇ aryl, or R^{E}-substituted or unsubstituted 6 membered heteroaryl comprising at least one nitrogen heteroatom;
L^{A} is -NHSO₂-, -SO₂NH-, or -NH-;
R^{A} is 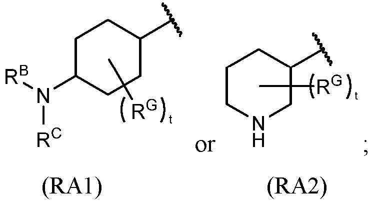
R^{B} and R^{C} are independently hydrogen or R^{N}-substituted or unsubstituted C₁₋₆ alkyl, R^{N}-substituted or unsubstituted C₃₋₇ cycloalkyl, or R^{N}-substituted or unsubstituted 3 to 7 membered heterocycloalkyl, or R^{B} and R^{C} are taken together with the nitrogen atom to which they are attached to form a R^{N}-substituted or unsubstituted 4 to 7 membered heterocycloalkyl;
R^{D} is hydrogen, halogen, -CN, -OR^{I}, -S(O)₂R^{I}, -NR^{J}R^{K}, R^{N}-substituted or unsubstituted C₁₋₆ alkyl, R^{N}-substituted or unsubstituted C₁₋₆ haloalkyl, R^{N}-substituted or unsubstituted C₃₋₇ cycloalkyl, R^{N}-substituted or unsubstituted 3 to 7 membered heterocycloalkyl, R^{N}-substituted or unsubstituted C₅₋₇ aryl, or R^{N}-substituted or unsubstituted 5 to 7 membered heteroaryl;
each R^{E} is hydrogen, halogen, -OR^{I}, -CN, -S(O)₂R^{I}, R^{M}-substituted or unsubstituted C₁₋₆ alkyl, R^{M}-substituted or unsubstituted C₁₋₆ haloalkyl, or R^{M}-substituted or unsubstituted C₃₋₇ cycloalkyl;
n is 0, 1, 2, 3, or 4;
R^{F} is R^{N}-substituted or unsubstituted C₁₋₆ alkyl, R^{N}-substituted or unsubstituted C₃₋₇ cycloalkyl, R^{N}-substituted or unsubstituted benzyl, or R^{N}-substituted or unsubstituted 3 to 7 membered heterocycloalkyl;
each R^{G} is independently halogen, -OR^{I}, R^{M}-substituted or unsubstituted C₁₋₆ alkyl, or R^{M}-substituted or unsubstituted C₁₋₆ haloalkyl;
t is 0, 1, 2, 3, 4, 5, or 6;
each R^{H} is independently halogen, C₁₋₃ alkyl, C₁₋₃ haloalkyl, or cyclopropyl;
j is 0, 1, or 2;
each R^{I}, R^{J}, and R^{K} is independently hydrogen, R^{M}-substituted or unsubstituted C₁₋₆ alkyl, R^{M}-substituted or unsubstituted C₁₋₆ haloalkyl, R^{M}-substituted or unsubstituted C₃₋₇ cycloalkyl, R^{M}-substituted or unsubstituted 3 to 7 membered heterocycloalkyl, R^{M}-substituted or unsubstituted C₅₋₇ membered aryl, or R^{M}-substituted or unsubstituted 5 to 7 membered heteroaryl;
each R^{M} is independently hydrogen, halogen, -CN, -OH, -OCH₃, -OCF₃, -S(O)₂H, - S(O)₂NH₂, -NH₂, -NH(CH₃), -N(CH₃)₂, -CF₃, -CHF₂, -CH₂F, -C(CH₃)₂F, -C(CH₃)F₂, unsubstituted C₁₋₆ alkoxy, unsubstituted C₁₋₆ alkyl, unsubstituted C₁₋₆ haloalkyl, unsubstituted C₃₋₇ cycloalkyl, unsubstituted 3 to 6 membered heterocycloalkyl, unsubstituted C₅₋₇ aryl, or unsubstituted 5 to 7 membered heteroaryl;
each R^{N} is independently hydrogen, halogen, -CN, -OR^{R}, -S(O)₂R^{R}, -NR^{S}R^{T}, R^{P}-substituted or unsubstituted C₁₋₆ alkyl, R^{P}-substituted or unsubstituted C₁₋₆ haloalkyl, R^{P}-substituted or unsubstituted C₁₋₆ alkoxy, R^{P}-substituted or unsubstituted C₃₋₇ cycloalkyl, R^{P}-substituted or unsubstituted 3 to 6 membered heterocycloalkyl, R^{P}-substituted or unsubstituted C₅₋₇ aryl, or R^{P}-substituted or unsubstituted 5 to 7 membered heteroaryl;
each R^{R}, R^{S} and R^{T} is independently hydrogen, R^{V}-substituted or unsubstituted C₁₋₆ alkyl, R^{V}-substituted or unsubstituted C₁₋₆ haloalkyl, R^{V}-substituted or unsubstituted C₃₋₇ cycloalkyl, or R^{V}-substituted or unsubstituted 3 to 7 membered heterocycloalkyl;
each R^{V} is independently hydrogen, halogen, -CN, -OH, -OCH₃, -OCF₃, -S(O)₂H, - S(O)₂NH₂, -NH₂, -NH(CH₃), -N(CH₃)₂, -CF₃, -CHF₂, -CH₂F, -C(CH₃)₂F, -C(CH₃)F₂, unsubstituted C₁₋₆ alkoxy, unsubstituted C₁₋₆ alkyl, unsubstituted C₁₋₆ haloalkyl, unsubstituted C₃₋₇ cycloalkyl, unsubstituted 3 to 6 membered heterocycloalkyl, unsubstituted C₅₋₇ aryl, or unsubstituted 5 to 7 membered heteroaryl;
each R^{P} is independently hydrogen, halogen, -CN, -OR^{W}, -S(O)₂R^{W}, -NR^{X}R^{Y}, R^{U}-substituted or unsubstituted C₁₋₆ alkyl, R^{U}-substituted or unsubstituted C₁₋₆ haloalkyl, R^{U}-substituted or unsubstituted C₃₋₇ cycloalkyl, R^{U}-substituted or unsubstituted 3 to 6 membered heterocycloalkyl, R^{U}-substituted or unsubstituted C₅₋₇ aryl, or R^{U}-substituted or unsubstituted 5 to 7 membered heteroaryl;
each R^{W}, R^{X} and R^{Y} is independently hydrogen, unsubstituted C₁₋₆ alkyl, unsubstituted C₁₋₆ haloalkyl, unsubstituted C₃₋₇ cycloalkyl, or unsubstituted 3 to 7 membered heterocycloalkyl, and
each R^{U} is independently hydrogen, halogen, -CN, -OH, -OCH₃, -OCF₃, -S(O)₂H, - S(O)₂NH₂, -NH₂, -NH(CH₃), -N(CH₃)₂, -CF₃, -CHF₂, -CH₂F, -C(CH₃)₂F, -C(CH₃)F₂, unsubstituted C₁₋₆ alkoxy, unsubstituted C₁₋₆ alkyl, unsubstituted C₁₋₆ haloalkyl, unsubstituted C₃₋₇ cycloalkyl, unsubstituted 3 to 6 membered heterocycloalkyl, unsubstituted C₅₋₇ aryl, or unsubstituted 5 to 7 membered heteroaryl;
wherein cycloalkyl refers to non-aromatic, saturated or unsaturated cyclic univalent hydrocarbon structures, which can consist of one ring or multiple rings, wherein a cycloalkyl comprising more than one ring can be fused, spiro, or bridged, or combinations thereof; and
wherein heterocycloalkyl refers to non-aromatic, saturated or unsaturated cyclic univalent hydrocarbon structures where one or more of the ring carbon atoms have been replaced by a heteroatom, which can consist of one ring or multiple rings, wherein a heterocycloalkyl comprising more than one ring can be fused, spiro, or bridged, or combinations thereof.

In certain embodiments, provided herein are compounds having the formula: 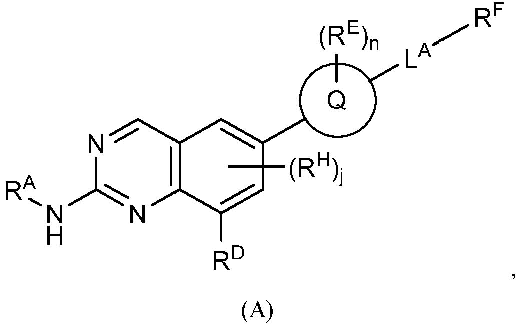 or a stereoisomer, tautomer, or pharmaceutically acceptable salt thereof, wherein:
Ring Q is R^{E}-substituted or unsubstituted phenyl, or R^{E}-substituted or unsubstituted 6 membered heteroaryl comprising at least one nitrogen heteroatom;
L^{A} is -NHSO₂-, -SO₂NH-, or -NH-;
R^{A} is 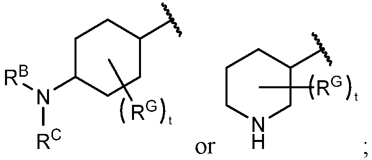
R^{B} and R^{C} are independently hydrogen or R^{N}-substituted or unsubstituted C₁₋₆ alkyl, R^{N}-substituted or unsubstituted C₃₋₇ cycloalkyl, or R^{N}-substituted or unsubstituted 3 to 7 membered heterocycloalkyl, or R^{B} and R^{C} are taken together with the nitrogen atom to which they are attached to form a R^{N}-substituted or unsubstituted 4 to 7 membered heterocycloalkyl;
R^{D} is hydrogen, halogen, -CN, -OR^{I}, -S(O)₂R^{I}, -NR^{J}R^{K}, R^{N}-substituted or unsubstituted C₁₋₆ alkyl, R^{N}-substituted or unsubstituted C₁₋₆ haloalkyl, R^{N}-substituted or unsubstituted C₃₋₇ cycloalkyl, R^{N}-substituted or unsubstituted 3 to 7 membered heterocycloalkyl, R^{N}-substituted or unsubstituted C₅₋₇ aryl, or R^{N}-substituted or unsubstituted 5 to 7 membered heteroaryl;
each R^{E} is hydrogen, halogen, -OR^{I}, -CN, -S(O)₂R^{I}, R^{M}-substituted or unsubstituted C₁₋₆ alkyl, R^{M}-substituted or unsubstituted C₁₋₆ haloalkyl, or R^{M}-substituted or unsubstituted C₃₋₇ cycloalkyl;
n is 0, 1, 2, 3, or 4;
R^{F} is R^{N}-substituted or unsubstituted C₁₋₆ alkyl, R^{N}-substituted or unsubstituted C₃₋₇ cycloalkyl, or R^{N}-substituted or unsubstituted 3 to 7 membered heterocycloalkyl;
each R^{G} is independently halogen, -OR^{I}, R^{M}-substituted or unsubstituted C₁₋₆ alkyl, or R^{M}-substituted or unsubstituted C₁₋₆ haloalkyl;
t is 0, 1, 2, 3, 4, 5, or 6;
each R^{H} is independently halogen, C₁₋₃ alkyl, C₁₋₃ haloalkyl, or cyclopropyl;
j is 0, 1, or 2;
each R^{I}, R^{J}, and R^{K} is independently hydrogen, R^{M}-substituted or unsubstituted C₁₋₆ alkyl, R^{M}-substituted or unsubstituted C₁₋₆ haloalkyl, R^{M}-substituted or unsubstituted C₃₋₇ cycloalkyl, R^{M}-substituted or unsubstituted 3 to 7 membered heterocycloalkyl, R^{M}-substituted or unsubstituted C₅₋₇ membered aryl, or R^{M}-substituted or unsubstituted 5 to 7 membered heteroaryl;
each R^{M} is independently hydrogen, halogen, -CN, -OH, -OCH₃, -OCF₃, -S(O)₂H, - S(O)₂NH₂, -NH₂, -NH(CH₃), -N(CH₃)₂, -CF₃, -CHF₂, -CH₂F, -C(CH₃)₂F, -C(CH₃)F₂, unsubstituted C₁₋₆ alkoxy, unsubstituted C₁₋₆ alkyl, unsubstituted C₁₋₆ haloalkyl, unsubstituted C₃₋₇ cycloalkyl, unsubstituted 3 to 6 membered heterocycloalkyl, unsubstituted C₅₋₇ aryl, or unsubstituted 5 to 7 membered heteroaryl;
each R^{N} is independently hydrogen, halogen, -CN, -OR^{R}, -S(O)₂R^{R}, -NR^{S}R^{T}, R^{P}-substituted or unsubstituted C₁₋₆ alkyl, R^{P}-substituted or unsubstituted C₁₋₆ haloalkyl, R^{P}-substituted or unsubstituted C₃₋₇ cycloalkyl, R^{P}-substituted or unsubstituted 3 to 6 membered heterocycloalkyl, R^{P}-substituted or unsubstituted C₅₋₇ aryl, or R^{P}-substituted or unsubstituted 5 to 7 membered heteroaryl;
each R^{R}, R^{S} and R^{T} is independently hydrogen, R^{V}-substituted or unsubstituted C₁₋₆ alkyl, R^{V}-substituted or unsubstituted C₁₋₆ haloalkyl, R^{V}-substituted or unsubstituted C₃₋₇ cycloalkyl, or R^{V}-substituted or unsubstituted 3 to 7 membered heterocycloalkyl;
each R^{V} is independently hydrogen, halogen, -CN, -OH, -OCH₃, -OCF₃, -S(O)₂H, - S(O)₂NH₂, -NH₂, -NH(CH₃), -N(CH₃)₂, -CF₃, -CHF₂, -CH₂F, -C(CH₃)₂F, -C(CH₃)F₂, unsubstituted C₁₋₆ alkoxy, unsubstituted C₁₋₆ alkyl, unsubstituted C₁₋₆ haloalkyl, unsubstituted C₃₋₇ cycloalkyl, unsubstituted 3 to 6 membered heterocycloalkyl, unsubstituted C₅₋₇ aryl, or unsubstituted 5 to 7 membered heteroaryl;
each R^{P} is independently hydrogen, halogen, -CN, -OR^{W}, -S(O)₂R^{W}, -NR^{X}R^{Y}, R^{U}-substituted or unsubstituted C₁₋₆ alkyl, R^{U}-substituted or unsubstituted C₁₋₆ haloalkyl, R^{U}-substituted or unsubstituted C₃₋₇ cycloalkyl, R^{U}-substituted or unsubstituted 3 to 6 membered heterocycloalkyl, R^{U}-substituted or unsubstituted C₅₋₇ aryl, or R^{U}-substituted or unsubstituted 5 to 7 membered heteroaryl;
each R^{W}, R^{X} and R^{Y} is independently hydrogen, unsubstituted C₁₋₆ alkyl, unsubstituted C₁₋₆ haloalkyl, unsubstituted C₃₋₇ cycloalkyl, or unsubstituted 3 to 7 membered heterocycloalkyl, and
each R^{U} is independently hydrogen, halogen, -CN, -OH, -OCH₃, -OCF₃, -S(O)₂H, - S(O)₂NH₂, -NH₂, -NH(CH₃), -N(CH₃)₂, -CF₃, -CHF₂, -CH₂F, -C(CH₃)₂F, -C(CH₃)F₂, unsubstituted C₁₋₆ alkoxy, unsubstituted C₁₋₆ alkyl, unsubstituted C₁₋₆ haloalkyl, unsubstituted C₃₋₇ cycloalkyl, unsubstituted 3 to 6 membered heterocycloalkyl, unsubstituted C₅₋₇ aryl, or unsubstituted 5 to 7 membered heteroaryl.

In one embodiment, the compound or pharmaceutically acceptable salt thereof has the formula: 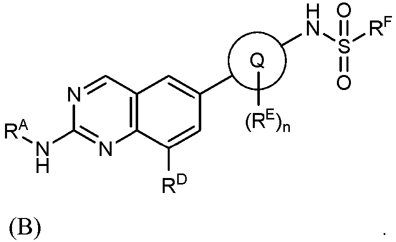 where R^{A}, R^{D}, R^{E}, R^{F}, Ring Q, and n are as defined herein.

In one embodiment, R^{D} is halogen, -CN, -OR^{I}, -S(O)₂R^{I}, or -NR^{J}R^{K}. In another embodiment, R^{D} is R^{N}-substituted or unsubstituted C₁₋₆ alkyl or R^{N}-substituted or unsubstituted C₁₋₆ haloalkyl. In another embodiment, R^{D} is R^{N}-substituted or unsubstituted C₃₋₇ cycloalkyl, R^{N}-substituted or unsubstituted 3 to 7 membered heterocycloalkyl. In still another embodiment, R^{D} is R^{N}-substituted or unsubstituted C₅₋₇ aryl, or R^{N}-substituted or unsubstituted 5 to 7 membered heteroaryl.

In one embodiment, R^{D} is halogen, -OR^{I}, -NR^{J}R^{K}, R^{N}-substituted or unsubstituted C₁₋₆ alkyl, R^{N}-substituted or unsubstituted C₁₋₆ haloalkyl, R^{N}-substituted or unsubstituted C₃₋₇ cycloalkyl, or R^{N}-substituted or unsubstituted 3 to 7 membered heterocycloalkyl.

In another embodiment, R^{D} is -OR^{I} or -NR^{J}R^{K}. In another embodiment, R^{D} is -OR^{I} and R^{I} is unsubstituted C₁₋₆ alkyl, or unsubstituted C₁₋₆ haloalkyl. In still another embodiment, R^{D} is -OR^{I} and R^{I} is methyl, ethyl, propyl, isopropyl, or butyl. In still another embodiment, R^{D} is - OR^{I} and R^{I} is ethyl or isopropyl. In one embodiment, R^{D} is -OR^{I} and R^{I} is R^{M}-substituted or unsubstituted 3 to 7 membered heterocycloalkyl. In one embodiment, R^{D} is -NR^{J}R^{K} and R^{J} and R^{K} are independently hydrogen or R^{M}-substituted or unsubstituted C₁₋₆ alkyl.

In one embodiment, R^{D} is R^{N}-substituted or unsubstituted C₁₋₆ alkyl or R^{N}-substituted or unsubstituted C₁₋₆ haloalkyl. In one embodiment, R^{D} is methyl, ethyl, propyl, or isopropyl. In one embodiment, R^{D} is hydrogen, methyl, ethyl, propyl, or isopropyl. In one preferred embodiment, R^{D} is ethyl. In another preferred embodiment, R^{D} is isopropyl. In still another preferred embodiment, R^{D} is hydrogen. In one embodiment, R^{D} is -C(CH₃)₂F, -C(CH₃)F₂, -CH₂F, -CHF₂, or -CF₃.

Further provided herein in one embodiment, R^{D} is R^{N}-substituted or unsubstituted C₃₋₇ cycloalkyl or R^{N}-substituted or unsubstituted 3 to 7 membered heterocycloalkyl. In one embodiment, R^{D} is R^{N}-substituted or unsubstituted 3 to 7 membered heterocycloalkyl. In one embodiment, R^{D} is unsubstituted 3 to 5 membered heterocycloalkyl, having one or more nitrogen or oxygen ring atom.

In one embodiment, each R^{E} is independently hydrogen, halogen, -OR^{I}, or -CN. In one embodiment, each R^{E} is independently R^{M}-substituted or unsubstituted C₁₋₆ alkyl or R^{M}-substituted or unsubstituted C₁₋₆ haloalkyl. In another embodiment, each R^{E} is independently R^{M}-substituted or unsubstituted C₃₋₇ cycloalkyl. In one embodiment, R^{E} is halogen and n is 1, 2, or 3. In another embodiment, n is 0. In another embodiment, R^{E} is F and n is 1, 2, or 3. In another embodiment, R^{E} is F and n is 1. In still another embodiment, R^{E} is F and n is 2. In still another embodiment, R^{E} is -OR^{I} where R^{I} is hydrogen or C₁₋₃ unsubstituted alkyl or C₁₋₃ unsubstituted haloalkyl and n is 1. In a further embodiment where R^{E} is -OR^{I} , R^{I} is hydrogen, methyl, or - OCF₃.

In one particular embodiment, L^{A} is -NHSO₂-.

In one preferred embodiment, R^{F} is R^{N}-substituted or unsubstituted C₁₋₆ alkyl or R^{N}-substituted or unsubstituted benzyl.

In one embodiment, R^{F} is as defined herein where R^{N} is hydrogen, halogen, -OH, - CN, -CF₃, -CHF₂, -CH₂F, -C(CH₃)₂F, -C(CH₃)F₂, methyl, ethyl, or propyl.

In one embodiment, R^{F} is as defined herein where R^{N} is halogen, -CN, R^{P}-substituted or unsubstituted C₁₋₆ alkoxy, R^{P}-substituted or unsubstituted C₁₋₆ alkyl, or R^{P}-substituted or unsubstituted C₁₋₆ haloalkyl. In another embodiment, R^{Y} is as defined herein where R^{N} is R^{P}-substituted or unsubstituted 3 to 6 membered heterocycloalkyl, R^{P}-substituted or unsubstituted C₅₋₇ cycloalkyl, R^{P}-substituted or unsubstituted C₅₋₇ aryl, or R^{P}-substituted or unsubstituted 5 to 7 membered heteroaryl. In one embodiment, R^{F} is as defined herein where R^{N} is halogen, -CN, unsubstituted C₁₋₆ alkoxy, unsubstituted C₁₋₆ alkyl, or unsubstituted C₁₋₆ haloalkyl. In another embodiment, R^{F} is as defined herein where R^{N} is unsubstituted 3 to 6 membered heterocycloalkyl, unsubstituted C₅₋₇ cycloalkyl, unsubstituted C₅₋₇ aryl, or unsubstituted 5 to 7 membered heteroaryl.

In one embodiment of above, R^{P} is independently hydrogen, halogen, -CN, -OR^{W}, - S(O)₂R^{W}, or -NR^{X}R^{Y}, where RW, RX, and RY are as defined herein. In one embodiment, R^{P} is independently hydrogen, halogen, -CN, -OH, -OCH₃, -OCF₃, -S(O)₂H, -S(O)₂NH₂, -NH₂, - NH(CH₃), -N(CH₃)₂, -CF₃, -CHF₂, -CH₂F, -C(CH₃)₂F, or -C(CH₃)F₂. In another embodiment, R^{P} is independently hydrogen, methyl, ethyl, propyl, or isopropyl. In another embodiment, R^{P} is independently hydrogen, butyl or isobutyl. In still another embodiment, R^{P} is independently hydrogen, unsubstituted C₁₋₆ haloalkyl, unsubstituted C₃₋₇ cycloalkyl, unsubstituted 3 to 6 membered heterocycloalkyl, unsubstituted C₅₋₇ aryl, or unsubstituted 5 to 7 membered heteroaryl.

In one embodiment, R^{F} is R^{N}-substituted or unsubstituted benzyl. In a further embodiment, R^{F} is unsubstituted benzyl. In still another embodiment, R^{F} is R^{N}-substituted benzyl and R^{N} is halogen, -CN, methyl, ethyl, or propyl. In still another embodiment, R^{F} is: 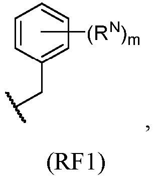 where R^{N} is halogen and m is 1 or 2. In one embodiment, R^{F} has formula RF1 where R^{N} is F or Cl and m is 1 or 2. In one embodiment, R^{F} has formula RF1 where R^{N} is F and m is 1 or 2.

In one embodiment, R^{F} is R^{N}-substituted or unsubstituted C₁₋₆ alkyl. In another embodiment, R^{F} is R^{N}-substituted C₁₋₆ alkyl and R^{N} is F or -CF₃. In still another embodiment, R^{F} is: 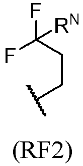 where R^{N} is F or methyl.

In one embodiment, R^{A} is:

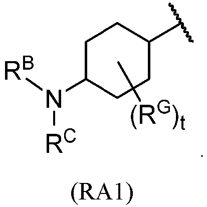

In one embodiment, R^{A} has formula (RA1) where R^{B} and R^{C} are independently hydrogen or R^{N}-substituted or unsubstituted C₁₋₆ alkyl. In another embodiment, R^{A} has formula (RA1) where R^{B} and R^{C} are independently R^{N}-substituted or unsubstituted C₃₋₇ cycloalkyl or R^{N}-substituted or unsubstituted 3 to 7 membered heterocycloalkyl. In still another embodiment, R^{A} has formula (RA1) where R^{B} and R^{C} are independently are taken together with the nitrogen atom to which they are attached to form a R^{N}-substituted or unsubstituted 4 to 7 membered heterocycloalkyl (e.g. azetidinyl, pyrrolidinyl, or piperidinyl). In one preferred embodiment, R^{A} has formula (RA1) where R^{B} and R^{C} are independently R^{N}-substituted or unsubstituted C₁₋₆ alkyl. In another preferred embodiment, R^{A} has formula (RA1) where R^{B} and R^{C} are each independently unsubstituted C₁₋₆ alkyl (e.g. methyl or ethyl). In still another embodiment, R^{A} has formula (RA1) where R^{B} is 4-membered heterocycloalkyl (e.g. azetidinyl or oxetanyl) and R^{C} is hydrogen or R^{N}-substituted or unsubstituted C₁₋₆ alkyl (e.g. methyl, ethyl). In one embodiment, R^{A} has formula (RA1), where R^{G} is halogen.

In one embodiment, R^{A} is:

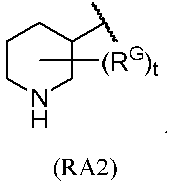

In one embodiment, R^{A} has formula (RA2), where R^{G} is halogen or R^{M}-substituted or unsubstituted C₁₋₆ alkyl, or R^{M}-substituted or unsubstituted C₁₋₆ haloalkyl. In one embodiment, R^{A} has formula (RA2), where R^{G} is F, methyl, ethyl, difluoromethyl, trifluoromethyl, fluorethyl, difluoroethyl, or trifluoroethyl.

In one embodiment, R^{A} has formula (RA1) where each R^{G} is independently halogen, - OR^{I}, R^{M}-substituted or unsubstituted C₁₋₆ alkyl, or R^{M}-substituted or unsubstituted C₁₋₆ haloalkyl.

In one embodiment, R^{G} is independently halogen or R^{M}-substituted or unsubstituted C₁₋₆ alkyl. In one embodiment, R^{G} is independently halogen or unsubstituted C₁₋₆ alkyl. In one embodiment, R^{G} is independently methyl or ethyl. In one embodiment, R^{G} is independently R^{M}-substituted or unsubstituted C₁₋₆ haloalkyl. In one embodiment, R^{G} is independently unsubstituted C₁₋₆ haloalkyl. In still another embodiment, R^{G} is independently -CF₃, -CHF₂, -CH₂F, -C(CH₃)₂F, -C(CH₃)F₂, -CH₃CH₂F, -CH₃CHF₂,-CH₃CF₃. In another embodiment, R^{G} is independently halogen. In still another embodiment, R^{G} is independently fluoro and t is 1 or 2. In another embodiment, R^{G} is independently fluoro and t is 1. In yet another embodiment, R^{G} is independently R^{M}-substituted or unsubstituted C₁₋₆ alkyl or R^{M}-substituted or unsubstituted C₁₋₆ haloalkyl. In one embodiment, t is 0.

In one particular embodiment, R^{A} has formula:

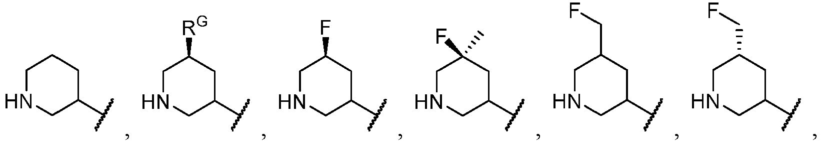

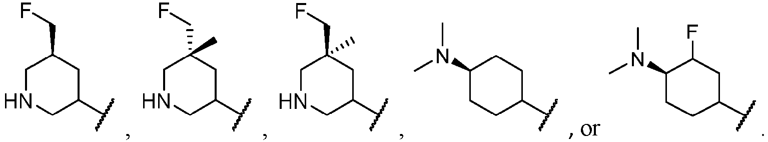

In one embodiment of the compounds or a pharmaceutically acceptable salt thereof described herein, each R^{H} is independently halogen, C₁₋₃ alkyl, or C₁₋₃ haloalkyl and j is 1 or 2. In another embodiment, each R^{H} is independently halogen or cyclopropyl and j is 1 or 2. In still another embodiment, R^{H} is halogen and j is 1. In another embodiment, R^{H} is methyl, ethyl, propyl, or isopropyl and j is 1. In one embodiment, R^{H} is ortho to R^{D} and j is 1. In a preferred embodiment, R^{H} is fluoro, methyl, ethyl, propyl, or isopropyl, R^{H} is ortho to R^{D} and j is 1.

In another embodiment of the compounds or a pharmaceutically acceptable salt thereof described herein, Ring Q is R^{E}-substituted or unsubstituted C₅₋₇ aryl. In another embodiment, Ring Q is R^{E}-substituted or unsubstituted phenyl. In still another embodiment, Ring Q has formula: 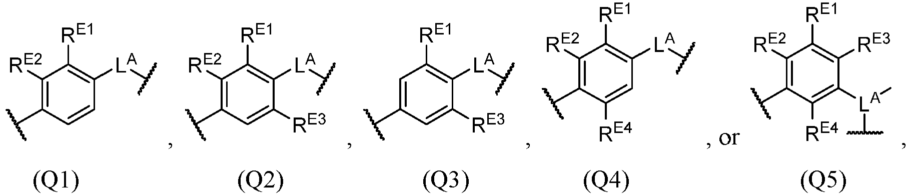 where R^{E1}, R^{E2}, R^{E3}, and R^{E4} are each independently hydrogen, halogen, or, R^{M}-substituted or unsubstituted C₁₋₆ alkyl. In one embodiment, R^{E1}, R^{E2}, R^{E3}, and R^{E4} are each independently hydrogen, halogen, or unsubstituted C₁₋₆ alkyl (e.g. methyl, ethyl, or propyl). In one embodiment, R^{E1}, R^{E2}, R^{E3}, and R^{E4} are each independently hydrogen or halogen (e.g. For Cl). In a preferred embodiment, R^{E1}, R^{E2}, R^{E3}, and R^{E4} are each independently hydrogen or F.

In one embodiment, Ring Q is pyridinyl. In another embodiment, Ring Q is pyrazinyl or pyrimidinyl. In one embodiment, Ring Q has formula: 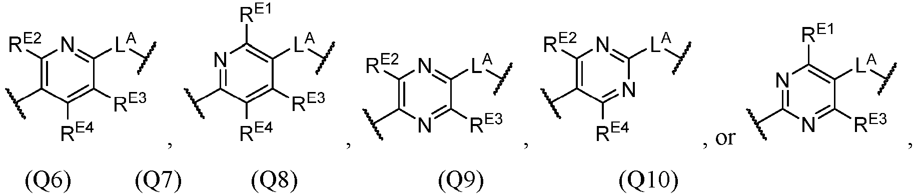 where R^{E1}, R^{E2}, R^{E3}, and R^{E4} are each independently hydrogen, halogen, or, R^{M}-substituted or unsubstituted C₁₋₆ alkyl. In a preferred embodiment, where Ring Q has formula (Q6)-(Q10), R^{E1}, R^{E2}, R^{E3}, and R^{E4} are each independently hydrogen or unsubstituted C₁₋₆ alkyl (e.g. methyl or ethyl).

In another embodiment, Ring Q has formula; 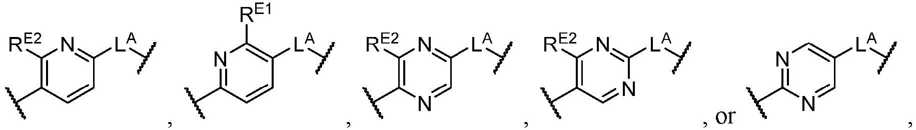 where R^{E1} and R^{E2} are independently unsubstituted C₁₋₆ alkyl.

In one embodiment, each R^{M} is independently hydrogen, halogen, -CN, -OH, -OCH₃, -OCF₃, -S(O)₂H, -S(O)₂NH₂, -NH₂, -NH(CH₃), -N(CH₃)₂, -CF₃, -CHF₂, -CH₂F, -C(CH₃)₂F, or - C(CH₃)F₂. In one embodiment, each R^{M} is independently hydrogen or halogen. In one embodiment, each R^{M} is independently -CN, -OH, or -OCH₃, -OCF₃. In one embodiment, each R^{M} is independently -S(O)₂H or -S(O)₂NH₂. In one embodiment, each R^{M} is independently -NH₂, -NH(CH₃) or -N(CH₃)₂. In one embodiment, each R^{M} is independently -CF₃, -CHF₂, -CH₂F, - C(CH₃)₂F, or -C(CH₃)F₂.

In one embodiment, each R^{M} is independently hydrogen, unsubstituted C₁₋₆ alkoxy, unsubstituted C₁₋₆ alkyl, or unsubstituted C₁₋₆ haloalkyl. In one embodiment, each R^{M} is independently methyl, ethyl, propyl, isopropyl, butyl or isobutyl. In one embodiment, each R^{M} is independently hydrogen, unsubstituted C₃₋₇ cycloalkyl, unsubstituted 3 to 6 membered heterocycloalkyl, unsubstituted C₅₋₇ aryl, or unsubstituted 5 to 7 membered heteroaryl.

In one embodiment, each R^{N} is independently hydrogen, halogen, -CN, -OR^{R}, - S(O)₂R^{R}, or -NR^{S}R^{T}. In one embodiment, each R^{N} is independently hydrogen. In one embodiment, each R^{N} is independently halogen. In one embodiment, each R^{N} is independently - OR^{R}, -S(O)₂R^{R}, or -NR^{S}R^{T}. In one embodiment, each R^{N} is independently hydrogen, halogen, - CN, -OH, -OCH₃, -OCF₃, -S(O)₂H, -S(O)₂NH₂, -NH₂, -NH(CH₃), -N(CH₃)₂, -CF₃, -CHF₂, -CH₂F, -C(CH₃)₂F, or -C(CH₃)F₂. In one embodiment, each R^{N} is independently hydrogen, halogen, -CN, -OH, -OCH₃, or -OCF₃. In one embodiment, each R^{N} is independently -S(O)₂H, -S(O)₂NH₂, - NH₂, -NH(CH₃), or -N(CH₃)₂. In one embodiment, each R^{N} is independently -CF₃, -CHF₂, -CH₂F, -C(CH₃)₂F, or -C(CH₃)F₂.

In one embodiment, each R^{N} is independently R^{P}-substituted or unsubstituted C₁₋₆ alkyl or R^{P}-substituted or unsubstituted C₁₋₆ haloalkyl. In one embodiment, each R^{N} is independently R^{P}-substituted or unsubstituted C₁₋₆ alkyl. In one embodiment, each R^{N} is independently R^{P}-substituted C₁₋₆ alkyl. In one preferred embodiment, each R^{N} is independently unsubstituted C₁₋₆ alkyl (e.g. methyl, ethyl, propyl, or isopropyl). In one embodiment, each R^{N} is independently R^{P}-substituted or unsubstituted C₁₋₆ haloalkyl. In one embodiment, each R^{N} is independently unsubstituted C₁₋₆ haloalkyl. In one embodiment, each R^{N} is independently R^{P}-substituted or unsubstituted C₁₋₆ alkoxy.

In one embodiment, each R^{N} is R^{P}-substituted or unsubstituted C₃₋₇ cycloalkyl, R^{P}-substituted or unsubstituted 3 to 6 membered heterocycloalkyl, R^{P}-substituted or unsubstituted C₅₋₇ aryl, or R^{P}-substituted or unsubstituted 5 to 7 membered heteroaryl.

In one embodiment, each R^{R}, R^{S} and R^{T} is independently hydrogen, R^{V}-substituted or unsubstituted C₁₋₆ alkyl, or R^{V}-substituted or unsubstituted C₁₋₆ haloalkyl. In one embodiment, each R^{R}, R^{S} and R^{T} is independently hydrogen, halogen, -CN, -OH, -OCH₃, -OCF₃, -S(O)₂H, - S(O)₂NH₂, -NH₂, -NH(CH₃), -N(CH₃)₂, -CF₃, -CHF₂, -CH₂F, -C(CH₃)₂F, or -C(CH₃)F₂. In one embodiment, each R^{R}, R^{S} and R^{T} is independently hydrogen, halogen, -CN, -OH, -OCH₃, or - OCF₃. In one embodiment, each R^{R}, R^{S} and R^{T} is independently -S(O)₂H, -S(O)₂NH₂, -NH₂, - NH(CH₃), or -N(CH₃)₂. In one embodiment, each R^{R}, R^{S} and R^{T} is independently -CF₃, -CHF₂, - CH₂F, -C(CH₃)₂F, or -C(CH₃)F₂.

In one embodiment, each R^{R}, R^{S} and R^{T} is independently R^{V}-substituted or unsubstituted C₃₋₇ cycloalkyl, or R^{V}-substituted or unsubstituted 3 to 7 membered heterocycloalkyl. In one embodiment, each R^{R}, R^{S} and R^{T} is independently unsubstituted C₃₋₇ cycloalkyl or unsubstituted 3 to 7 membered heterocycloalkyl.

In one embodiment, each R^{V} is independently hydrogen, halogen, -CN, -OH, -OCH₃, -OCF₃, -S(O)₂H, -S(O)₂NH₂, -NH₂, -NH(CH₃), -N(CH₃)₂, -CF₃, -CHF₂, -CH₂F, -C(CH₃)₂F, or - C(CH₃)F₂. In one embodiment, each R^{V} is independently hydrogen or halogen. In one embodiment, each R^{V} is independently -CN, -OH, or -OCH₃, -OCF₃. In one embodiment, each R^{V} is independently -S(O)₂H or -S(O)₂NH₂. In one embodiment, each R^{V} is independently -NH₂, -NH(CH₃) or -N(CH₃)₂. In one embodiment, each R^{V} is independently -CF₃, -CHF₂, -CH₂F, - C(CH₃)₂F, or -C(CH₃)F₂.

In one embodiment, each R^{V} is independently hydrogen, unsubstituted C₁₋₆ alkoxy, unsubstituted C₁₋₆ alkyl, or unsubstituted C₁₋₆ haloalkyl. In one embodiment, each R^{V} is independently methyl, ethyl, propyl, isopropyl, butyl or isobutyl. In one embodiment, each R^{V} is independently hydrogen, unsubstituted C₃₋₇ cycloalkyl, unsubstituted 3 to 6 membered heterocycloalkyl, unsubstituted C₅₋₇ aryl, or unsubstituted 5 to 7 membered heteroaryl.

In one embodiment, each R^{P} is independently hydrogen, halogen, -CN, -OR^{W}, - S(O)₂R^{W}, -NR^{X}R^{Y}, R^{U}-substituted or unsubstituted C₁₋₆ alkyl, R^{U}-substituted or unsubstituted C₁₋₆ haloalkyl, R^{U}-substituted or unsubstituted C₃₋₇ cycloalkyl, R^{U}-substituted or unsubstituted 3 to 6 membered heterocycloalkyl, R^{U}-substituted or unsubstituted C₅₋₇ aryl, or R^{U}-substituted or unsubstituted 5 to 7 membered heteroaryl.

In one embodiment, each R^{P} is independently hydrogen, halogen, -CN, -OR^{W}, - S(O)₂R^{W}, or -NR^{X}R^{Y}. In one embodiment, each R^{P} is independently hydrogen. In one embodiment, each R^{P} is independently halogen. In one embodiment, each R^{P} is independently - OR^{W}, -S(O)₂R^{W}, or -NR^{X}R^{Y}. In one embodiment, each R^{P} is independently hydrogen, halogen, - CN, -OH, -OCH₃, -OCF₃, -S(O)₂H, -S(O)₂NH₂, -NH₂, -NH(CH₃), -N(CH₃)₂, -CF₃, -CHF₂, -CH₂F, -C(CH₃)₂F, or -C(CH₃)F₂. In one embodiment, each R^{P} is independently hydrogen, halogen, -CN, -OH, -OCH₃, or -OCF₃. In one embodiment, each R^{P} is independently -S(O)₂H, -S(O)₂NH₂, - NH₂, -NH(CH₃), or -N(CH₃)₂. In one embodiment, each R^{P} is independently -CF₃, -CHF₂, -CH₂F, -C(CH₃)₂F, or -C(CH₃)F₂.

In one embodiment, each R^{P} is independently R^{U}-substituted or unsubstituted C₁₋₆ alkyl or R^{U}-substituted or unsubstituted C₁₋₆ haloalkyl. In one embodiment, each R^{P} is independently R^{U}-substituted or unsubstituted C₁₋₆ alkyl. In one embodiment, each R^{Y} is independently R^{U}-substituted C₁₋₆ alkyl. In one preferred embodiment, each R^{Y} is independently unsubstituted C₁₋₆ alkyl (e.g. methyl, ethyl, propyl, or isopropyl). In one embodiment, each R^{P} is independently R^{U}-substituted or unsubstituted C₁₋₆ haloalkyl. In one embodiment, each R^{P} is independently unsubstituted C₁₋₆ haloalkyl.

In one embodiment, each R^{P} is R^{U}-substituted or unsubstituted C₃₋₇ cycloalkyl, R^{U}-substituted or unsubstituted 3 to 6 membered heterocycloalkyl, R^{U}-substituted or unsubstituted C₅₋₇ aryl, or R^{U}-substituted or unsubstituted 5 to 7 membered heteroaryl.

In one embodiment, each R^{W}, R^{X} and R^{Y} is independently hydrogen, unsubstituted C₁₋₆ alkyl, or unsubstituted C₁₋₆ haloalkyl. In one embodiment, each R^{W}, R^{X} and R^{Y} is independently methyl, ethyl, propyl, or isopropyl. In one embodiment, each R^{W}, R^{X} and R^{Y} is independently - CF₃, -CHF₂, -CH₂F, -C(CH₃)₂F, -C(CH₃)F₂, -CH₃CH₂F, -CH₃CHF₂,-CH₃CF₃.

In one embodiment, each R^{W}, R^{X} and R^{Y} is independently hydrogen, unsubstituted C₃₋₇ cycloalkyl, or unsubstituted 3 to 7 membered heterocycloalkyl.

In one embodiment, each R^{U} is independently hydrogen, halogen, -CN, -OH, -OCH₃, -OCF₃, -S(O)₂H, -S(O)₂NH₂, -NH₂, -NH(CH₃), -N(CH₃)₂, -CF₃, -CHF₂, -CH₂F, -C(CH₃)₂F, or - C(CH₃)F₂. In one embodiment, each R^{U} is independently hydrogen or halogen. In one embodiment, each R^{U} is independently -CN, -OH, or -OCH₃, -OCF₃. In one embodiment, each R^{U} is independently -S(O)₂H or -S(O)₂NH₂. In one embodiment, each R^{U} is independently -NH₂, -NH(CH₃) or -N(CH₃)₂. In one embodiment, each R^{U} is independently -CF₃, -CHF₂, -CH₂F, - C(CH₃)₂F, or -C(CH₃)F₂.

In one embodiment, each R^{U} is independently hydrogen, unsubstituted C₁₋₆ alkoxy, unsubstituted C₁₋₆ alkyl, or unsubstituted C₁₋₆ haloalkyl. In one embodiment, each R^{U} is independently methyl, ethyl, propyl, isopropyl, butyl or isobutyl. In one embodiment, each R^{U} is independently hydrogen, unsubstituted C₃₋₇ cycloalkyl, unsubstituted 3 to 6 membered heterocycloalkyl, unsubstituted C₅₋₇ aryl, or unsubstituted 5 to 7 membered heteroaryl.

In one embodiment of the compounds or a pharmaceutically acceptable salt thereof described herein, the compound or pharmaceutically acceptable salt thereof has formula: 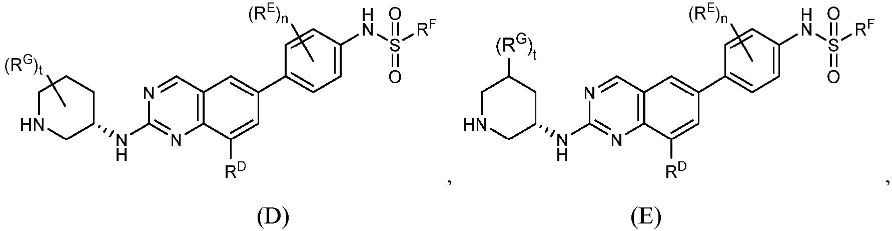 or 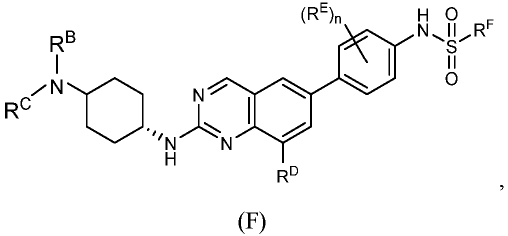 where R^{B}, R^{C}, R^{D}, R^{E}, R^{F}, R^{G}, n, and t are as described herein.

In one embodiment, the compound or pharmaceutically acceptable salt thereof is a compound set forth in Table 1.

**Table 1:**

| Cmpd No. | Structure | Name | IRE1α (alpha) HTRF (IC₅₀) (µM) | Mass Spec. M+H /1 | ¹H NMR | Synthetic Method |
|---|---|---|---|---|---|---|
| 1001. | 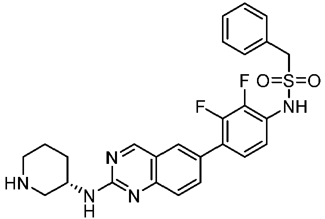 | *(S)-N-(2,3-*Difluoro-4-(2-(piperidin-3-ylamino)quinazol in-6-yl)phenyl)-1-phenylmethane sulfonamide hydrochloride | 0.00057 | 510.1 | (400 MHz, CD₃OD) δ 9.09 (s, 1H), 7.94 - 7.82 (m, 2H), 7.58 - 7.53 (m, 1H), 7.39 - 7.38 (m, 2H), 7.38 - 7.28 (m, 4H), 7.19 - 7.14 (m, 1H), 4.44 (s, 2H), 4.20 - 4.16 (m, 1H), 3.41 - 3.33 (m, 1H), 3.08 (d, *J* = 12.4 Hz, 1H), 2.80 - 2.72 (m, 2H), 2.14 - 2.12 (m, 1H), 1.94 - 1.90 (m, 1H), 1.76 - 1.65 (m, 2H). | Ex. 1001 |
| 1002. | 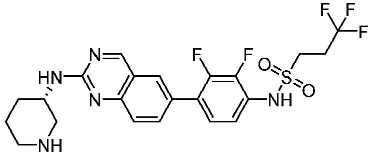 | *(S)-N-(2,3-*difluoro-4-(2-(piperidin-3-ylamino)quinazol in-6-yl)phenyl)-3,3,3-trifluoropropane-1-sulfonamide | 0.0076 | 516.1 | (300 MHz, DMSO-*d*₆) δ 9.20 (s, 1H), 7.95 (s, 1H), 7.87 (*d, J* = 8.8 Hz, 1H), 7.54 - 7.51 (m, 2H), 7.22 - 7.20 (m, 1H), 7.08 (d, *J =* 8.8 Hz, 1H), 4.30 - 4.12 (m, 1H), 3.42 - 3.35 (m, 2H), 3.17 - 3.14 (m, 2H), 2.97 - 2.94 m, 2H), 2.83 - 2.77 (m, 2H), 2.65 - 2.60 (m, 2H), 2.03 - 1.87(m, 2H), 1.74 - 1.54 (m, 2H). | Ex. 1001 |
| 1003. | 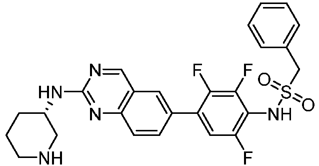 | (S)-1-phenyl-N-(2,3,6-trifluoro-4-(2-(piperidin-3-ylamino)quinazol in-6-yl)phenyl) methanesulfonam ide | 0.00065 | 528.2 | (300 MHz, DMSO-*d*₆) δ 9.20 (s, 1H), 8.30 - 8.05 (brs, 1H), 8.02 (s, 1H),7.90 (d, *J =* 9.0 Hz, 1H), 7.57 - 7.51 (m, 2H), 7.41 - 7.37 (m, 2H), 7.34 - 7.23 (m, 3H), 7.20 - 7.12 (m, 1H), 4.17 (s, 3H), 3.37 - 3.31 (m, 2H), 3.09 (d, *J* = 12.2 Hz, 1H), 2.81 - 2.71 (m, 2H), 2.07 - 1.98 (m, 1H), 1.86 - 1.83 (m, 1H), 1.68 - 1.57 (m, 2H). | Ex. 1001 |
| 1004. | 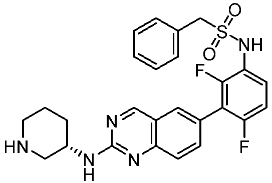 | *(S)-N-(2,4-*difluoro-3-(2-(piperidin-3-ylamino)quinazol in-6-yl)phenyl)-1-phenylmethane sulfonamide | 0.1500 | 510.2 | (300 MHz, CD₃OD) δ 9.11 (s, 1H), 7.89 (s, 1H), 7.78 (d, *J* = 9.0 Hz, 1H), 7.62 (d, *J =* 8.8 Hz, 1H), 7.49 - 7.41 (m, 3H), 7.41 - 7.28 (m, 3H), 7.07 - 6.94 (m, 1H), 4.46 (brs, 2H), 4.20 - 4.14 (m, 1H), 3.38 - 3.34 (m, 1H), 3.03 (d, *J* = 12.9 Hz, 1H), 2.77 - 2.56 (m, 2H), 2.16 - 2.13 (m, 1H), 1.90 - 1.86 (m, 1H), 1.73 - 1.61 (m, 2H). | Ex. 1001 |
| 1005. | 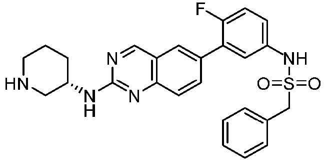 | (S)-N-(4-fluoro-3-(2-(piperidin-3-ylamino)quinazol in-6-yl)phenyl)-1-phenylmethane sulfonamide | 0.0790 | 492.2 | (400 MHz, CD₃OD) δ 9.10 (s, 1H), 7.92 - 7.84 (m, 2H), 7.60 (d, *J =* 8.8 Hz, 1H), 7.36 - 7.26 (m, 6H), 7.18 - 7.15 (m, 2H), 4.45 (s, 2H), 4.14 - 4.13 (m, 1H), 3.35 - 3.33 (m, 1H), 2.98 (dd, *J* = 12.7, 4.2 Hz, 1H), 2.68 - 2.56 (m, 2H), 2.15 - 2.13(m, 1H), 1.84 - 1.80 (m, 1H), 1.69 - 1.62 (m, 2H) | Ex. 1005 |
| 1006. | 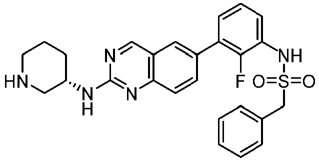 | (S)-N-(2-fluoro-3-(2-(piperidin-3-ylamino)quinazol in-6-yl)phenyl)-1-phenylmethane sulfonamide | 0.100 | 492.2 | (300 MHz, CD₃OD) δ 9.12 (s, 1H), 7.99 - 7.88 (m, 2H), 7.62 (d, *J =* 8.7 Hz, 1H), 7.52 - 7.25 (m, 7H), 7.30 - 7.12 (m, 1H), 4.49 (s, 2H), 4.12 (brs, 1H), 3.48 - 3.43 (m, 1H), 3.04 - 2.99 (m, 1H), 2.75 - 2.54 (m, 2H), 2.16 - 2.12 (m, 1H), 1.90 - 1.85 (m, 1H), 1.74 - 1.60 (m, 2H). | Ex. 1005 |
| 1007. | 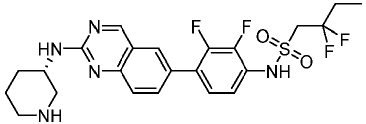 | *(S)-N-(2,3-*difluoro-4-(2-(piperidin-3-ylamino)quinazol in-6-yl)phenyl)-2,2-difluorobutane-1-sulfonamide | 0.0029 | 512.3 | (400 MHz, CD₃OD) δ 9.04 (s, 1H), 7.88 - 7.79 (m, 2H), 7.54 (d, *J* = 9.1 Hz, 1H), 7.39 - 7.30 (m, 1H), 7.19 - 7.09 (m, 1H), 4.34 - 4.18 (m, 1H), 3.67 (t, *J* = 13.6 Hz, 2H), 3.54 - 3.43 (m, 1H), 3.26 - 3.13 (m, 1H), 3.11 - 2.92 (m, 2H), 2.30 - 2.12 (m, 3H), 2.10 - 2.00 (m, 1H), 1.91 - 1.64 (m, 2H), 1.05 (t, *J* = 7.5 Hz, 3H). | Ex. 1001 |
| 1008. | 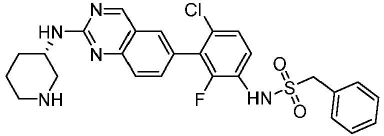 | (S)-N-(4-chloro-2-fluoro-3-(2-(piperidin-3-ylamino)quinazol in-6-yl)phenyl)-1-phenylmethane sulfonamide | 0.0300 | 526.1 | (300 MHz, CD₃OD) δ 9.09 (s, 1H), 7.77 (d, *J* = 1.9 Hz, 1H), 7.67 - 7.64 (m, 1H), 7.59 - 7.56 (m, 1H), 7.46 - 7.36 (m, 3H), 7.31 - 7.19 (m, 4H), 4.44 (s, 2H), 4.19 - 1.17 (m, 1H), 3.40 - 3.33 (m, 1H), 3.07 - 3.02 (m, 1H), 2.76 - 2.61 (m, 2H), 2.15 - 2.12 (m, 1H), 1.92 - 1.88 (m, 1H), 1.71 - 1.66 (m, 2H). | Ex. 1005 |
| 1009. | 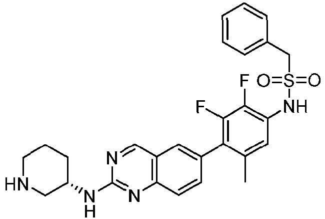 | *(S)-N-(2,3-*difluoro-5-methyl -4-(2-(piperidin-3-ylamino) quinazolin-6-yl)phenyl)-1-phenylmethane sulfonamide | 0.0049 | 524.2 | (300 MHz, CD₃OD) δ 9.10 (s, 1H), 7.69 (s, 1H), 7.61 (d, *J* = 1.8 Hz, 2H), 7.42 - 7.40 (m, 2H), 7.38 - 7.32 (m, 3H), 7.07 (d, *J=* 7.8 Hz, 1H), 4.48 (s, 2H), 4.23 - 4.20 (m, 1H), 3.40 (d, *J* = 12.0 Hz, 1H), 3.07 (d, *J* = 12.9 Hz, 1H), 2.79 - 2.67 (m, 2H), 2.15 (d, *J* = 11.7 Hz, 1H), 2.08 (s, 3H), 1.92 (d, *J =* 14.1 Hz, 1H), 1.72 - 1.67 (m, 2H). | Ex. 1005 |
| 1010. | 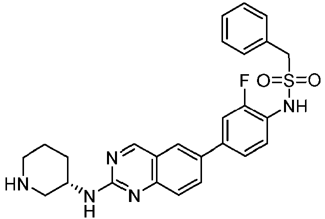 | (S)-N-(2-fluoro-4-(2-(piperidin-3-ylamino)quinazol in-6-yl)phenyl)-1-phenylmethane sulfonamide | 0.0110 | 492.3 | (300 MHz, CD₃OD) δ 9.20 (s, 1H), 8.09 - 8.05 (m, 2H), 7.66 (d, *J =* 8.4 Hz, 1H), 7.59 - 7.40 (m, 3H), 7.38 - 7.33 (m, 5H), 4.51 (s, 2H), 4.40 (d, *J* = 4.2 Hz, 1H), 3.69 - 3.67 (m, 1H), 3.35 - 3.32 (m, 1H), 3.11 - 3.03 (m, 2H), 2.23 - 2.11 (m, 2H), 1.95 - 1.80 (m, 2H). | Ex. 1005 |
| 1011. | 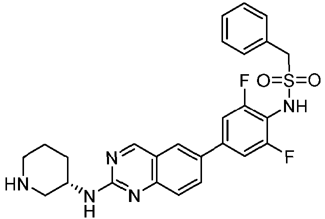 | (*S*)-*N*-(2,6-difluoro-4-(2-(piperidin-3-ylamino)quinazol in-6-yl)phenyl)-1-phenylmethane sulfonamide | 0.00053 | 510.1 | (400 MHz, DMSO-*d*₆) 9.15 (s, 1H), 8.19 (d, *J* = 2.3 Hz, 1H), 8.13 - 8.03 (m, 1H), 7.55 - 7.39 (m, 6H), 7.39 - 7.28 (m, 3H), 4.32 (s, 2H), 4.05 (s, 1H), 3.21 (s, 1H), 2.95 (d, *J =* 12.4 Hz, 1H), 2.69 - 2.57 (m, 1H), 2.59 - 2.49 (m, 2H), 2.52 - 2.42 (m, 1H), 1.95 (s, 1H), 1.75 (s, 1H), 1.56 (d, *J* = 9.1 Hz, 2H). | Ex. 1005 |
| 1012. | 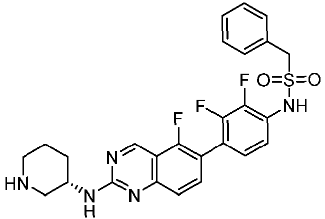 | *(S)-N-(2,3-*difluoro-4-(5-fluoro-2-(piperidin-3-ylamino)quinazol in-6-yl)phenyl)-1-phenylmethane sulfonamide | 0.0150 | 528.2 | (300 MHz, DMSO-*d*₆) δ 9.36 (s, 1H), 8.18 (s, 2H), 7.86 - 7.68 (m, 2H), 7.41 - 7.16 (m, 7H), 7.05 - 6.96 (m, 1H), 4.21 (s, 3H), 3.40 - 3.30 (m, 1H), 3.18 - 3.06 (m, 1H), 2.78 - 2.68 (m, 2H), 2.03 - 1.92 (m, 1H), 1.91 - 1.81 (m, 1H), 1.72 - 1.53 (m, 2H). | Ex. 1001 |
| 1013. | 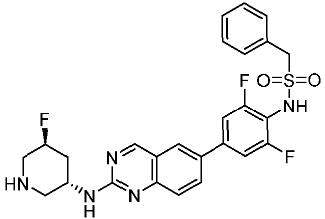 | N-(2,6-difluoro-4-(2-(((3*S*,5*S*)-5-fluoropiperidin-3-yl)amino)quinazo lin-6-yl)phenyl)-1-phenylmethane sulfonamide | 0.00051 | 528.2 | (300 MHz, DMSO-*d*₆) 9.18 (s, 1H), 8.28 (d, *J* = 2.1 Hz, 1H), 8.19 - 8.09 (m, 1H), 7.67 (d, *J* = 9.2 Hz, 2H), 7.57 (d, *J* = 7.9 Hz, 2H), 7.48 - 7.35 (m, 5H), 5.01 - 4.85 (m, 1H), 4.51 (s, 2H), 4.31 (s, 1H), 3.21 - 2.97 (m, 2H), 2.94 - 2.69 (m, 1H), 2.6 - 2.54 (m, 3H), 2.32 - 2.14 (m, 1H), 2.03 - 1.68 (m, 1H). | Ex. 1005 |
| 1014. | 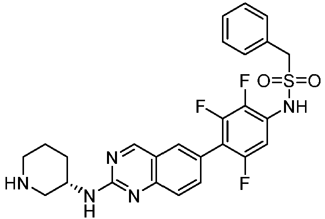 | (S)-1-phenyl-N-(2,3,5-trifluoro-4-(2-(piperidin-3-ylamino)quinazol in-6-yl)phenyl) methane sulfonamide hydrochloride | 0.0021 | 528.2 | (300 MHz, DMSO-*d*₆) δ 10.41 (s, 1H), 9.32 - 9.29 (m, 1H), 9.15 - 8.90 (m, 2H), 8.00 (s, 2H), 7.82 (d, *J* = 4.5 Hz,1H), 7.67 (d, *J =* 4.5 Hz, 1H), 7.43 - 7.36 (m, 5H), 7.08 - 7.03 (m, 1H), 4.68 (s, 2H), 4.35 - 4.25 (m, 2H), 3.44 - 3.38 (m, 1H), 3.30 - 3.15 (m,1H), 2.93 - 2.86 (m, 2H), 2.02 - 1.60 (m, 4H). | Ex. 1009 |
| 1015. | 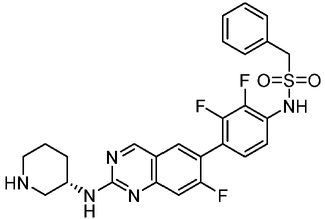 | *(S)-N-(2,3-*difluoro-4-(7-fluoro-2-(piperidin-3-ylamino)quinazol in-6-yl)phenyl)-1-phenylmethane sulfonamide | 0.0031 | 528.2 | (400 MHz, DMSO-*d*₆) δ 9.17 (s, 1H), 8.15 (s, 1H), 7.94 (d, *J =* 8.4 Hz, 1H), 7.69 (d, *J* = 7.6 Hz, 1H), 7.32 - 7.21 (m, 6H), 7.00 - 6.96 (m, 1H), 4.18 (s, 3H), 3.32 - 3.29 (m, 1H), 3.07 - 3.04 (m, 1H), 2.75 - 2.68 (m, 2H), 1.99 - 1.96 (m, 1H), 1.86 - 1.83 (m, 1H), 1.67 - 1.57 (m, 2H). | Ex. 1001 |
| 1016. | 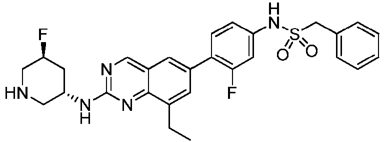 | N-(4-(8-ethyl-2-(((3*S*,5*S*)-5-fluoropiperidin-3-yl)amino)quinazo lin-6-yl)-3-fluorophenyl)-1-phenylmethane sulfonamide formate | 0.00046 | 538.1 | (400 MHz, *d₆*-DMSO) δ 9.15 (s, 1H), 8.19 (s, 1H), 7.79 (s, 1H), 7.71 (s, 1H), 7.56 (t, *J =* 8.9 Hz, 1H), 7.46 - 7.34 (m, 4H), 7.33 - 7.25 (m, 2H), 7.16 - 7.00 (m, 2H), 4.84 (d, *J =* 47.3 Hz, 1H), 4.58 (s, 2H), 4.21 (s, 1H), 3.18 - 3.08 (m, 1H), 3.03 (q, *J* = 7.4 Hz, 2H), 2.98 - 2.87 (m, 1H), 2.84 - 2.61 (m, 1H), 2.29 - 2.14 (m, 1H), 1.98 - 1.74 (m, 1H), 1.29 (t, *J =* 7.4 Hz, 3H). | *Ex. 1016* |
| 1017. | 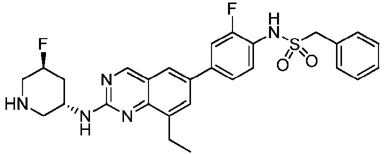 | N-(4-(8-ethyl-2-(((3*S*,5*S*)-5-fluoropiperidin-3-yl)amino)quinazo lin-6-yl)-2-fluorophenyl)-1-phenylmethane sulfonamide | 0.00018 | 538.1 | (400 MHz, d6-dmso) δ 9.13 (s, 1H), 7.96 (d, *J* = 1.8 Hz, 1H), 7.91 (d, *J* = 1.8 Hz, 1H), 7.66 (d, *J* = 12.1 Hz, 1H), 7.52 (d, *J= 10.1* Hz, 1H), 7.44 - 7.29 (m, 7H), 4.83 (d, *J* = 48.1 Hz, 1H), 4.47 (s, 2H), 4.21 (br s, 1H), 3.16 - 3.07 (m, 1H), 3.04 (q, *J* = 7.3 Hz, 2H), 2.98 - 2.87 (m,1H), 2.82 - 2.64 (m, 1H), 2.59 - 2.51 (m, 1H), 2.30 - 2.13 (m, 1H), 2.00 - 1.76 (m, 1H), 1.31 (t, *J* = 7.5 Hz, 3H). | *Ex. 1017* |
| 1018. | 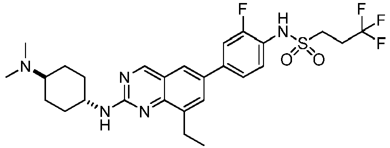 | N-(4-(2-(((1,4-*trans*)-4-(dimethylamino)c yclohexyl)amino) -8-ethyl quinazolin-6-yl)-2-fluorophenyl)-3,3,3-trifluoropropane-1-sulfonamide | 0.00025 | 568.2 | (400 MHz, d6-dmso) δ 9.09 (s, 1H), 7.90 (dd, *J* = 13.5, 1.9 Hz, 2H), 7.57 (d, *J=* 12.9 Hz, 1H), 7.44 (dd, *J =* 15.7, 7.3 Hz, 2H), 7.39 - 7.28 (m, 1H), 3.88 - 3.66 (m, 1H), 3.23 - 3.11 (m, 2H), 3.02 (q, *J=* 7.6 Hz, 2H), 2.82 - 2.64 (m, 4H), 2.34 (s, 6H), 2.21 - 2.03 (m, 1H), 1.98 - 1.83 (m, 2H), 1.47 - 1.34 (m, 3H), 1.31 (t, *J* = 7.5 Hz, 3H). | *Ex. 1018* |

In one embodiment, the compound or pharmaceutically acceptable salt thereof is a compound set forth in Table 2, with the exception of compounds 628, 629, 630, 637, and 638, or pharmaceutically acceptable salts thereof, which are disclosed herein for reference.

**Table 2**

| | Structure | Name | Mw (m/z) |
|---|---|---|---|
| 600 | 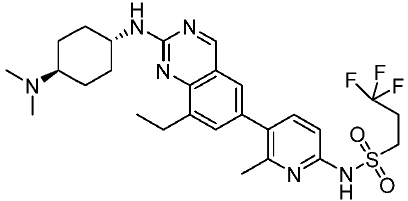 | *N*-(5-(2-(((*1r,4r*)-4-(dimethylamino) cyclohexyl)amino)-8-ethylquinazolin-6-yl)-6-methylpyridin-2-yl)-3,3,3-trifluoropropane-1-sulfonamide | 564.25 |
| 601 | 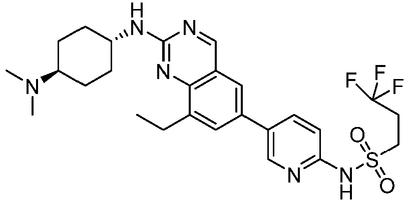 | *N*-(5-(2-(((*1r,4r*)-4-(dimethylamino) cyclohexyl)amino)-8-ethylquinazolin-6-yl)pyridin-2-yl)-3,3,3-trifluoropropane-1-sulfonamide | 550.23 |
| 602 | 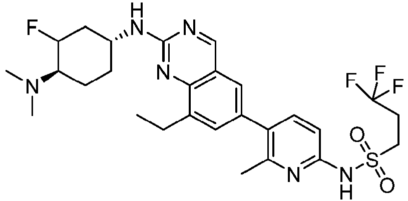 | *N*-(5-(2-(((1*R*,4*R*)-4-(dimethylamino)-3-fluorocyclohexyl)amino)-8-ethyl quinazolin-6-yl)-6-methylpyridin-2-yl)-3,3,3-trifluoropropane-1-sulfonamide | 582.24 |
| 603 | 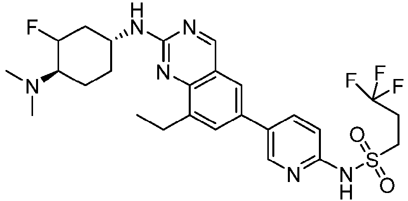 | *N*-(5-(2-(((1*R*,4*R*)-4-(dimethylamino)-3-fluorocyclohexyl)amino)-8-ethyl quinazolin-6-yl)pyridin-2-yl)-3,3,3-trifluoropropane-1-sulfonamide | 568.22 |
| 604 | 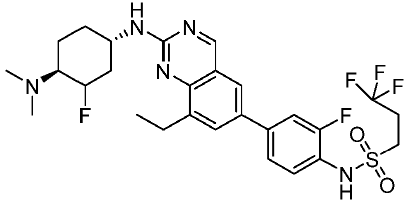 | *N*-(4-(2-(((1*S,*4*S*)-4-(dimethylamino)-3-fluorocyclohexyl)amino)-8-ethyl quinazolin-6-yl)-2-fluorophenyl)-3,3,3-trifluoropropane-1-sulfonamide | 585.22 |
| 605 | 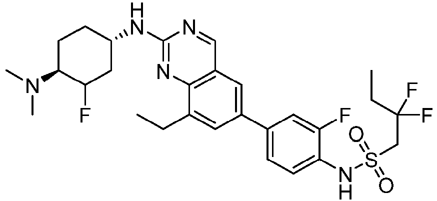 | *N*-(4-(2-(((1*S*,4*S*)-4-(dimethylamino)-3-fluorocyclohexyl)amino)-8-ethyl quinazolin-6-yl)-2-fluorophenyl)-2,2-difluorobutane-1-sulfonamide | 581.24 |
| 606 | 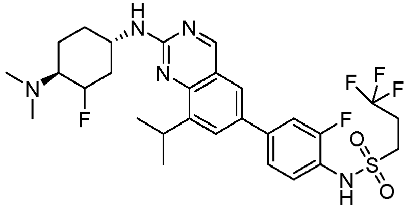 | *N*-(4-(2-(((1*S*,4*S*)-4-(dimethylamino)-3-fluorocyclohexyl)amino)-8-isopropyl quinazolin-6-yl)-2-fluorophenyl)-3,3,3-trifluoropropane-1-sulfonamide | 599.24 |
| 607 | 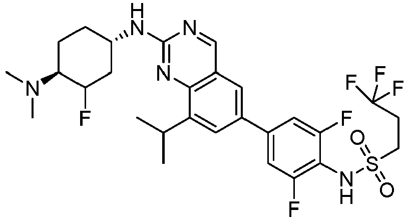 | *N*-(4-(2-(((*1S,4S*)-4-(dimethylamino)-3-fluorocyclohexyl)amino)-8-isopropyl quinazolin-6-yl)-2,6-difluorophenyl)-3,3,3-trifluoropropane-1-sulfonamide | 617.23 |
| 608 | 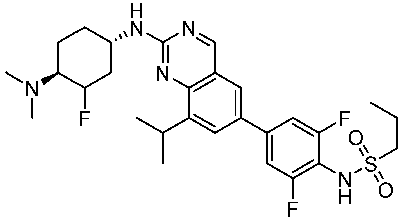 | *N*-(4-(2-(((*1S,4S*)-4-(dimethylamino)-3-fluorocyclohexyl)amino)-8-isopropyl quinazolin-6-yl)-2,6-difluorophenyl)propane-1-sulfonamide | 563.25 |
| 609 | 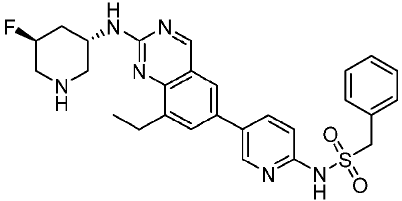 | *N*-(5-(8-ethyl-2-(((*3S,5S*)-5-fluoropiperidin-3-yl)amino)quinazolin-6-yl)pyridin-2-yl)-1-phenylmethane sulfonamide | 520.21 |
| 610 | 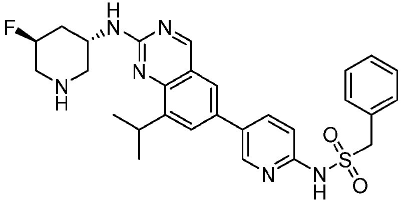 | *N*-(5-(2-(((3S,5S)-5-fluoropiperidin-3-yl)amino)-8-isopropylquinazolin-6-yl)pyridin-2-yl)-1-phenylmethane sulfonamide | 534.22 |
| 611 | 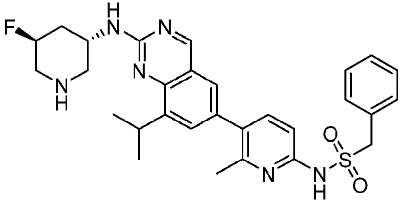 | *N*-(5-(2-(((*3S,5S*)-5-fluoropiperidin-3-yl)amino)-8-isopropylquinazolin-6-yl)-6-methylpyridin-2-yl)-1-phenylmethane sulfonamide | 548.24 |
| 612 | 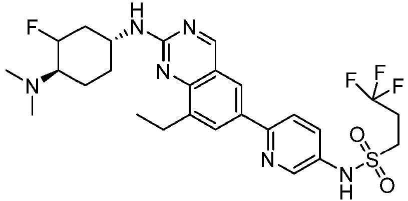 | *N*-(6-(2-(((*1R,4R*)-4-(dimethylamino)-3-fluorocyclohexyl)amino)-8-ethyl quinazolin-6-yl)pyridin-3-yl)-3,3,3-trifluoropropane-1-sulfonamide | 568.22 |
| 613 | 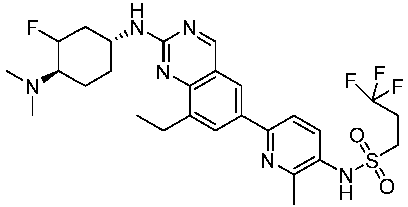 | *N*-(6-(2-(((*1R,4R*)-4-(dimethylamino)-3-fluorocyclohexyl)amino)-8-ethyl quinazolin-6-yl)-2-methylpyridin-3-yl)-3,3,3-trifluoropropane-1-sulfonamide | 582.24 |
| 614 | 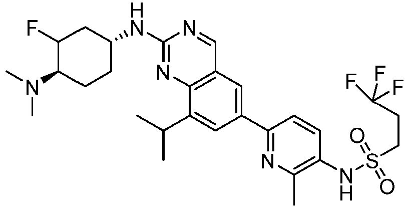 | *N*-(6-(2-(((*1R,4R*)-4-(dimethylamino)-3-fluorocyclohexyl)amino)-8-isopropyl quinazolin-6-yl)-2-methylpyridin-3-yl)-3,3,3-trifluoropropane-1-sulfonamide | 596.26 |
| 615 | 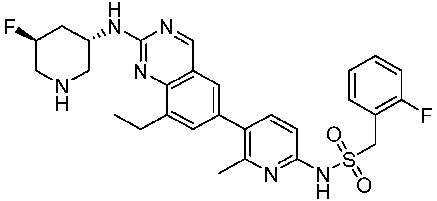 | *N*-(5-(8-ethyl-2-(((*3S,5S*)-5-fluoropiperidin-3-yl)amino)quinazolin-6-yl)-6-methylpyridin-2-yl)-1-(2-fluorophenyl)methanesulfonamide | 552.21 |
| 616 | 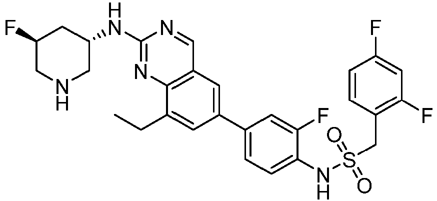 | 1-(2,4-difluorophenyl)-*N*-(4-(8-ethyl-2-(((*3S,5S*)-5-fluoropiperidin-3-yl)amino) quinazolin-6-yl)-2-fluorophenyl)methane sulfonamide | 573.18 |
| 617 | 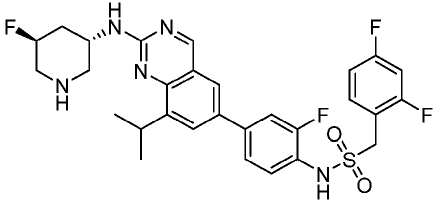 | 1-(2,4-difluorophenyl)-N-(2-fluoro-4-(2-(((*3S,*5S)-5- fluoropiperidin-3-yl)amino)-8-isopropylquinazolin-6-yl)phenyl) methanesulfonamide | 587.20 |
| 618 | 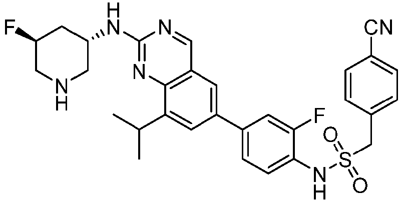 | 1-(4-cyanophenyl)-N-(2-fluoro-4-(2-(((*3S,5S*)-5-fluoropiperidin-3-yl)amino)-8-isopropylquinazolin-6-yl)phenyl) methanesulfonamide | 576.21 |
| 619 | 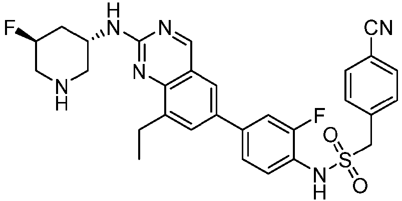 | 1-(4-cyanophenyl)-N-(4-(8-ethyl-2-(((*3S,5S*)-5-fluoropiperidin-3-yl)amino)quinazolin-6-yl)-2-fluorophenyl)methanesulfonamide | 562.20 |
| 620 | 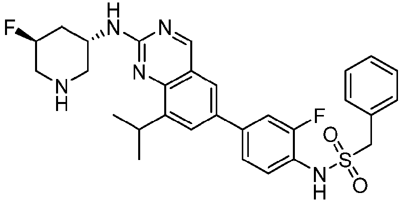 | *N*-(2-fluoro-4-(2-(((*3S,5S*)-5-fluoropiperidin-3-yl)amino)-8-isopropyl quinazolin-6-yl)phenyl)-1-phenylmethane sulfonamide | 551.22 |
| 621 | 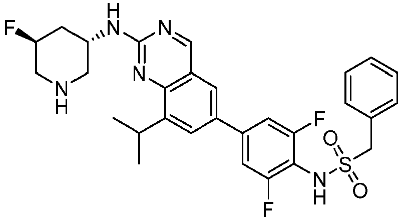 | *N*-(2,6-difluoro-4-(2-(((*3S,5S*)-5-fluoropiperidin-3-yl)amino)-8-isopropyl quinazolin-6-yl)phenyl)-1-phenylmethane sulfonamide | 569.21 |
| 622 | 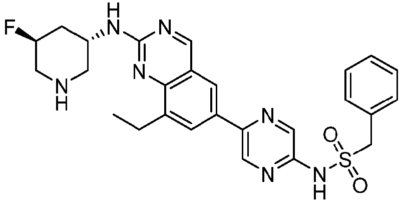 | *N*-(5-(8-ethyl-2-(((3S, *5S)-5-*fluoropiperidin-3-yl)amino)quinazolin-6-yl)pyrazin-2-yl)-1-phenylmethane sulfonamide | 521.20 |
| 623 | 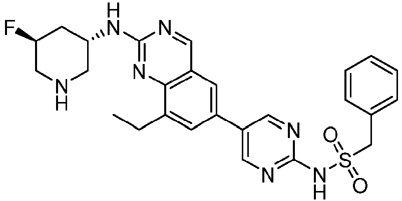 | *N*-(5-(8-ethyl-2-(((3S, *5S)-5-*fluoropiperidin-3-yl)amino)quinazolin-6-yl)pyrimidin-2-yl)-1-phenylmethane sulfonamide | 521.20 |
| 624 | 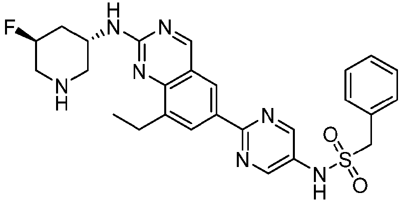 | *N*-(2-(8-ethyl-2-(((3S, *5S)-5-*fluoropiperidin-3-yl)amino)quinazolin-6-yl)pyrimidin-5-yl)-1-phenylmethane sulfonamide | 521.20 |
| 625 | 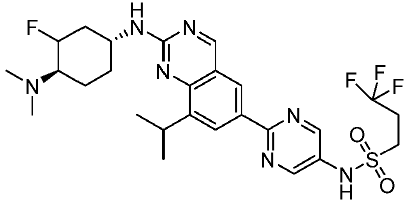 | *N*-(2-(2-(((*1R,4R*)-4-(dimethylamino)-3-fluorocyclohexyl)amino)-8-isopropyl quinazolin-6-yl)pyrimidin-5-yl)-3,3,3-trifluoropropane-1-sulfonamide | 583.24 |
| 626 | 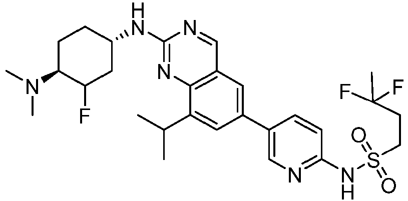 | *N-(*5-(2-(((*1S,4S*)-4-(dimethylamino)-3-fluorocyclohexyl)amino)-8-isopropyl quinazolin-6-yl)pyridin-2-yl)-3,3-difluorobutane-1-sulfonamide | 578.27 |
| 627 | 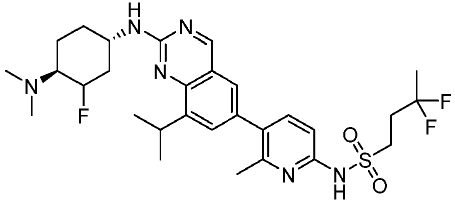 | *N*-(5-(2-(((*1S,4S*)-4-(dimethylamino)-3-fluorocyclohexyl)amino)-8-isopropyl quinazolin-6-yl)-6-methylpyridin-2-yl)-3,3-difluorobutane-1-sulfonamide | 592.28 |
| 628 | 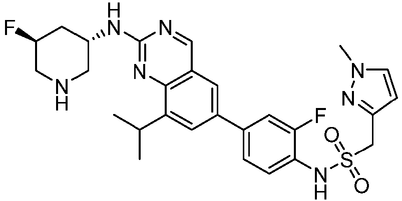 | *N*-(2-fluoro-4-(2-(((*3S,5S*)-5-fluoropiperidin-3-yl)amino)-8-isopropyl quinazolin-6-yl)phenyl)-1-(1-methyl-1H-pyrazol-3-yl)methanesulfonamide | 555.22 |
| 629 | 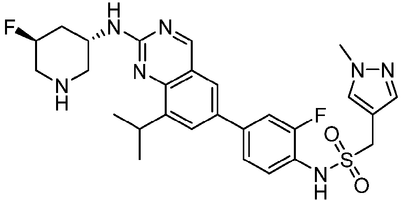 | *N*-(2-fluoro-4-(2-(((*3S,5S*)-5-fluoropiperidin-3-yl)amino)-8-isopropyl quinazolin-6-yl)phenyl)-1-(1-methyl-1H-pyrazol-4-yl)methanesulfonamide | 555.22 |
| 630 | 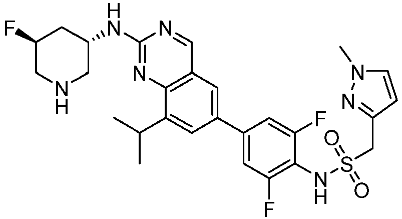 | *N*-(2,6-difluoro-4-(2-(((*3S,5S*)-5-fluoropiperidin-3-yl)amino)-8-isopropyl quinazolin-6-yl)phenyl)-1-(1-methyl-1H-pyrazol-3-yl)methanesulfonamide | 573.21 |
| 631 | 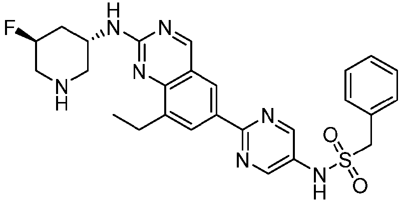 | *N*-(2-(8-ethyl-2-(((3S, *5S)-5-*fluoropiperidin-3-yl)amino)quinazolin-6-yl)pyrimidin-5-yl)-1-phenylmethane sulfonamide | 521.20 |
| 632 | 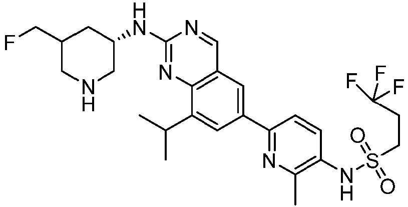 | 3,3,3-trifluoro-*N*-(6-(2-(((3S)-5-(fluoromethyl)piperidin-3-yl)amino)-8-isopropylquinazolin-6-yl)-2-methyl pyridin-3-yl)propane-1-sulfonamide | 568.64 |
| 633 | 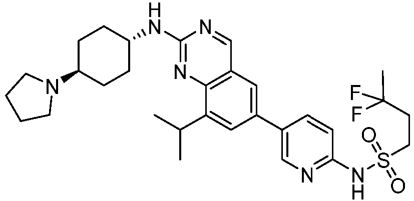 | 3,3-difluoro-*N*-(5-(8-isopropyl-2-(((*1r,4r*)-4-(pyrrolidin*-*1-yl)cyclohexyl) amino)quinazolin-6-yl)pyridin-2-yl)butane-1-sulfonamide | 586.29 |
| 634 | 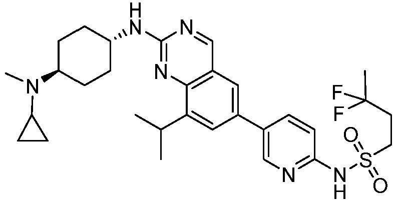 | *N*-(5-(2-(((*1r,4r*)-4-(cyclopropyl(methyl)amino)cyclohexyl)a mino)-8-isopropylquinazolin-6-yl)pyridin -2-yl)-3,3-difluorobutane-1-sulfonamide | 586.29 |
| 635 | 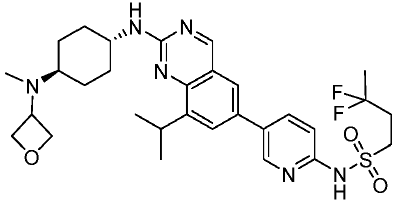 | 3,3-difluoro-*N*-(5-(8-isopropyl-2-(((*1r,4r*)-4-(methyl(oxetan-3-yl)amino) cyclohexyl)amino)quinazolin-6-yl)pyridin-2-yl)butane-1-sulfonamide | 602.29 |
| 636 | 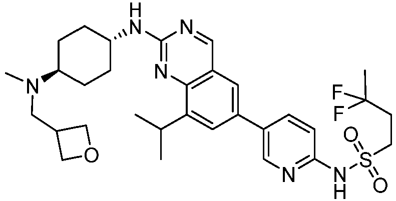 | 3,3-difluoro-*N*-(5-(8-isopropyl-2-(((*1r,4r*) -4-(methyl(oxetan-3-ylmethyl)amino) cyclohexyl)amino)quinazolin-6-yl)pyridin-2-yl)butane-1-sulfonamide | 616.30 |
| 637 | 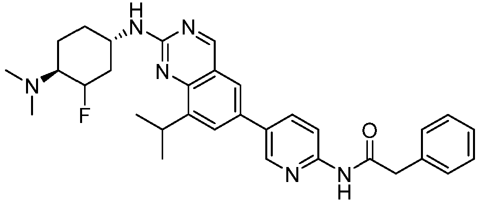 | N-(5-(2-(((1S,4S)-4-(dimethylamino)-3-fluorocyclohexyl)amino)-8-isopropyl quinazolin-6-yl)pyridin-2-yl)-2-phenylacetamide | 540.30 |
| 638 | 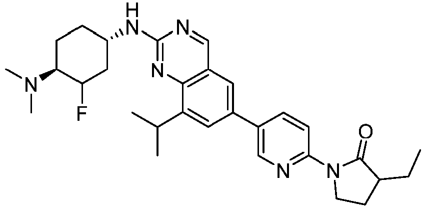 | 1-(5-(2-(((1S,4S)-4-(dimethylamino)-3-fluorocyclohexyl)amino)-8-isopropyl quinazolin-6-yl)pyridin-2-yl)-3-ethylpyrrolidin-2-one | 518.32 |
| 639 | 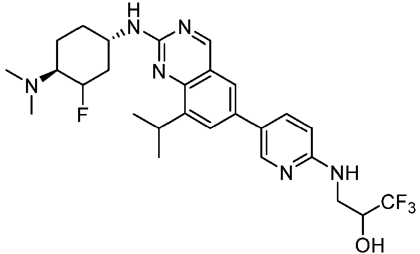 | 3-((5-(2-(((1S,4S)-4-(dimethylamino)-3-fluorocyclohexyl)amino)-8-isopropyl quinazolin-6-yl)pyridin-2-yl)amino)-1,1,1-trifluoropropan-2-ol | 534.27 |

### Synthesis of Compounds and Pharmaceutically Acceptable Salts Thereof

Compounds and pharmaceutically acceptable salts thereof as described herein can be synthesized by synthetic routes that include processes analogous to those well-known in the chemical arts, particularly in light of the description contained herein, and those for other heterocycles described in: Comprehensive Heterocyclic Chemistry II, Editors Katritzky and Rees, Elsevier, 1997, e.g. Volume 3; Liebigs Annalen der Chemie, (9):1910-16, (1985); Helvetica Chimica Acta, 41:1052-60, (1958); Arzneimittel-Forschung, 40(12):1328-31, (1990). Starting materials are generally available from commercial sources such as Aldrich Chemicals (Milwaukee, WI) or are readily prepared using methods (e.g., prepared by methods generally described in Louis F. Fieser and Mary Fieser, Reagents for Organic Synthesis, v. 1-23, Wiley, N.Y. (1967-2006 ed.), or Beilsteins Handbuch der organischen Chemie, 4, Aufl. ed. Springer-Verlag, Berlin, including supplements (also available via the Beilstein online database). Compounds and pharmaceutically acceptable salts thereof as described herein can also be made following the procedures found in US 8476434, US 7880000, WO 2005/113494, US 7868177, and WO 2007/100646.

Synthetic chemistry transformations and protecting group methodologies (protection and deprotection) useful in synthesizing compounds and pharmaceutically acceptable salts thereof as described herein and necessary reagents and intermediates include, for example, those described in R. Larock, Comprehensive Organic Transformations, VCH Publishers (1989); T. W. Greene and P. G. M. Wuts, Protective Groups in Organic Synthesis, 3rd Ed., John Wiley and Sons (1999); and L. Paquette, ed., Encyclopedia of Reagents for Organic Synthesis, John Wiley and Sons (1995) and subsequent editions thereof.

Compounds and pharmaceutically acceptable salts thereof as described herein can be prepared singly or as compound libraries comprising at least 2, for example 5 to 1,000 compounds, or 10 to 100 compounds. Libraries of compounds and pharmaceutically acceptable salts thereof as described herein can be prepared by a combinatorial split and mix approach or by multiple parallel syntheses using, for example, either solution phase or solid phase chemistry. Thus, disclosed herein is a compound library comprising at least 2 compounds, or pharmaceutically acceptable salts thereof as described herein.

The Examples provide exemplary methods for preparing compounds and pharmaceutically acceptable salts thereof as described herein. Those skilled in the art will appreciate that other synthetic routes can be used to synthesize compounds and pharmaceutically acceptable salts thereof as described herein. Although specific starting materials and reagents are depicted and discussed in the Examples, other starting materials and reagents can be easily substituted to provide a variety of derivatives and/or reaction conditions. In addition, many of the exemplary compounds or pharmaceutically acceptable salts thereof as prepared by the described methods can be further modified in light of this disclosure using conventional chemistry.

In preparing compounds and pharmaceutically acceptable salts thereof as described herein, protection of remote functionality (e.g., primary or secondary amine) of intermediates can be necessary. The need for such protection will vary depending on the nature of the remote functionality and the conditions of the preparation methods. Suitable amino-protecting groups include acetyl, trifluoroacetyl, t-butoxycarbonyl (BOC), benzyloxycarbonyl (CBz) and 9-fluorenylmethyleneoxycarbonyl (Fmoc). The need for such protection can be readily determined. For a general description of protecting groups and their use, see T. W. Greene, Protective Groups in Organic Synthesis, John Wiley & Sons, New York, 1991.

In the methods of preparing compounds and pharmaceutically acceptable salts thereof as described herein, it can be advantageous to separate reaction products from one another and/or from starting materials. The desired products of each step or series of steps is separated and/or purified to the desired degree of homogeneity by the techniques common in the art. Typically such separations involve multiphase extraction, crystallization from a solvent or solvent mixture, distillation, sublimation, or chromatography. Chromatography can involve any number of methods including, for example: reverse-phase and normal phase; size exclusion; ion exchange; high, medium and low pressure liquid chromatography methods and apparatus; small scale analytical; simulated moving bed (SMB) and preparative thin or thick layer chromatography, as well as techniques of small scale thin layer and flash chromatography.

Another class of separation methods involves treatment of a mixture with a reagent selected to bind to or render otherwise separable a desired product, unreacted starting material, reaction by product, or the like. Such reagents include adsorbents or absorbents such as activated carbon, molecular sieves, ion exchange media, or the like. Alternatively, the reagents can be acids in the case of a basic material, bases in the case of an acidic material, binding reagents such as antibodies, binding proteins, selective chelators such as crown ethers, liquid/liquid ion extraction reagents (LIX), or the like. Selection of appropriate methods of separation depends on the nature of the materials involved, such as, boiling point and molecular weight in distillation and sublimation, presence or absence of polar functional groups in chromatography, stability of materials in acidic and basic media in multiphase extraction, and the like.

Diastereomeric mixtures can be separated into their individual diastereomers on the basis of their physical chemical differences by methods such as by chromatography and/or fractional crystallization. Enantiomers can be separated by converting the enantiomeric mixture into a diastereomeric mixture by reaction with an appropriate optically active compound (e.g., chiral auxiliary such as a chiral alcohol or Mosher's acid chloride), separating the diastereomers and converting (e.g., hydrolyzing) the individual diastereoisomers to the corresponding pure enantiomers. Also, some of the compounds or pharmaceutically acceptable salts thereof described herein can be atropisomers (e.g., substituted biaryls). Enantiomers can also be separated by use of a chiral HPLC column.

A single stereoisomer, e.g., an enantiomer, substantially free of its stereoisomer can be obtained by resolution of the racemic mixture using a method such as formation of diastereomers using optically active resolving agents (Eliel, E. and Wilen, S. "Stereochemistry of Organic Compounds," John Wiley & Sons, Inc., New York, 1994; Lochmuller, C. H., (1975) J. Chromatogr., 113(3):283-302). Racemic mixtures of chiral compounds or pharmaceutically acceptable salts thereof described herein can be separated and isolated by any suitable method, including: (1) formation of ionic, diastereomeric salts with chiral compounds and separation by fractional crystallization or other methods, (2) formation of diastereomeric compounds with chiral derivatizing reagents, separation of the diastereomers, and conversion to the pure stereoisomers, and (3) separation of the substantially pure or enriched stereoisomers directly under chiral conditions. See: "Drug Stereochemistry, Analytical Methods and Pharmacology," Irving W. Wainer, Ed., Marcel Dekker, Inc., New York (1993).

Under method (1), diastereomeric salts can be formed by reaction of enantiomerically pure chiral bases such as brucine, quinine, ephedrine, strychnine, α-methyl-β-phenylethylamine (amphetamine), and the like with asymmetric compounds bearing acidic functionality, such as carboxylic acid and sulfonic acid. The diastereomeric salts can be induced to separate by fractional crystallization or ionic chromatography. For separation of the optical isomers of amino compounds, addition of chiral carboxylic or sulfonic acids, such as camphorsulfonic acid, tartaric acid, mandelic acid, or lactic acid can result in formation of the diastereomeric salts.

Alternatively, by method (2), the substrate to be resolved is reacted with one enantiomer of a chiral compound to form a diastereomeric pair (E. and Wilen, S. "Stereochemistry of Organic Compounds", John Wiley & Sons, Inc., 1994, p. 322). Diastereomeric compounds can be formed by reacting asymmetric compounds with enantiomerically pure chiral derivatizing reagents, such as methyl derivatives, followed by separation of the diastereomers and hydrolysis to yield the pure or enriched enantiomer. A method of determining optical purity involves making chiral esters, such as a methyl ester, e.g., (-) methyl chloroformate in the presence of base, or Mosher ester, α-methoxy-α-(trifluoromethyl)phenyl acetate (Jacob III. J. Org. Chem. (1982) 47:4165), of the racemic mixture, and analyzing the ¹H NMR spectrum for the presence of the two atropisomeric enantiomers or diastereomers. Stable diastereomers of atropisomeric compounds can be separated and isolated by normal- and reverse-phase chromatography following methods for separation of atropisomeric naphthyl-isoquinolines (WO 96/15111). By method (3), a racemic mixture of two enantiomers can be separated by chromatography using a chiral stationary phase ("Chiral Liquid Chromatography" (1989) W. J. Lough, Ed., Chapman and Hall, New York; Okamoto, J. Chromatogr., (1990) 513:375-378). Enriched or purified enantiomers can be distinguished by methods used to distinguish other chiral molecules with asymmetric carbon atoms, such as optical rotation and circular dichroism.

### Administration of Compounds

Compounds or pharmaceutically acceptable salts thereof described herein can be administered by any route appropriate to the condition to be treated. Suitable routes include oral, parenteral (including subcutaneous, intramuscular, intravenous, intraarterial, intradermal, intrathecal and epidural), transdermal, rectal, nasal, topical (including buccal and sublingual), vaginal, intraperitoneal, intrapulmonary and intranasal. For local immunosuppressive treatment, the compounds can be administered by intralesional administration, including perfusing or otherwise contacting the graft with the inhibitor before transplantation. It will be appreciated that the preferred route can vary with for example the condition of the recipient. Where the compound is administered orally, it can be formulated as a pill, capsule, tablet, etc. with a pharmaceutically acceptable carrier or excipient. In one preferred embodiment, the compound or pharmaceutically acceptable salt thereof is formulated for oral administration as a pill, a capsule, or a tablet. Where the compound or pharmaceutically acceptable salt thereof is administered parenterally, it can be formulated with a pharmaceutically acceptable parenteral vehicle and in a unit dosage injectable form, as detailed below.

Thus, in one embodiment provided herein is a pharmaceutical composition comprising a compound or pharmaceutically acceptable salt thereof as described herein and one or more pharmaceutically acceptable excipients. In one embodiment, compounds or pharmaceutically acceptable salts thereof described herein are administered as pharmaceutical compositions capable of being administered to a subject orally or parenterally. The compounds described herein can be formulated for topical or parenteral use where the compound is dissolved or otherwise suspended in a solution suitable for injections, suspensions, syrups, creams, ointments, gels, sprays, solutions and emulsions.

Oral administration can promote patient compliance in taking the compound (e.g. formulated as a pharmaceutical composition), thereby increasing compliance and efficacy. Oral pharmaceutical compositions comprising a compound or pharmaceutically acceptable salt thereof described herein include, but are not limited to, tablets (e.g. coated, non-coated and chewable) and capsules (e.g. hard gelatin capsules, soft gelatin capsules, enteric coated capsules, and sustained release capsules). Tablets can be prepared by direct compression, by wet granulation, or by dry granulation. Oral pharmaceutical compositions comprising a compound or pharmaceutically acceptable salt thereof described herein can be formulated for delayed or prolonged release. In one preferred embodiment, the oral pharmaceutical composition comprises a compound or pharmaceutically acceptable salt thereof formulated in a tablet.

A dose to treat human patients can range from about 10 mg to about 1000 mg of a compound or pharmaceutically acceptable salt thereof described herein. A typical dose can be about 100 mg to about 300 mg of the compound. A dose can be administered once a day (QD), twice per day (BID), or more frequently, depending on the pharmacokinetic and pharmacodynamic properties, including absorption, distribution, metabolism, and excretion of the particular compound. Administration as used herein refers to the frequency of dosing and not, for example, the number of individual units a patient described herein must take for a dose. Thus, in some embodiments, a patient may take two or more dosage units (e.g. two or more pills/tablets/capsules) QD. In addition, toxicity factors can influence the dosage and administration regimen. When administered orally, the pill, capsule, or tablet can be ingested daily or less frequently for a specified period of time. The regimen can be repeated for a number of cycles of therapy.

### Methods of Treatment

As already noted, references herein to methods of treatment are to be interpreted as references to the compounds and pharmaceutical compositions of the present invention for use in a method for treatment of the human or animal body by therapy. It is also noted that, in the present invention, the compound, salt, or composition for use in a method of treatingan IRE 1-related disease or disorder, is one in which the IRE1-related disease or disorder is cancer.

In general, compounds or pharmaceutically acceptable salts thereof can be useful for treating a patient having a disease or disorder arising from: abnormal cell growth, function, or behavior associated with the UPR pathway such as cancer; an immune disorder; cardiovascular disease; viral infection; inflammation; a metabolism/endocrine disorder; or a neurological disorder by administering an effective amount of a compound or pharmaceutically acceptable salt thereof described herein. In one example of such methods, compounds or pharmaceutically acceptable salts thereof are useful for treating a patient having an IRE1-related disease or disorder arising from: abnormal cell growth, function, or behavior associated with the UPR pathway such as cancer; an immune disorder; cardiovascular disease; viral infection; inflammation; a metabolism/endocrine disorder; or a neurological disorder by administering an effective amount of a compound or pharmaceutically acceptable salt thereof described herein.

Described herein are methods of treating an IRE1-related disease or disorder by administering to a patient having an IRE1-related disease or disorder as described herein, an effective amount of a compound or a pharmaceutically acceptable salt thereof described herein. In an embodiment of the present invention, a method of treating cancer is by administering to a patient having cancer an effective amount of a compound or pharmaceutically acceptable salt thereof described herein. The cancer is an IRE1-related disease or disorder.

The methods provided herein include treatment of solid tumors/cancers by administering an effective amount of a compound or pharmaceutically acceptable salt thereof described herein to a patient having a solid tumor/cancer provided herein. For example, administration of an effective amount of a compound or pharmaceutically acceptable salt thereof described herein can be performed for patients having breast cancer, ovary cancer, cervix cancer, prostate cancer, testis cancer, genitourinary tract cancer, esophagus cancer, larynx cancer, glioblastoma, neuroblastoma, stomach cancer, skin cancer, keratoacanthoma, lung cancer, epidermoid carcinoma, large cell cancer, non-small cell lung cancer (NSCLC), small cell carcinoma, lung adenocarcinoma, bone cancer, colon cancer, adenoma, pancreatic cancer, adenocarcinoma, thyroid cancer, follicular carcinoma, undifferentiated carcinoma, papillary carcinoma, seminoma, melanoma, sarcoma, bladder carcinoma, liver carcinoma and biliary passages, kidney carcinoma, buccal cavity cancer, naso-pharyngeal cancer, pharynx cancer, lip cancer, tongue cancer, mouth cancer, small intestine cancer, colon-rectum cancer, large intestine cancer, rectum cancer, bronchial cancer, hepatocellular cancer, gastric cancer, endometrial cancer, melanoma, renal cancer, urinary bladder cancer, uterine corpus cancer, and uterine cervix cancer.

In another embodiment, the methods provided herein include treatment of cancer by administering to a patient having cancer an effective amount of a compound or pharmaceutically acceptable salt thereof where the cancer comprises squamous cell carcinoma, small-cell lung cancer, non-small cell lung cancer (NSCLC), lung adenocarcinoma, squamous cell lung cancer, peritoneum cancer, hepatocellular cancer, stomach cancer, gastrointestinal cancer, esophageal cancer, pancreatic cancer, glioblastoma, cervical cancer, ovarian cancer, liver cancer, bladder cancer, breast cancer, colon cancer, rectal cancer, colorectal cancer, endometrial cancer, uterine cancer, salivary gland carcinoma, renal cancer, prostate cancer, vulval cancer, thyroid cancer, hepatocellular carcinoma (HCC), anal carcinoma, penile carcinoma, or head and neck cancer.

In certain embodiments, the cancer is breast cancer. The breast cancer can be Stage I, II, III, or IV as understood in the art. In one embodiment, the breast cancer is triple negative breast cancer (TNBC). In another embodiment, the breast cancer is Her2 negative breast cancer. In still another embodiment, the breast cancer is HR+ breast cancer.

Also provided herein are methods of treating a hematological cancer in a patient having such a hematological cancer by administering an effective amount of a compound or pharmaceutically acceptable salt thereof described herein. The hematological cancer can be, for example, lymphorrma, lymphocytic leukemia (acute (ALL) and chronic (CLL)), multiple myeloma (MM), acute myelogenous leukemia (AML), chronic myelogenous leukemia (CML), myelodysplastic syndrome (MDS), myeloproliferative disease (MPD), or non-Hodgkin lymphoma. In one embodiment, the methods herein include treating a patient having multiple myeloma (MM), acute myelogenous leukemia (AML), chronic myelogenous leukemia (CML), or myelodysplastic syndrome (MDS) by administering an effective amount of a compound or pharmaceutically acceptable salt thereof described herein.

In one embodiment is a method of treating MM by administering to a patient having MM an effective amount of compound or pharmaceutically acceptable salt thereof described herein. The MM can be stage I, II, III, or IV as understood in the art. In another embodiment is a method of treating AML by administering to a patient having AML an effective amount of a compound or pharmaceutically acceptable salt thereof described herein. The AML can be stage I, II, III, or IV as understood in the art. In another embodiment is a method of treating CML by administering to a patient having CML an effective amount of a compound or pharmaceutically acceptable salt thereof described herein. The CML can be stage I, II, III, or IV as understood in the art. In another embodiment is a method of treating MDS by administering to a patient having MDS an effective amount of a compound or pharmaceutically acceptable salt thereof described herein. It is further understood that such cancers can be relapsed or refractory as provided herein.

In one embodiment, the cancer is an IRE1-mediated cancer (i.e. a cancer having abnormal expression or activity of IRE1 relative to a control). In one embodiment, the IRE1-mediated cancer has increased expression of IRE1. In another embodiment, the IRE1-mediated cancer has increased activity of IRE1. Such increases can be measured against a control (e.g. against a patient having predetermined IRE1 function, expression, activity; or for example measure in a single patient before, during, or after treatment with a compound or pharmaceutically acceptable salt thereof described herein). Cancers as provided above include IRE1-mediated cancers.

The methods and uses described herein also include embodiments where a compound or pharmaceutically acceptable salt thereof is administered in combination with one or more additional therapeutic agent(s) selected from the group consisting of an anti-inflammatory agent, a corticosteroid, an immunomodulatory agent, anti-cancer agent as described herein, an apoptosis-enhancer, a neurotropic factor, an agent for treating cardiovascular disease, an agent for treating liver disease, an anti-viral agent, an agent for treating blood disorders, an agent for treating diabetes, an agent for treating metabolic disorders, an agent for treating autoimmune disorders, an agent for treating immunodeficiency disorders, and combinations thereof.

In one embodiment of the methods provided herein a compound or pharmaceutically acceptable salt thereof is administered in combination with one or more additional therapeutic agents comprising a corticosteroid, a proteasome inhibitor, an immunomodulatory agent, an anti-CD38 antibody, an anti-VEGF-A antibody, an anti-PD-1 antibody, an anti-PD-L1 antibody, or an anti-interleukin-6 antibody, or a combination thereof.

In another embodiment of the methods provided herein a compound or pharmaceutically acceptable salt thereof is administered in combination as described herein where the additional therapeutic agent is a corticosteroid, a proteasome inhibitor, an IMiD, an antibody, or a combination thereof.

In one embodiment, a compound or pharmaceutically acceptable salt thereof is administered in combination with a proteasome inhibitor. In one embodiment, the proteasome inhibitor comprises carfilzomib, bortezomib, or ixazomib. In one embodiment, a compound or pharmaceutically acceptable salt thereof is administered in combination with a IMiD, where the IMiD is lenalidomide or pomalidomide. In one embodiment of the methods provided herein a compound or pharmaceutically acceptable salt thereof is administered in combination with a corticosteroid where the corticosteroid comprises dexamethasone.

In another embodiment, a compound or pharmaceutically acceptable salt thereof is administered in combination with an anti-PD-L1 antibody. The anti-PD-L1 antibody can be avelumab, durvalumab, or atezolizumab. In still another embodiment, a compound or pharmaceutically acceptable salt thereof is administered in combination with an anti-PD-1 antibody. The anti-PD-1 antibody can be pembrolizumab or nivolumab.

The methods provided herein can also further comprise administration of radiotherapy. In certain embodiments, the radiotherapy can be administered before administration of a compound or pharmaceutically acceptable salt thereof described herein.

Further provided herein is a compound or pharmaceutically acceptable salt thereof described herein, for use in a method for treating an IRE1-related disease or disorder, where the IRE1-related disease or disorder is as set forth herein. In one embodiment the compound or pharmaceutically acceptable salt thereof as described herein is for use in a method of treating a cancer as set forth above. In a preferred embodiment, the cancer is MM, AML, CML, or MDS.

Further provided herein is a use of a compound or pharmaceutically acceptable salt thereof described herein in the manufacture of a medicament for the treatment of an IRE1-related disease or disorder, where the IRE1-related disease or disorder is as set forth herein. In one embodiment the IRE1-related disease or disorder is a cancer as set forth above. In a preferred embodiment, the cancer is MM, AML, CML, or MDS. It is to be understood that embodiments herein referring to a method (e.g. a method of treating) can further refer to a use or a compound for use as set forth herein.

The methods and uses described herein are also applicable to patients that have been previously treated with one or more therapies prior to receiving administration of a compound or pharmaceutically acceptable salt thereof described herein. It is well known in the art that patients may be treated with one or more treatment regimens - especially for hematological cancers such as those described herein. Cancers can be relapse or refractory (r/r) (e.g. a patient having rrMM, rrAML, rrCML, or rrMDS). A "refractory" cancer refers to cancer that progresses despite active treatment. A "relapse" cancer generally refers to cancer that occurs in the absence of therapy following successful treatment with one or more anticancer agents. Accordingly, in one embodiment provided herein are methods of treating r/r cancer (e.g. rrMM, rrAML, rrCML, or rrMDS) in a patient having such a cancer by administering a compound or pharmaceutically acceptable salt thereof described herein. Such methods can include co-administration with one or more anticancer agents described herein as set forth above.

Accordingly, in one embodiment, a patient may have been treated with one or more anticancer agents. In one particular embodiment, a patient has been treated with 2 or more anticancer agents as provided herein for the treatment of a hematological disease, such as for example MM or AML. In one embodiment, a patient treated according to the methods provided herein has been previously administered one or more proteasome inhibitors such as bortezomib, carfilzomib, or ixazomib. In one embodiment, a patient treated according to the methods provided herein has been previously administered one or more IMiDs such as thalidomide, lenalidomide, or pomalidomide. In another embodiment, a patient treated according to the methods provided herein has been previously administered chemotherapy (e.g. cytarbine, cladribine, fludarabine, mitoxantrone, etoposide, 6-TG, hydroxyurea, methotrexate, decitabine, or an anthracyclin). In another embodiment, a patient treated according to the methods provided herein has been previously administered one or more corticosteroids such as dexamethasone. Such corticosteroids are often administered with other anticancer agents as understood in the art. In still another embodiment, a patient treated according to the methods provided herein has been previously administered one or more antibodies such as, for example, daratumumab, gemtuzumab ozogamicin, atezolizumab, alemtuzumab, rituximab, obinutuzumab, or ofatumumab. In still another embodiment, a patient treated according to the methods provided herein has been previously administered one or more FLT3 inhibitor (e.g. midostaurin or gilteritinib). In yet another embodiment, a patient treated according to the methods provided herein has been previously administered one or more Bcl-2 inhibitors such as venetoclax or navitoclax. In yet another embodiment, a patient treated according to the methods provided herein has been previously administered one or more of ibrutinib, idelalisib, or duvelisib. In another embodiment, a patient treated according to the methods provided herein has been previously administered an IMiD as described herein in combination with a proteasome inhibitor and optionally a corticosteroid.

A compound or pharmaceutically acceptable salt thereof described herein can be administered as a first line (1L) therapy (e.g. administration prior to administration of another anticancer agent, including chemotherapy). Thus, in certain instances a patient may be chemotherapy naive.

It is understood that the methods described herein include administration of a pharmaceutical composition comprising a compound or pharmaceutically acceptable salt thereof as provided herein. Such pharmaceutical compositions also comprise one or more pharmaceutically acceptable carrier excipients. In some embodiments, the compound is selected from Table 1 or Table 2, or a pharmaceutically acceptable salt thereof. In one embodiment, the compound or pharmaceutically acceptable salt thereof is one set forth in Table 1. In one embodiment, the compound or pharmaceutically acceptable salt thereof is one set forth in Table 2.

More generally, also disclosed herein is a method of treating a disease caused by abnormal levels of IRE1 activity in a human or animal patient in need of such treatment with a compound or a pharmaceutically acceptable salt thereof described herein. The disease can be caused by an amount of IRE1 activity that is too low or too high. For example, the disease can be caused by a deficiency in IRE1 activity or by abnormally high IRE1 activity (e.g., hyperactivity of IRE1). The method includes administering to the patient an effective amount of a compound or a pharmaceutically acceptable salt thereof described herein that modulates IRE1 activity (an IRE1 modulator compound).

IRE1 deficiency can be measured as a decreased amount of IRE1 activity compared to normal levels of IRE1 activity in a particular subject or a population of healthy subjects. The decreased amount of IRE1 activity results in excessive amounts of misfolded protein accumulation thereby causing the disease state.

IRE1 hyperactivity can be measured as an increased amount of IRE1 activity compared to normal levels of IRE1 activity in a particular subject or a population of healthy subjects. The increased amount of IRE1 activity can result in, for example, excessive amounts of cell proliferation thereby causing the disease state.

In some instances of this disclosure, the disease is associated with IRE1 deficiency. Such diseases include, but are not limited to, cystic fibrosis, retinitis pigmentosa, diabetes, or a neurodegenerative disease. The neurodegenerative disease can include Alexander's disease, Alper's disease, Alzheimer's disease, Amyotrophic lateral sclerosis, Ataxia telangiectasia, Batten disease (also known as Spielmeyer-Vogt-Sjogren-Batten disease). Bovine spongiform encephalopathy (BSF), Canavan disease, Cockayne syndrome, Corticobasal degeneration, Creutzfeldt-Jakob disease, Huntington's disease, HIV-associated dementia, Kennedy's disease, Krabbe's disease, Lewy body dementia, Machado-Joseph disease (Spinocerebellar ataxia type 3), Multiple sclerosis, Multiple System Atrophy, Narcolepsy, Neuroborreliosis, Parkinson's disease, Pelizaeus-Merzbacher Disease, Pick's disease, Primary lateral sclerosis, Prion diseases, Refsum's disease, Sandhoff s disease, Schilder's disease, Subacute combined degeneration of spinal cord secondary to Pernicious Anaemia, Schizophrenia, Spinocerebellar ataxia (multiple types with varying characteristics), Spinal muscular atrophy, Steele-Richardson-Olszewski disease, or Tabes dorsalis.

In other instances, the disease is associated with abnormally high IRE1. Such diseases include, but are not limited, to cancers (i.e., in accordance with the present invention), inflammatory diseases, and autoimmune diseases. Exemplary cancers include, but am not limited to, breast cancer and multiple myeloma. In one embodiment, the disease is multiple myeloma. In one embodiment, the disease is a triple-negative breast cancer. Exemplary inflammatory diseases include, but are not limited to, asthma, chronic inflammation, chronic prostatitis, glomerulonephritis, hypersensitivities, inflammatory bowel diseases, pelvic inflammatory disease; reperfusion injury, rheumatoid arthritis, transplant rejection, and vasculitis. Exemplary autoimmune diseases include, but are not limited to, XBP1-linked Crohn's disease, Coeliac disease, diabetes mellitus type 1 (IDDM), systemic lupus erythematosus (SLE), Sjogren's syndrome, Churg-Strauss Syndrome, Hashimoto's thyroiditis, Graves' disease, idiopathic thrombocytopenic purpura, and rheumatoid arthritis. In one instance, the disease is XBP1-linked. Crohn's disease.

### Pharmaceutical Formulations

Compounds or pharmaceutically acceptable salts thereof as described herein can be formulated in accordance with standard pharmaceutical practice as a pharmaceutical composition. Thus, further provided herein is a pharmaceutical composition comprising a compound or pharmaceutically acceptable salt thereof and one or more pharmaceutically acceptable excipients.

A typical formulation is prepared by mixing a compound or pharmaceutically acceptable salt thereof as described herein and an excipient. Suitable carriers, diluents and excipients include, but are not limited to, materials such as carbohydrates, waxes, water soluble and/or swellable polymers, hydrophilic or hydrophobic materials, gelatin, oils, solvents, water and the like. The particular excipient used will depend upon the means and purpose for which the compound or pharmaceutically acceptable salt thereof as described herein is being applied. Solvents are generally selected based on solvents recognized as safe (GRAS) to be administered to a mammal. In general, safe solvents are non-toxic aqueous solvents such as water and other non-toxic solvents that are soluble or miscible in water. Suitable aqueous solvents include water, ethanol, propylene glycol, polyethylene glycols (e.g., PEG 400, PEG 300), etc. and mixtures thereof. The formulations can also include one or more buffers, stabilizing agents, surfactants, wetting agents, lubricating agents, emulsifiers, suspending agents, preservatives, antioxidants, opaquing agents, glidants, processing aids, colorants, sweeteners, perfuming agents, flavoring agents and other known additives to provide an elegant presentation of the drug (i.e., a compound described herein or pharmaceutical composition thereof) or aid in the manufacturing of the pharmaceutical product (i.e., medicament).

The formulations can be prepared using conventional dissolution and mixing procedures. For example, the bulk drug substance (i.e., compound or pharmaceutically acceptable salt thereof as described herein or stabilized form thereof (e.g., complex with a cyclodextrin derivative or other known complexation agent) is dissolved in a suitable solvent in the presence of one or more of the excipients described above. The compound or pharmaceutically acceptable salt thereof as described herein is typically formulated into pharmaceutical dosage forms to provide an easily controllable dosage of the drug and to enable patient compliance with the prescribed regimen.

The pharmaceutical composition (or formulation) for application can be packaged in a variety of ways depending upon the method used for administering the drug. Generally, an article for distribution includes a container having deposited therein the pharmaceutical formulation in an appropriate form. Suitable containers include materials such as bottles (plastic and glass), sachets, ampoules, plastic bags, metal cylinders, and the like. The container can also include a tamper-proof assemblage to prevent indiscreet access to the contents of the package. In addition, the container has deposited thereon a label that describes the contents of the container. The label can also include appropriate warnings.

Pharmaceutical formulations of the compounds or pharmaceutically acceptable salts thereof as described herein can be prepared for various routes and types of administration. For example, a compound or pharmaceutically acceptable salt thereof as described herein having the desired degree of purity can optionally be mixed with one or more pharmaceutically acceptable excipients (Remington's Pharmaceutical Sciences (1980) 16th edition, Osol, A. Ed.), in the form of a lyophilized formulation, milled powder, or an aqueous solution. Formulation can be conducted by mixing at ambient temperature at the appropriate pH, and at the desired degree of purity, with physiologically acceptable carriers, i.e., carriers that are non-toxic to recipients at the dosages and concentrations employed. The pH of the formulation depends mainly on the particular use and the concentration of compound, but can range from about 3 to about 8. For example, formulation in an acetate buffer at pH 5 can be a suitable embodiment.

The pharmaceutical composition ordinarily can be stored as a solid composition, a lyophilized formulation or as an aqueous solution.

The pharmaceutical compositions described herein can be formulated, dosed and administered in a fashion, i.e., amounts, concentrations, schedules, course, vehicles and route of administration, consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The effective amount of the compound to be administered will be governed by such considerations, and is the minimum amount necessary to ameliorate, or treat the hyperproliferative disorder.

As a general proposition, the initial pharmaceutically effective amount of the inhibitor administered parenterally per dose will be in the range of about 0.01-100 mg/kg, namely about 0.1 to 20 mg/kg of patient body weight per day, with the typical initial range of compound used being 0.3 to 15 mg/kg/day. In another embodiment, a pharmaceutical composition described herein comprises an effective amount of a compound or pharmaceutically acceptable salt thereof in an amount of about: 1mg-10mg; 10mg-25mg; 20mg-50mg; 50mg-75mg; 70mg-100mg;100mg-150mg; 100mg-200mg; 100mg-500mg; 200mg-500mg; 250mg-500mg; 500mg-1000mg; or 750mg-1000mg.

Acceptable pharmaceutically acceptable excipients are nontoxic to recipients at the dosages and concentrations employed, and include buffers such as phosphate, citrate and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as TWEEN^{™}, PLURONICS^{™} or polyethylene glycol (PEG). The active pharmaceutical ingredients can also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

Sustained-release preparations of compounds or pharmaceutically acceptable salts thereof as described herein may be prepared. Suitable examples of sustained-release preparations include semipermeable matrices of solid hydrophobic polymers containing a compound or pharmaceutically acceptable salt thereof as described herein , which matrices are in the form of shaped articles, e.g., films, or microcapsules. Examples of sustained-release matrices include polyesters, hydrogels (for example, poly(2-hydroxyethyl-methacrylate), or poly(vinyl alcohol)), polylactides (US 3773919), copolymers of L-glutamic acid and gamma-ethyl-L-glutamate, nondegradable ethylene-vinyl acetate, degradable lactic acid-glycolic acid copolymers such as the LUPRON DEPOT^{™} (injectable microspheres composed of lactic acid-glycolic acid copolymer and leuprolide acetate) and poly-D-(-)-3-hydroxybutyric acid.

The formulations include those suitable for the administration routes detailed herein. The formulations can conveniently be presented in unit dosage form and can be prepared by any methods. Techniques and formulations generally are found in Remington's Pharmaceutical Sciences (Mack Publishing Co., Easton, PA). Such methods include the step of bringing into association the active ingredient with the carrier which constitutes one or more accessory ingredients. In general the formulations are prepared by uniformly and intimately bringing into association the active ingredient with liquid carriers or finely divided solid carriers or both, and then, if necessary, shaping the product.

Formulations of a compound or pharmaceutically acceptable salt thereof as described herein suitable for oral administration can be prepared as discrete units such as pills, capsules, cachets or tablets each containing a predetermined amount of such compound or pharmaceutically acceptable salt thereof. Compressed tablets can be prepared by compressing in a suitable machine the active ingredient in a free-flowing form such as a powder or granules, optionally mixed with a binder, lubricant, inert diluent, preservative, surface active or dispersing agent. Molded tablets can be made by molding in a suitable machine a mixture of the powdered active ingredient moistened with an inert liquid diluent. The tablets can optionally be coated or scored and optionally are formulated so as to provide slow or controlled release of the active ingredient therefrom. Tablets, troches, lozenges, aqueous or oil suspensions, dispersible powders or granules, emulsions, hard or soft capsules, e.g., gelatin capsules, syrups or elixirs can be prepared for oral use. Formulations of compounds or pharmaceutically acceptable salts thereof as described herein intended for oral use can be prepared according to any method for the manufacture of pharmaceutical compositions and such compositions can contain one or more agents including sweetening agents, flavoring agents, coloring agents and preserving agents, in order to provide a palatable preparation. Tablets containing the active ingredient in admixture with non-toxic pharmaceutically acceptable excipient which are suitable for manufacture of tablets are acceptable. These excipients can be, for example, inert diluents, such as calcium or sodium carbonate, lactose, calcium or sodium phosphate; granulating and disintegrating agents, such as maize starch, or alginic acid; binding agents, such as starch, gelatin or acacia; and lubricating agents, such as magnesium stearate, stearic acid or talc. Tablets can be uncoated or can be coated by known techniques including microencapsulation to delay disintegration and adsorption in the gastrointestinal tract and thereby provide a sustained action over a longer period. For example, a time delay material such as glyceryl monostearate or glyceryl distearate alone or with a wax can be employed.

For treatment of the eye or other external tissues, e.g., mouth and skin, the formulations are preferably applied as a topical ointment or cream containing the active ingredient(s) in an amount of, for example, 0.075 to 20% w/w. When formulated in an ointment, the active ingredients can be employed with either a paraffinic or a water-miscible ointment base. Alternatively, the active ingredients can be formulated in a cream with an oil-in-water cream base. If desired, the aqueous phase of the cream base can include a polyhydric alcohol, i.e., an alcohol having two or more hydroxyl groups such as propylene glycol, butane 1,3-diol, mannitol, sorbitol, glycerol and polyethylene glycol (including PEG 400) and mixtures thereof. The topical formulations can desirably include a compound which enhances absorption or penetration of the active ingredient through the skin or other affected areas. Examples of such dermal penetration enhancers include dimethyl sulfoxide and related analogs. The oily phase of the emulsions of compositions provided herein can be constituted from known ingredients in a known manner. While the phase can comprise merely an emulsifier, it desirably comprises a mixture of at least one emulsifier with a fat or an oil or with both a fat and an oil. Preferably, a hydrophilic emulsifier is included together with a lipophilic emulsifier which acts as a stabilizer. It is also preferred to include both an oil and a fat. Together, the emulsifier(s) with or without stabilizer(s) make up the so-called emulsifying wax, and the wax together with the oil and fat make up the so-called emulsifying ointment base which forms the oily dispersed phase of the cream formulations. Emulsifiers and emulsion stabilizers suitable for use in the formulation of described herein include Tween^{®} 60, Span^{®} 80, cetostearyl alcohol, benzyl alcohol, myristyl alcohol, glyceryl mono-stearate and sodium lauryl sulfate.

Aqueous suspensions comprising compounds or pharmaceutically acceptable salts thereof as described herein can contain the active materials in admixture with excipients suitable for the manufacture of aqueous suspensions. Such excipients include a suspending agent, such as sodium carboxymethylcellulose, croscarmellose, povidone, methylcellulose, hydroxypropyl methylcellulose, sodium alginate, polyvinylpyrrolidone, gum tragacanth and gum acacia, and dispersing or wetting agents such as a naturally occurring phosphatide (e.g., lecithin), a condensation product of an alkylene oxide with a fatty acid (e.g., polyoxyethylene stearate), a condensation product of ethylene oxide with a long chain aliphatic alcohol (e.g., heptadecaethyleneoxycetanol), a condensation product of ethylene oxide with a partial ester derived from a fatty acid and a hexitol anhydride (e.g., polyoxyethylene sorbitan monooleate). The aqueous suspension can also contain one or more preservatives such as ethyl or n-propyl p-hydroxybenzoate, one or more coloring agents, one or more flavoring agents and one or more sweetening agents, such as sucrose or saccharin.

The pharmaceutical compositions of compounds or pharmaceutically acceptable salts thereof as described herein can be in the form of a sterile injectable preparation, such as a sterile injectable aqueous or oleaginous suspension. This suspension can be formulated using suitable dispersing or wetting agents and suspending agents which have been mentioned above. The sterile injectable preparation can also be a sterile injectable solution or suspension in a non-toxic parenterally acceptable diluent or solvent, such as a solution in 1,3-butanediol or prepared as a lyophilized powder. Among the acceptable vehicles and solvents that can be employed are water, Ringer's solution and isotonic sodium chloride solution. In addition, sterile fixed oils can conventionally be employed as a solvent or suspending medium. For this purpose any bland fixed oil can be employed including synthetic mono- or diglycerides. In addition, fatty acids such as oleic acid can likewise be used in the preparation of injectables.

The amount of active ingredient that can be combined with the carrier material to produce a single dosage form will vary depending upon the host treated and the particular mode of administration. For example, a time-release formulation intended for oral administration to humans can contain approximately 1 to 1000 mg of active material compounded with an appropriate and convenient amount of carrier material which can vary from about 5 to about 95% of the total compositions (weight: weight). The pharmaceutical composition can be prepared to provide easily measurable amounts for administration. For example, an aqueous solution intended for intravenous infusion can contain from about 3 to 500 µg of the active ingredient per milliliter of solution in order that infusion of a suitable volume at a rate of about 30 mL/hr can occur.

Formulations suitable for parenteral administration include aqueous and non-aqueous sterile injection solutions which can contain anti-oxidants, buffers, bacteriostats and solutes which render the formulation isotonic with the blood of the intended recipient; and aqueous and non-aqueous sterile suspensions which can include suspending agents and thickening agents.

Formulations suitable for topical administration to the eye also include eye drops wherein the active ingredient is dissolved or suspended in a suitable carrier, especially an aqueous solvent for the active ingredient. The active ingredient is preferably present in such formulations in a concentration of about 0.5 to 20% w/w, for example about 0.5 to 10% w/w, for example about 1.5% w/w.

Formulations suitable for topical administration in the mouth include lozenges comprising the active ingredient in a flavored basis, usually sucrose and acacia or tragacanth; pastilles comprising the active ingredient in an inert basis such as gelatin and glycerin, or sucrose and acacia; and mouthwashes comprising the active ingredient in a suitable liquid carrier.

Formulations for rectal administration can be presented as a suppository with a suitable base comprising for example cocoa butter or a salicylate.

Formulations suitable for intrapulmonary or nasal administration have a particle size for example in the range of 0.1 to 500 microns (including particle sizes in a range between 0.1 and 500 microns in increments microns such as 0.5, 1, 30 microns, 35 microns, etc.), which is administered by rapid inhalation through the nasal passage or by inhalation through the mouth so as to reach the alveolar sacs. Suitable formulations include aqueous or oily solutions of the active ingredient. Formulations suitable for aerosol or dry powder administration can be prepared according to conventional methods and can be delivered with other therapeutic agents such as compounds heretofore used in the treatment or prophylaxis disorders as described below.

Formulations suitable for vaginal administration can be presented as pessaries, tampons, creams, gels, pastes, foams or spray formulations containing in addition to the active ingredient such carriers considered to be appropriate.

The formulations can be packaged in unit-dose or multi-dose containers, for example sealed ampoules and vials, and can be stored in a freeze-dried (lyophilized) condition requiring only the addition of the sterile liquid carrier, for example water, for injection immediately prior to use. Extemporaneous injection solutions and suspensions are prepared from sterile powders, granules and tablets of the kind previously described. Preferred unit dosage formulations are those containing a daily dose or unit daily sub-dose, as herein above recited, or an appropriate fraction thereof, of the active ingredient.

The compounds or pharmaceutically acceptable salts thereof as described herein can be used in veterinary compositions comprising at least one active ingredient as above defined together with a veterinary carrier. Veterinary carriers are materials useful for the purpose of administering the composition and can be solid, liquid or gaseous materials which are otherwise inert or acceptable in the veterinary field and are compatible with the active ingredient. These veterinary compositions can be administered parenterally, orally or by any other desired route.

### Combination Therapy

The compounds and pharmaceutically acceptable salts thereof described herein can be employed alone or in combination with additional therapeutic agents for the treatment of a disease or disorder described herein, such as inflammation or a hyperproliferative disorder (e.g., cancer). In certain embodiments, a compound or a pharmaceutically acceptable salt thereof as described herein is combined in a pharmaceutical combination formulation, or dosing regimen as combination therapy, with an additional, second therapeutic compound that has anti-inflammatory or anti-hyperproliferative properties or that is useful for treating an inflammation, immune-response disorder, or hyperproliferative disorder (e.g., cancer, as in the present invention). The additional therapeutic can be a Bcl-2 inhibitor, a JAK inhibitor, a PI3K inhibitor, an mTOR inhibitor, an anti-inflammatory agent, an immunomodulatory agent, anti-cancer agent as described herein, an apoptosis-enhancer, a neurotropic factor, an agent for treating cardiovascular disease, an agent for treating liver disease, an anti-viral agent, an agent for treating blood disorders, an agent for treating diabetes, and an agent for treating immunodeficiency disorders. The second therapeutic agent can be an NSAID anti-inflammatory agent. The second therapeutic agent can be an anti-cancer agent as described herein. The second compound of the pharmaceutical combination formulation or dosing regimen preferably has complementary activities to the compound or pharmaceutically acceptable salt thereof described herein such that they do not adversely affect each other. Such compounds are suitably present in combination in amounts that are effective for the purpose intended. In one embodiment, a composition provided herein comprises a compound or a stereoisomer, tautomer, solvate, metabolite, or pharmaceutically acceptable salt thereof, in combination with a therapeutic agent such as an NSAID.

The combination therapy can be administered as a simultaneous or sequential regimen. When administered sequentially, the combination can be administered in two or more administrations. The combined administration includes coadministration, using separate formulations or a single pharmaceutical formulation, and consecutive administration in either order, wherein preferably there is a time period while both (or all) active agents simultaneously exert their biological activities.

Suitable dosages for any of the above coadministered agents are those presently used and can be lowered due to the combined action (synergy) of the newly identified agent and other therapeutic agents or treatments.

The combination therapy can provide "synergy" and prove "synergistic", i.e., the effect achieved when the active ingredients used together is greater than the sum of the effects that results from using the compounds separately. A synergistic effect can be attained when the active ingredients are: (1) co-formulated and administered or delivered simultaneously in a combined, unit dosage formulation; (2) delivered by alternation or in parallel as separate formulations; or (3) by some other regimen. When delivered in alternation therapy, a synergistic effect can be attained when the compounds are administered or delivered sequentially, e.g., by different injections in separate syringes, separate pills or capsules, or separate infusions. In general, during alternation therapy, an effective dosage of each active ingredient is administered sequentially, i.e., serially, whereas in combination therapy, effective dosages of two or more active ingredients are administered together.

In a particular embodiment of therapy, a compound or a or pharmaceutically acceptable salt thereof described herein can be combined with other therapeutic, hormonal or antibody agents such as those described herein, as well as combined with surgical therapy and radiotherapy. Combination therapies provided herein thus comprise the administration of at least one compound of or pharmaceutically acceptable salt thereof described herein, and the use of at least one other cancer treatment method as provided herein. The amounts of the compound(s) or pharmaceutically acceptable salts thereof described herein, and the other pharmaceutically active therapeutic agent(s) and the relative timings of administration will be selected in order to achieve the desired combined therapeutic effect.

In some embodiments, a compound or a pharmaceutically acceptable salt thereof described herein, is used in combination with an aromatase inhibitor, a phosphoinositide 3-kinase (PI3K)/mTOR pathway inhibitor, a CDK 4/6 inhibitor, a HER-2 inhibitor, a SERM, a SERD, an EGFR inhibitor, a PD-1 inhibitor, poly ADP-ribose polymerase (PARP) inhibitor, a histone deacetylase (HDAC) inhibitor, an HSP90 inhibitor, a VEGFR inhibitor, an AKT inhibitor, chemotherapy, or any combination thereof.

In some embodiments, a pharmaceutical composition comprising a compound or a pharmaceutically acceptable salt thereof described herein, is administered in combination with a therapeutic agent selected from paclitaxel, anastrozole, exemestane, cyclophosphamide, epirubicin, fulvestrant, letrozole, palbociclib, gemcitabine, trastuzumab (HERCEPTIN^{®}, Genentech), trastuzumab emtansine (KADCYLA^{®}, Genentech), pegfilgrastim, filgrastim, tamoxifen, docetaxel, toremifene, vinorelbine, capecitabine, and ixabepilone.

In some embodiments, a compound or a pharmaceutically acceptable salt thereof described herein, is used in combination with hormone blocking therapy, chemotherapy, radiation therapy, monoclonal antibodies, or combinations thereof.

Also provided herein are methods of inhibiting or killing a cancer cell expressing Ire1 by contacting the cancer cell expressing Ire1 with a compound or pharmaceutically acceptable salt thereof described herein. In one embodiment of the methods, the contacting is performed in vivo (e.g. the contacting is a result of administration of a compound or pharmaceutically acceptable salt thereof described herein). Thus, in another embodiment of the methods the inhibition or killing of the cancer cell occurs in vivo. In still another embodiment, the cancer cell expressing IRE1 is in a human patient described herein.

### Metabolites of Compounds of Described Herein

Also disclosed herein are *in vivo* metabolic products of compounds or pharmaceutically acceptable salts thereof described herein. Such products can result for example from the oxidation, reduction, hydrolysis, amidation, deamidation, esterification, deesterification, enzymatic cleavage, and the like, of the administered compound. Accordingly, disclosed herein are compounds produced by a process comprising contacting a compound or pharmaceutically acceptable salt thereof described herein with a mammal for a period of time sufficient to yield a metabolic product thereof.

Metabolite products typically are identified by preparing a radiolabelled (e.g., ¹⁴C or ³H) isotope of a compound or pharmaceutically acceptable salt thereof as described herein , administering it parenterally in a detectable dose (e.g., greater than about 0.5 mg/kg) to an animal such as rat, mouse, guinea pig, monkey, or to man, allowing sufficient time for metabolism to occur (typically about 30 seconds to 30 hours) and isolating its conversion products from the urine, blood or other biological samples. These products are easily isolated since they are labeled (others are isolated by the use of antibodies capable of binding epitopes surviving in the metabolite). The metabolite structures are determined in conventional fashion, e.g., by MS, LC/MS or NMR analysis. In general, analysis of metabolites is done in the same way as conventional drug metabolism studies. The metabolite products, so long as they are not otherwise found in vivo, are useful in diagnostic assays for therapeutic dosing of the compounds or pharmaceutically acceptable salts thereof described herein.

### Articles of Manufacture

Also provided herein is an article of manufacture, or kit, containing materials useful for the treatment of the diseases and disorders described above (e.g. cancer, as in the present invention). In one embodiment, the kit comprises a container comprising a compound or pharmaceutically acceptable salt thereof described herein. The kit can further comprise a label or package insert on or associated with the container. Suitable containers include, for example, bottles, vials, syringes, blister pack, etc. The container can be formed from a variety of materials known in the art such as metal, glass, or plastic. The container can hold a compound or pharmaceutically acceptable salt thereof or a formulation thereof which is effective for treating the condition and can have a sterile access port (for example, the container can be an intravenous solution bag or a vial having a stopper pierceable by a hypodermic injection needle). At least one active agent in the composition is a compound or a pharmaceutically acceptable salt thereof described herein. The label or package insert indicates that the composition is used for treating the condition of choice, such as cancer. In addition, the label or package insert can indicate that the patient to be treated is one having a disorder such as a hyperproliferative disorder, atherosclerosis, neurodegeneration, cardiac hypertrophy, pain, migraine or a neurotraumatic disease or event. In one embodiment, the label or package inserts indicates that the composition comprising a compound or pharmaceutically acceptable salt thereof described herein can be used to treat a disorder resulting from abnormal cell growth. In one embodiment, the label or package inserts indicates that the composition comprising a compound or pharmaceutically acceptable salt thereof described herein can be used to treat a disorder resulting from atherosclerosis. The label or package insert can also indicate that the composition can be used to treat other disorders. Alternatively, or additionally, the article of manufacture can further comprise a second container comprising a pharmaceutically acceptable buffer, such as bacteriostatic water for injection (BWFI), phosphate-buffered saline, Ringer's solution and dextrose solution. It can further include other materials desirable from a commercial and user standpoint, including other buffers, diluents, filters, needles, and syringes.

The kit can further comprise directions for the administration of the compound or pharmaceutically acceptable salt thereof described herein and, if present, the second pharmaceutical formulation. For example, if the kit comprises a first composition comprising a compound or pharmaceutically acceptable salt thereof described herein and a second pharmaceutical formulation, the kit can further comprise directions for the simultaneous, sequential or separate administration of the first and second pharmaceutical compositions to a patient in need thereof (e.g. according to the co-administration routes discussed herein). Accordingly, in one embodiment is a kit for treating a condition mediated by IRE1 where the kit comprises a compound or pharmaceutically acceptable salt thereof (formulated as a pharmaceutical composition described herein) and instructions for use.

In another embodiment, the kits are suitable for the delivery of solid oral forms of a compound or pharmaceutically acceptable salt thereof described herein, such as tablets or capsules. Such a kit preferably includes a number of unit dosages. Such kits can include a card having the dosages oriented in the order of their intended use. An example of such a kit is a blister pack. Blister packs are well known in the packaging industry and are widely used for packaging pharmaceutical unit dosage forms. If desired, a memory aid can be provided, for example in the form of numbers, letters, or other markings or with a calendar insert, designating the days in the treatment schedule in which the dosages can be administered.

According to one embodiment, a kit can comprise (a) a first container with a compound or pharmaceutically acceptable salt thereof described herein contained therein; and optionally (b) a second container with a second pharmaceutical formulation contained therein, wherein the second pharmaceutical formulation comprises a second compound with anti-hyperproliferative activity. Alternatively, or additionally, the kit can further comprise a third container comprising a pharmaceutically-acceptable buffer, such as bacteriostatic water for injection (BWFI), phosphate-buffered saline, Ringer's solution and dextrose solution. It can further include other materials desirable from a commercial and user standpoint, including other buffers, diluents, filters, needles, and syringes.

In certain other embodiments wherein the kit comprises a composition comprising a compound or a pharmaceutically acceptable salt thereof described herein and a second therapeutic agent, the kit can comprise a container for containing the separate compositions such as a divided bottle or a divided foil packet, however, the separate compositions can also be contained within a single, undivided container. Typically, the kit comprises directions for the administration of the separate components. The kit form is particularly advantageous when the separate components are preferably administered in different dosage forms (e.g., oral and parenteral), are administered at different dosage intervals, or when titration of the individual components of the combination is desired by the prescribing physician.

### Embodiments:

Embodiment 1: A compound having formula (A): 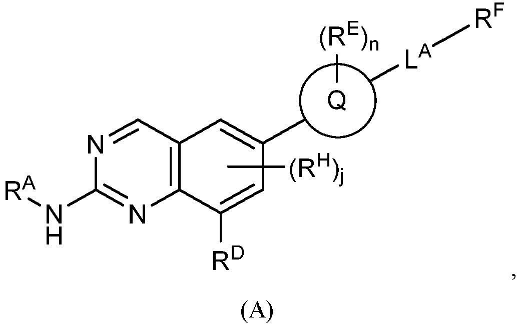 or a stereoisomer, tautomer, or pharmaceutically acceptable salt thereof, wherein:
Ring Q is R^{E}-substituted or unsubstituted C₅₋₇ aryl, or R^{E}-substituted or unsubstituted 6 membered heteroaryl comprising at least one nitrogen heteroatom;
L^{A} is -NHSO₂-, -SO₂NH-, or -NH-;
R^{A} is 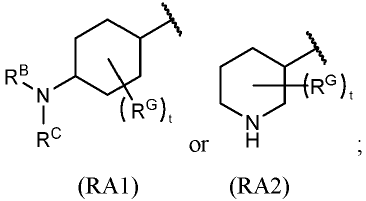
R^{B} and R^{C} are independently hydrogen or R^{N}-substituted or unsubstituted C₁₋₆ alkyl, R^{N}-substituted or unsubstituted C₃₋₇ cycloalkyl, or R^{N}-substituted or unsubstituted 3 to 7 membered heterocycloalkyl, or R^{B} and R^{C} are taken together with the nitrogen atom to which they are attached to form a R^{N}-substituted or unsubstituted 4 to 7 membered heterocycloalkyl;
R^{D} is hydrogen, halogen, -CN, -OR^{I}, -S(O)₂R^{I}, -NR^{J}R^{K}, R^{N}-substituted or unsubstituted C₁₋₆ alkyl, R^{N}-substituted or unsubstituted C₁₋₆ haloalkyl, R^{N}-substituted or unsubstituted C₃₋₇ cycloalkyl, R^{N}-substituted or unsubstituted 3 to 7 membered heterocycloalkyl, R^{N}-substituted or unsubstituted C₅₋₇ aryl, or R^{N}-substituted or unsubstituted 5 to 7 membered heteroaryl;
each R^{E} is hydrogen, halogen, -OR^{I}, -CN, -S(O)₂R^{I}, R^{M}-substituted or unsubstituted C₁₋₆ alkyl, R^{M}-substituted or unsubstituted C₁₋₆ haloalkyl, or R^{M}-substituted or unsubstituted C₃₋₇ cycloalkyl;
n is 0, 1, 2, 3, or 4;
R^{F} is R^{N}-substituted or unsubstituted C₁₋₆ alkyl, R^{N}-substituted or unsubstituted C₃₋₇ cycloalkyl, R^{N}-substituted or unsubstituted benzyl, or R^{N}-substituted or unsubstituted 3 to 7 membered heterocycloalkyl;
each R^{G} is independently halogen, -OR^{I}, R^{M}-substituted or unsubstituted C₁₋₆ alkyl, or R^{M}-substituted or unsubstituted C₁₋₆ haloalkyl;
t is 0, 1, 2, 3, 4, 5, or 6;
each R^{H} is independently halogen, C₁₋₃ alkyl, C₁₋₃ haloalkyl, or cyclopropyl;
j is 0, 1, or 2;
each R^{I}, R^{J}, and R^{K} is independently hydrogen, R^{M}-substituted or unsubstituted C₁₋₆ alkyl, R^{M}-substituted or unsubstituted C₁₋₆ haloalkyl, R^{M}-substituted or unsubstituted C₃₋₇ cycloalkyl, R^{M}-substituted or unsubstituted 3 to 7 membered heterocycloalkyl, R^{M}-substituted or unsubstituted C₅₋₇ membered aryl, or R^{M}-substituted or unsubstituted 5 to 7 membered heteroaryl;
each R^{M} is independently hydrogen, halogen, -CN, -OH, -OCH₃, -OCF₃, -S(O)₂H, - S(O)₂NH₂, -NH₂, -NH(CH₃), -N(CH₃)₂, -CF₃, -CHF₂, -CH₂F, -C(CH₃)₂F, -C(CH₃)F₂, unsubstituted C₁₋₆ alkoxy, unsubstituted C₁₋₆ alkyl, unsubstituted C₁₋₆ haloalkyl, unsubstituted C₃₋₇ cycloalkyl, unsubstituted 3 to 6 membered heterocycloalkyl, unsubstituted C₅₋₇ aryl, or unsubstituted 5 to 7 membered heteroaryl;
each R^{N} is independently hydrogen, halogen, -CN, -OR^{R}, -S(O)₂R^{R}, -NR^{S}R^{T}, R^{P}-substituted or unsubstituted C₁₋₆ alkyl, R^{P}-substituted or unsubstituted C₁₋₆ haloalkyl, R^{P}-substituted or unsubstituted C₁₋₆ alkoxy, R^{P}-substituted or unsubstituted C₃₋₇ cycloalkyl, R^{P}-substituted or unsubstituted 3 to 6 membered heterocycloalkyl, R^{P}-substituted or unsubstituted C₅₋₇ aryl, or R^{P}-substituted or unsubstituted 5 to 7 membered heteroaryl;
each R^{R}, R^{S} and R^{T} is independently hydrogen, R^{V}-substituted or unsubstituted C₁₋₆ alkyl, R^{V}-substituted or unsubstituted C₁₋₆ haloalkyl, R^{V}-substituted or unsubstituted C₃₋₇ cycloalkyl, or R^{V}-substituted or unsubstituted 3 to 7 membered heterocycloalkyl;
each R^{V} is independently hydrogen, halogen, -CN, -OH, -OCH₃, -OCF₃, -S(O)₂H, - S(O)₂NH₂, -NH₂, -NH(CH₃), -N(CH₃)₂, -CF₃, -CHF₂, -CH₂F, -C(CH₃)₂F, -C(CH₃)F₂, unsubstituted C₁₋₆ alkoxy, unsubstituted C₁₋₆ alkyl, unsubstituted C₁₋₆ haloalkyl, unsubstituted C₃₋₇ cycloalkyl, unsubstituted 3 to 6 membered heterocycloalkyl, unsubstituted C₅₋₇ aryl, or unsubstituted 5 to 7 membered heteroaryl;
each R^{P} is independently hydrogen, halogen, -CN, -OR^{W}, -S(O)₂R^{W}, -NR^{X}R^{Y}, R^{U}-substituted or unsubstituted C₁₋₆ alkyl, R^{U}-substituted or unsubstituted C₁₋₆ haloalkyl, R^{U}-substituted or unsubstituted C₃₋₇ cycloalkyl, R^{U}-substituted or unsubstituted 3 to 6 membered heterocycloalkyl, R^{U}-substituted or unsubstituted C₅₋₇ aryl, or R^{U}-substituted or unsubstituted 5 to 7 membered heteroaryl;
each R^{W}, R^{X} and R^{Y} is independently hydrogen, unsubstituted C₁₋₆ alkyl, unsubstituted C₁₋₆ haloalkyl, unsubstituted C₃₋₇ cycloalkyl, or unsubstituted 3 to 7 membered heterocycloalkyl; and
each R^{U} is independently hydrogen, halogen, -CN, -OH, -OCH₃, -OCF₃, -S(O)₂H, - S(O)₂NH₂, -NH₂, -NH(CH₃), -N(CH₃)₂, -CF₃, -CHF₂, -CH₂F, -C(CH₃)₂F, -C(CH₃)F₂, unsubstituted C₁₋₆ alkoxy, unsubstituted C₁₋₆ alkyl, unsubstituted C₁₋₆ haloalkyl, unsubstituted C₃₋₇ cycloalkyl, unsubstituted 3 to 6 membered heterocycloalkyl, unsubstituted C₅₋₇ aryl, or unsubstituted 5 to 7 membered heteroaryl;
wherein cycloalkyl refers to non-aromatic, saturated or unsaturated cyclic univalent hydrocarbon structures, which can consist of one ring or multiple rings, wherein a cycloalkyl comprising more than one ring can be fused, spiro, or bridged, or combinations thereof; and
wherein heterocycloalkyl refers to non-aromatic, saturated or unsaturated cyclic univalent hydrocarbon structures where one or more of the ring carbon atoms have been replaced by a heteroatom, which can consist of one ring or multiple rings, wherein a heterocycloalkyl comprising more than one ring can be fused, spiro, or bridged, or combinations thereof.

Embodiment 2: The compound or pharmaceutically acceptable salt thereof of embodiment 1, wherein the compound has the formula:

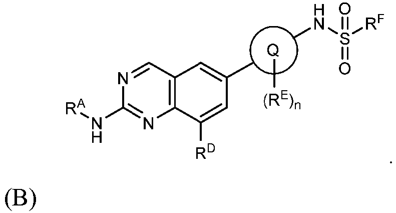

Embodiment 3: The compound or pharmaceutically acceptable salt thereof of embodiment 1 or 2, wherein R^{D} is halogen, -OR^{I}, -NR^{J}R^{K}, R^{N}-substituted or unsubstituted C₁₋₆ alkyl, R^{N}-substituted or unsubstituted C₁₋₆ haloalkyl, R^{N}-substituted or unsubstituted C₃₋₇ cycloalkyl, or R^{N}-substituted or unsubstituted 3 to 7 membered heterocycloalkyl.

Embodiment 4: The compound or pharmaceutically acceptable salt thereof of any one of embodiments 1 to 3, wherein R^{D} is -OR^{I} or -NR^{J}R^{K}.

Embodiment 5: The compound or pharmaceutically acceptable salt thereof of any one of embodiments 1 to 4, wherein R^{D} is -OR^{I} and R^{I} is R^{M}-substituted or unsubstituted C₁₋₆ alkyl, or R^{M}-substituted or unsubstituted C₁₋₆ haloalkyl.

Embodiment 6: The compound or pharmaceutically acceptable salt thereof of any one of embodiments 1 to 5, wherein R^{D} is -OR^{I} and R^{I} is unsubstituted C₁₋₆ alkyl, or unsubstituted C₁₋₆ haloalkyl.

Embodiment 7: The compound or pharmaceutically acceptable salt thereof of any one of embodiments 1 to 6, wherein R^{D} is -OR^{I} and R^{I} is methyl, ethyl, propyl, isopropyl, or butyl.

Embodiment 8: The compound or pharmaceutically acceptable salt thereof of any one of embodiments 1 to 7, wherein R^{D} is -OR^{I} and R^{I} is ethyl or isopropyl.

Embodiment 9: The compound or pharmaceutically acceptable salt thereof of any one of embodiments 1 to 4, wherein R^{D} is -OR^{I} and R^{I} is R^{M}-substituted or unsubstituted 3 to 7 membered heterocycloalkyl.

Embodiment 10: The compound or pharmaceutically acceptable salt thereof of any one of embodiments 1 to 4, wherein R^{D} is -NR^{J}R^{K} and R^{J} and R^{K} are independently hydrogen or R^{M}-substituted or unsubstituted C₁₋₆ alkyl.

Embodiment 11: The compound or pharmaceutically acceptable salt thereof of any one of embodiments 1 to 3, wherein R^{D} is R^{N}-substituted or unsubstituted C₁₋₆ alkyl or R^{N}-substituted or unsubstituted C₁₋₆ haloalkyl.

Embodiment 12: The compound or pharmaceutically acceptable salt thereof of embodiment 11, wherein R^{D} is methyl, ethyl, propyl, or isopropyl.

Embodiment 13: The compound or pharmaceutically acceptable salt thereof of embodiment 11, wherein R^{D} is -C(CH₃)₂F, -C(CH₃)F₂, -CH₂F, -CHF₂, or -CF₃.

Embodiment 14: The compound or pharmaceutically acceptable salt thereof of any one of embodiments 1 to 3, wherein R^{D} is R^{N}-substituted or unsubstituted C₃₋₇ cycloalkyl or R^{N}-substituted or unsubstituted 3 to 7 membered heterocycloalkyl.

Embodiment 15: The compound or pharmaceutically acceptable salt thereof of embodiments 1 to 3, wherein R^{D} is R^{N}-substituted or unsubstituted 3 to 7 membered heterocycloalkyl.

Embodiment 16: The compound or pharmaceutically acceptable salt thereof of any one of embodiments 1 to 15, wherein R^{E} is halogen and n is 1, 2, or 3.

Embodiment 17: The compound or pharmaceutically acceptable salt thereof of any one of embodiments 1 to 16, wherein R^{E} is F and n is 1, 2, or 3.

Embodiment 18: The compound or pharmaceutically acceptable salt thereof of any one of embodiments 1 to 15, wherein
R^{E} is -OR^{I} ;
R^{I} is hydrogen or C₁₋₃ unsubstituted alkyl or C₁₋₃ unsubstituted haloalkyl; and
n is 1.

Embodiment 19: The compound or pharmaceutically acceptable salt thereof of any one of embodiments 1 to 18, wherein L^{A} is -NHSO₂-.

Embodiment 20: The compound or pharmaceutically acceptable salt thereof of any one of embodiments 1 to 19, wherein R^{F} is R^{N}-substituted or unsubstituted C₁₋₆ alkyl or R^{N}-substituted or unsubstituted benzyl.

Embodiment 21: The compound or pharmaceutically acceptable salt thereof of any one of embodiments 1 to 20, wherein R^{F} is R^{N}-substituted or unsubstituted benzyl.

Embodiment 22: The compound or pharmaceutically acceptable salt thereof of any one of embodiments 1 to 20, wherein R^{F} is R^{N}-substituted or unsubstituted C₁₋₆ alkyl.

Embodiment 23: The compound or pharmaceutically acceptable salt thereof of any one of embodiments 20 to 22, wherein R^{N} is halogen, -CN, R^{P}-substituted or unsubstituted C₁₋₆ alkoxy, R^{P}-substituted or unsubstituted C₁₋₆ alkyl, or R^{P}-substituted or unsubstituted C₁₋₆ haloalkyl.

Embodiment 24: The compound or pharmaceutically acceptable salt thereof of any one of embodiments 20 to 22, wherein R^{N} is R^{P}-substituted or unsubstituted 3 to 6 membered heterocycloalkyl, R^{P}-substituted or unsubstituted C₅₋₇ cycloalkyl, R^{P}-substituted or unsubstituted C₅₋₇ aryl, or R^{P}-substituted or unsubstituted 5 to 7 membered heteroaryl.

Embodiment 25: The compound or pharmaceutically acceptable salt thereof of any one of embodiments 20 to 23, wherein R^{N} is hydrogen, halogen, -OH, -CN, -CF₃, -CHF₂, -CH₂F, -C(CH₃)₂F, -C(CH₃)F₂, methyl, ethyl, or propyl.

Embodiment 26: The compound or pharmaceutically acceptable salt thereof of embodiment 21, wherein R^{F} is unsubstituted benzyl.

Embodiment 27: The compound or pharmaceutically acceptable salt thereof of embodiment 21, wherein R^{F} is R^{N}-substituted benzyl and R^{N} is halogen, -CN, methyl, ethyl, or propyl.

Embodiment 28: The compound or pharmaceutically acceptable salt thereof of embodiment 21, wherein R^{F} is: 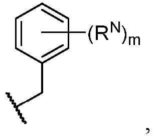 wherein R^{N} is halogen and m is 1 or 2.

Embodiment 29: The compound or pharmaceutically acceptable salt thereof of embodiment 22, wherein R^{F} is R^{N}-substituted C₁₋₆ alkyl and R^{N} is halogen, unsubstituted C₁₋₆ haloalkyl, or R^{P}-substituted or unsubstituted 5 to 7 membered heteroaryl.

Embodiment 30: The compound or pharmaceutically acceptable salt thereof of embodiment 22, wherein R^{F} is R^{N}-substituted C₁₋₆ alkyl and R^{N} is F or -CF₃.

Embodiment 31: The compound or pharmaceutically acceptable salt thereof of embodiment 22, wherein R^{F} is: 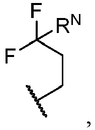 wherein R^{N} is F or methyl.

Embodiment 32: The compound or pharmaceutically acceptable salt thereof of any one of embodiments 1 to 31, wherein j is 0.

Embodiment 33: The compound or pharmaceutically acceptable salt thereof of any one of embodiments 1 to 32, wherein R^{B} and R^{C} are each independently R^{N}-substituted or unsubstituted C₁₋₆ alkyl.

Embodiment 34: The compound or pharmaceutically acceptable salt thereof of embodiment 33, wherein R^{N} is independently R^{P}-substituted or unsubstituted 3 to 5 membered heterocycloalkyl or halogen.

Embodiment 35: The compound or pharmaceutically acceptable salt thereof of embodiment 33, wherein R^{B} and R^{C} are each independently unsubstituted C₁₋₆ alkyl.

Embodiment 36: The compound or pharmaceutically acceptable salt thereof of any one of embodiments 1 to 32, wherein R^{B} is 4-membered heterocycloalkyl and R^{C} is hydrogen or R^{N}-substituted or unsubstituted C₁₋₆ alkyl.

Embodiment 37: The compound or pharmaceutically acceptable salt thereof of any one of embodiments 1 to 36, wherein R^{A} is:

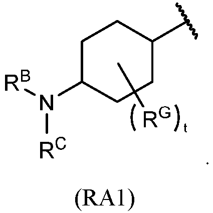

Embodiment 38: The compound or pharmaceutically acceptable salt thereof of any one of embodiments 1 to 32, wherein R^{A} is:

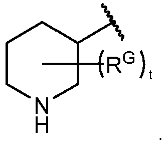

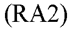

Embodiment 39: The compound or pharmaceutically acceptable salt thereof of any one of embodiments 1 to 38, wherein R^{G} is independently halogen or R^{M}-substituted or unsubstituted C₁₋₆ alkyl.

Embodiment 40: The compound or pharmaceutically acceptable salt thereof of any one of embodiments 1 to 39, wherein R^{G} is independently halogen.

Embodiment 41: The compound or pharmaceutically acceptable salt thereof of any one of embodiments 1 to 40, wherein R^{G} is independently fluoro and t is 1 or 2.

Embodiment 42: The compound or pharmaceutically acceptable salt thereof of any one of embodiments 1 to 40, wherein R^{G} is fluoro and t is 1.

Embodiment 43: The compound or pharmaceutically acceptable salt thereof of any one of embodiments 1 to 38, wherein R^{G} is independently R^{M}-substituted or unsubstituted C₁₋₆ alkyl or R^{M}-substituted or unsubstituted C₁₋₆ haloalkyl.

Embodiment 44: The compound or pharmaceutically acceptable salt thereof of any one of embodiments 1 to 43, wherein R^{A} has formula:

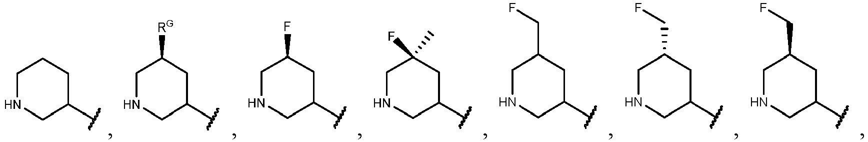

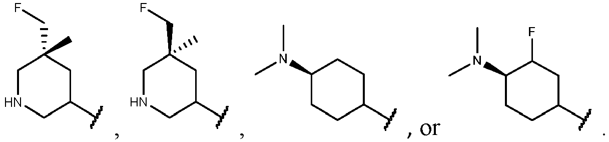

Embodiment 45: The compound or pharmaceutically acceptable salt thereof of any one of embodiments 1 to 44, wherein R^{H} is halogen and j is 1.

Embodiment 46: The compound or pharmaceutically acceptable salt thereof of any one of embodiments 1 to 44, wherein R^{H} is methyl, ethyl, propyl, or isopropyl and j is 1.

Embodiment 47: The compound or pharmaceutically acceptable salt thereof of any one of embodiments 1 to 46, wherein R^{H} is ortho to R^{D} and j is 1.

Embodiment 48: The compound or pharmaceutically acceptable salt thereof of any one of embodiments 1 to 47, wherein Ring Q is R^{E}-substituted or unsubstituted C₅₋₇ aryl.

Embodiment 49: The compound or pharmaceutically acceptable salt thereof of any one of embodiments 1 to 48, wherein Ring Q is R^{E}-substituted or unsubstituted phenyl.

Embodiment 50: The compound or pharmaceutically acceptable salt thereof of any one of embodiments 1 to 49, wherein Ring Q has formula: 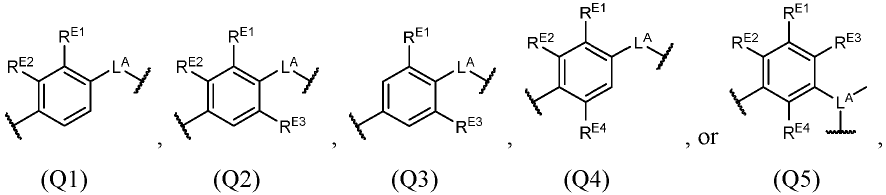 wherein R^{E1}, R^{E2}, R^{E3}, and R^{E4} are each independently hydrogen, halogen, or, R^{M}-substituted or unsubstituted C₁₋₆ alkyl.

Embodiment 51: The compound or pharmaceutically acceptable salt thereof of embodiment 50, wherein R^{E1}, R^{E2}, R^{E3}, and R^{E4} are each independently hydrogen or halogen.

Embodiment 52: The compound or pharmaceutically acceptable salt thereof of any one of embodiments 1 to 47, wherein Ring Q is R^{E}-substituted or unsubstituted 6 membered heteroaryl.

Embodiment 53: The compound or pharmaceutically acceptable salt thereof of any one of embodiments 1 to 51, wherein the compound has the formula: 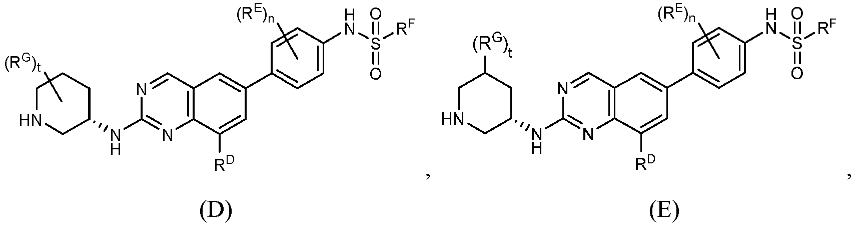 or

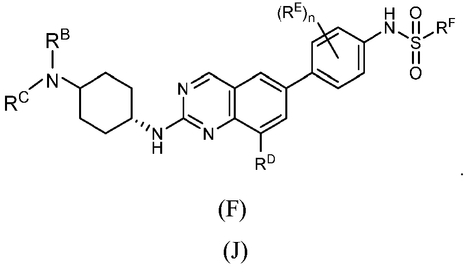

Embodiment 54: A compound or pharmaceutically acceptable salt thereof of Table 1.

Embodiment 55: A compound or pharmaceutically acceptable salt thereof of Table 2, with the exception of compounds 628, 629, 630, 637, and 638.

Embodiment 56: A pharmaceutical composition comprising a compound or pharmaceutically acceptable salt thereof of any one of embodiments 1 to 55 and one or more pharmaceutically acceptable excipients.

Embodiment 57: A compound according to any one of embodiments 1 to 55 or a pharmaceutically acceptable salt thereof, or the pharmaceutical composition of embodiment 56, for use in a method for treating an IRE1-related disease or disorder, wherein the IRE1-related disease or disorder is cancer.

Embodiment 58: The compound for use of embodiment 57, wherein the cancer is squamous cell carcinoma, small-cell lung cancer, non-small cell lung cancer (NSCLC), lung adenocarcinoma, squamous cell lung cancer, peritoneum cancer, hepatocellular cancer, stomach cancer, gastrointestinal cancer, esophageal cancer, pancreatic cancer, glioblastoma, cervical cancer, ovarian cancer, liver cancer, bladder cancer, breast cancer, colon cancer, rectal cancer, colorectal cancer, endometrial cancer, uterine cancer, salivary gland carcinoma, renal cancer, prostate cancer, vulval cancer, thyroid cancer, hepatocellular carcinoma (HCC), anal carcinoma, penile carcinoma, or head and neck cancer.

Embodiment 59: The compound for use of embodiment 57, wherein the cancer is lymphoma, lymphocytic leukemia, multiple myeloma (MM), acute myelogenous leukemia (AML), chronic myelogenous leukemia (CML), myelodysplastic syndrome (MDS), or myeloproliferative disease (MPD).

Embodiment 60: The compound for use of embodiment 57, wherein the cancer is multiple myeloma.

Embodiment 61: The compound for use of embodiment 57, wherein the cancer is a triple-negative breast cancer (TNBC).

Embodiment 62: The compound for use of any one of embodiments 57 to 61, further comprising administering one or more additional therapeutic agent(s) selected from the group consisting of an anti-inflammatory agent, a corticosteroid, an immunomodulatory agent, anti-cancer agent, an apoptosis-enhancer, a neurotropic factor, an agent for treating cardiovascular disease, an agent for treating liver disease, an anti-viral agent, an agent for treating blood disorders, an agent for treating diabetes, an agent for treating metabolic disorders, an agent for treating autoimmune disorders, and an agent for treating immunodeficiency disorders.

Embodiment 63: The compound for use of embodiment 62, wherein the additional therapeutic agent is a corticosteroid, a proteasome inhibitor, an immunomodulatory agent, an anti-CD38 antibody, an anti-VEGF-A antibody, an anti-PD-1 antibody, an anti-PD-L1 antibody, or an anti-interleukin-6 antibody, or a combination thereof.

Embodiment 64: The compound for use of embodiment 62, wherein the corticosteroid comprises dexamethasone.

Embodiment 65: The compound for use of embodiment 62, wherein proteasome inhibitor comprises carfilzomib, ixazomib, or bortezomib.

Embodiment 66: The compound for use of embodiment 62, wherein immunomodulatory agent comprises lenalidomide or pomalidomide.

Embodiment 67: The compound for use of embodiment 62, wherein the anti-PD-L1 antibody comprises avelumab, durvalumab, or atezolizumab.

Embodiment 68: The compound for use of embodiment 62, wherein the anti-PD-1 antibody comprises pembrolizumab or nivolumab.

Embodiment 69: The compound for use of any one of embodiments 57 to 68, further comprising administering radiotherapy.

### Examples:

The following Examples are presented by way of illustration, not limitation.

### ABBREVIATIONS

ACN: acetonitrile
DCM: dichloromethane
DMF: *N,N-*dimethylformamide
DMSO: dimethyl sulfoxide
EtOAc: ethyl acetate
EtOH: ethanol
h: hour
HCl: hydrochloric acid
HPLC: High-performance liquid chromatography
IPA: isopropyl acetate
LCMS: Liquid chromatography-mass spectrometry
Na₂SO₄: sodium sulfate
THF: tetrahydrofuran

### Example 1:

### Example 1001: (S)-N-(2,3-Difluoro-4-(2-(piperidin-3-ylamino)quinazolin-6-yl)phenyl)-1-phenylmethanesulfonamide Compound 1001

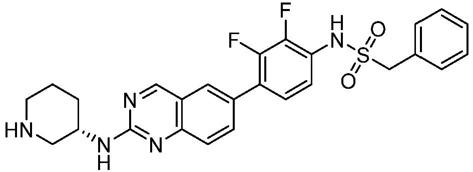

### Step 1: tert-Butyl (S)-3-((6-bromoquinazolin-2-yl)amino)piperidine-1-carboxylate

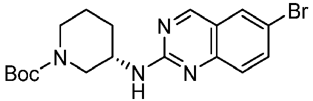

A mixture of 6-bromo-2-chloroquinazoline (2.0 g, 8.2 mmol), *tert*-butyl (3*S*)-3-amino-1-piperidinecarboxylate (2.0 g, 10 mmol), *N,N-*diisopropylethylamine (5.0 mL, 30 mmol) and cesium fluoride (4.0 g, 26 mmol) in dimethyl sulfoxide (50 mL) was stirred for 2h at 80 °C. The resulting solution was diluted with water and extracted with ethyl acetate. The organic layers were washed with brine, dried over sodium sulfate and concentrated. The organic layer was concentrated in vacuum. The residue was purified by silica flash chromatography eluting with ethyl acetate/petroleum ether (1:1) to afford the title compound (2.0 g, 59.8% yield) as a yellow solid.

### Step 2: tert-Butyl (S)-3-((6-(4-amino-2,3-difluorophenyl)quinazolin-2-yl)amino)piperidine-1-carboxylate

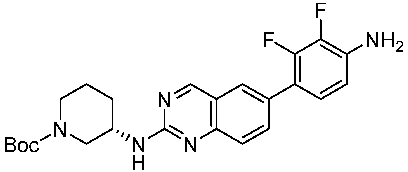

Under nitrogen, a solution of *tert*-butyl (*S*)-3-((6-(4-amino-2,3-difluorophenyl)quinazolin-2-yl)amino)piperidine-1-carboxylate (896 mg, 2.2 mmol) in 1,4-dioxane (50 mL) and water (5 mL) was added 2,3-difluoro-4-(4,4,5,5-tetramethyl-1,3,2-dioxaborolan-2-yl)aniline (673 mg, 2.6 mmol), (1,1'-bis(diphenylphosphino)ferrocene)dichloropalladium(II) (160 mg, 0.2 mmol) and sodiumbicarbonate (554 mg, 6.6 mmol). The mixture was stirred for 5 h at 90 °C. The solvent was removed under vacuum. The residue was purified by silica flash chromatography eluting with ethyl acetate/petroleum ether (30%) to afford the title compound (850 mg, 84.8% yield) as a yellow solid.

### Step 3: tert-Butyl (S)-3-((6-(2,3-difluoro-4-((phenylmethyl)sulfonamido)phenyl)quinazolin-2-yl)amino)piperidine-1-carboxylate

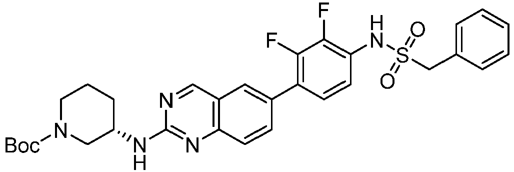

A solution of *tert*-butyl (*S*)-3-((6-(4-amino-2,3-difluorophenyl)quinazolin-2-yl)amino)piperidine-1-carboxylate (300 mg, 0.66 mmol) and alpha-toluenensulfonylchloride (251 mg, 1.32 mmol) in dichloromethane (2.0 mL) was added pyridine (780 mg, 9.9 mmol) and stirred for 20 h at 20 °C. The reaction was quenched with water and extracted with ethyl acetate. The organic layer was washed with brine, dried over anhydrous sodium sulphate and concentrated under vacuum. The residue was purified by silica flash chromatography eluting with ethyl acetate/petroleum ether (70%) to afford the title compound (350 mg, 87.2% yield) as a yellow oil. LCMS (ESI): [M+H]⁺ = 610.2

### Step 4: (S)-N-(2,3-Difluoro-4-(2-(piperidin-3-ylamino)quinazolin-6-yl)phenyl)-1-phenylmethanesulfonamide hydrochloride

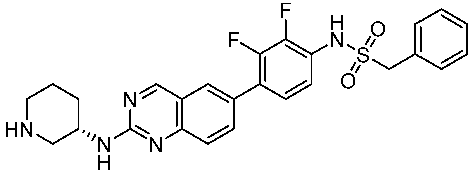

A solution of *tert*-butyl (*S*)-3-((6-(2,3-difluoro-4-((phenylmethyl)sulfonamido)phenyl)quinazolin-2-yl)amino)piperidine-1-carboxylate (100 mg, 0.16 mmol) in 4 M HCl in 1,4-Dioxane (5 mL) was stirred at 20 °C for 0.5 h. The solvent was removed under vacuum. The residue was purified by Prep-HPLC to afford the title compound (48 mg, 53.6% yield) as a yellow solid and as HCl salt.

### Example 1002: (S)-N-(2,3-Difluoro-4-(2-(piperidin-3-ylamino)quinazolin-6-yl)phenyl)-3,3,3-trifluoropropane-1-sulfonamide Compound 1002

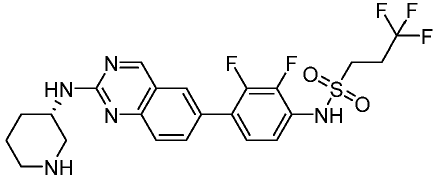

The title compound was prepared according to Example 1001. This provides the title compound (26.5 mg, 35.2% yield) as a white solid.

### Example 1003: (S)-1-Phenyl-N-(2,3,6-trifluoro-4-(2-(piperidin-3-ylamino)quinazolin-6-yl)phenyl)methanesulfonamide Compound 1003

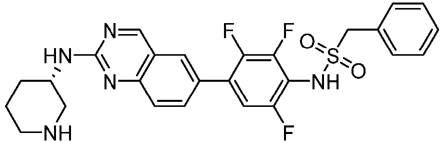

The title compound was prepared according to Example 1001. This provides the title compound (44.3 mg, 32.9%) as a white solid.

### Example 1004: (S)-N-(2,4-Difluoro-3-(2-(piperidin-3-ylamino)quinazolin-6-yl)phenyl)-1-phenylmethanesulfonamide Compound 1004

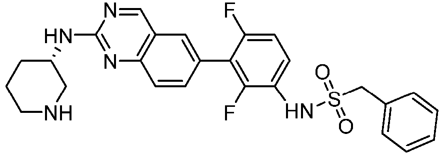

The title compound was prepared according to Example 1001. This provides the title compound (41.4 mg, 25.9% yield) as a yellow solid.

### Example 1005: (S)-N-(4-Fluoro-3-(2-(piperidin-3-ylamino)quinazolin-6-yl)phenyl)-1-phenylmethanesulfonamide Compound 1005

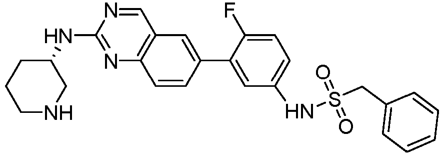

### Step 1: Benzyl (S)-3-((6-(2-fluoro-5-((phenylmethyl)sulfonamido)phenyl)quinazolin-2-yl)amino)piperidine-1-carboxylate

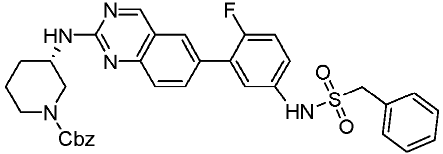

The title compound was prepared according to step 3 of Example 1001. This provides the title compound (150 mg, 80.7% yield) as a yellow solid. LCMS (ESI): [M+H]⁺ = 626.2

### Step 2: (S)-N-(4-Fluoro-3-(2-(piperidin-3-ylamino)quinazolin-6-yl)phenyl)-1-phenylmethanesulfonamide

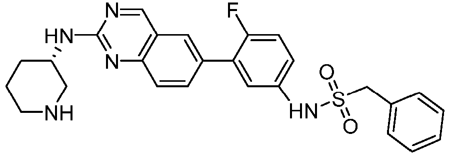

To a mixture of benzyl (*S*)-3-((6-(2-fluoro-5-((phenylmethyl)sulfonamido)phenyl)quinazolin-2-yl)amino)piperidine-1-carboxylate (150 mg, 0.24 mmol) in acetonitrile (1 mL) and dichloromethane (1 mL) was added dimethyl sulfide (0.5 mL) and boron trifluoride diethyl etherate (0.5 mL), the mixture was stirred for 1h at room temperature. The reaction was quenched with sat. sodium carbonate and dichloromethane. The solvent was concentrated in vacuum. The residue was purified by Prep-HPLC to afford the title compound (38.9 mg, 32.9% yield) as a white solid.

### Example 1006: (S)-N-(2-Fluoro-3-(2-(piperidin-3-ylamino)quinazolin-6-yl)phenyl)-1-phenylmethanesulfonamide Compound 1006

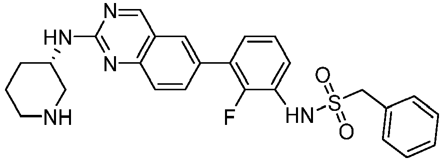

The title compound was prepared according to Example 1005. This provides the title compound (41.4 mg, 25.8% yield) as a yellow solid. LCMS (ESI) [M+H]⁺ = 492.2; ¹H NMR

### Example 1007: (S)-N-(2,3-Difluoro-4-(2-(piperidin-3-ylamino)quinazolin-6-yl)phenyl)-2,2-difluorobutane-1-sulfonamide Compound 1007

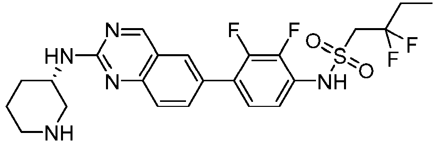

The title compound was prepared according to Example 1001. This provides the title compound (80 mg, 63.8% yield) as a white solid.

### Example 1008: (S)-N-(4-Chloro-2-fluoro-3-(2-(piperidin-3-ylamino)quinazolin-6-yl)phenyl)-1-phenylmethanesulfonamide Compound 1008

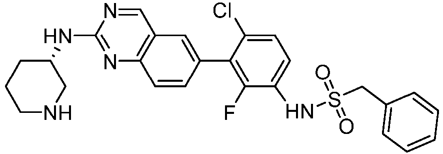

The title compound was prepared according to Example 1005. This provides the title compound (44.7 mg, 31.1% yield) as a yellow solid.

### Example 1009: (S)-N-(2,3-Difluoro-5-methyl-4-(2-(piperidin-3-ylamino)quinazolin-6-yl)phenyl)-1-phenylmethanesulfonamide Compound 1009

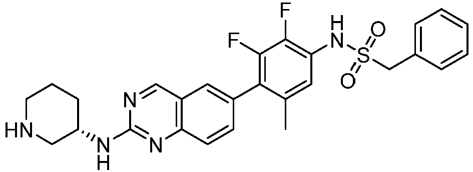

### Step 1: tert-Butyl (2,3-difluoro-4-hydroxy-5-methylphenyl)carbamate

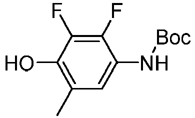

A solution of 4-amino-2,3-difluoro-6-methyl-phenol (350 mg, 2.2 mmol) in 1,4-dioxane (12 mL) and water (6 mL) was added sodium bicarbonate (370 mg, 4.4 mmol) and di-*tert*-butyl dicarbonate (576 mg, 2.6 mmol). The mixture was stirred for 16 h at 20 °C. The reaction mixture was diluted with water. The resulting solution was extracted with ethyl acetate and the organic layers were combined. The organic layer was dried over anhydrous sodium sulfate and concentrated under vacuum. The residue was purified by silica flash chromatography eluting with ethyl acetate/petroleum ether (16%) to afford the title compound (200 mg, 35.1% yield) as a white solid. LCMS (ESI): [M+H]⁺ = 260.1.

### Step 2: 4-((tert-Butoxycarbonyl)amino)-2,3-difluoro-6-methylphenyl trifluoromethanesulfonate

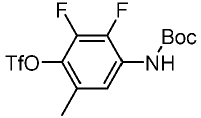

A solution of *tert*-butyl (2,3-difluoro-4-hydroxy-5-methylphenyl)carbamate (0.53 g, 2.0 mmol) in dichloromethane (3 mL) was added pyridine (0.32 g, 4.1 mmol) and trifluoromethanesulfonic anhydride (0.69 g, 2.5 mmol) at 0 °C. The resulting solution was stirred for 2 h at the same temperature. The reaction mixture was quenched with water. The resulting solution was extracted with dichloromethane and the organic layers were combined. The organic layer was dried over anhydrous sodium sulfate and concentrated under vacuum to afford the title compound (650 mg, 83% yield) as yellow oil. The crude product would be directly used for the next step reaction without purification. LCMS (ESI): [M+H]⁺ = 392.1.

### Step 3: tert-Butyl (2,3-difluoro-5-methyl-4-(4,4,5,5-tetramethyl-1,3,2-dioxaborolan-2-yl)phenyl)carbamate

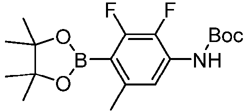

Under nitrogen, a solution of 4-((*tert*-butoxycarbonyl)amino)-2,3-difluoro-6-methylphenyl trifluoromethanesulfonate (650 mg, 1.6 mmol), bis(pinacolato)diboron (633 mg, 2.5 mmol), 1,1'-bis(diphenylphosphino)ferrocene-palladium(II)dichloride (68 mg, 0.08 mmol) and potassium acetate (490 mg, 5.0 mmol) in 1,4-dioxane (7 mL) was stirred for 16 h at 90 °C. The reaction mixture was diluted with water. The resulting solution was extracted with ethyl acetate and the organic layers were combined. The organic layer was dried over anhydrous sodium sulfate and concentrated under vacuum. The residue was purified by silica flash chromatography eluting with ethyl acetate/petroleum ether (3%) to afford the title compound (340 mg, 55.4% yield) as yellow oil. LCMS (ESI): [M+H]⁺ = 370.2.

### Step 4: Benzyl (S)-3-((6-(4-((tert-butoxycarbonyl)amino)-2,3-difluoro-6-methylphenyl)quinazolin-2-yl)amino)piperidine-1-carboxylate

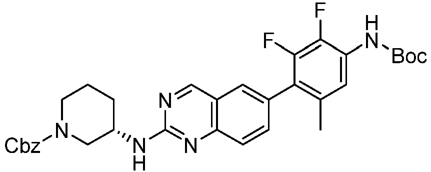

The title compound was prepared according to step 3 of Example 1001. This provides the title compound (440 mg, 83.7% yield) as a yellow solid. LCMS (ESI): [M+H]⁺ = 604.2.

### Step 5: Benzyl (S)-3-((6-(4-amino-2,3-difluoro-6-methylphenyl)quinazolin-2-yl)amino)piperidine-1-carboxylate

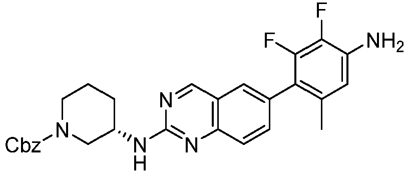

A solution of Benzyl (*S*)-3-((6-(4-((*tert*-butoxycarbonyl)amino)-2,3-difluoro-6-methylphenyl)quinazolin-2-yl)amino)piperidine-1-carboxylate (120 mg, 0.20 mmol) in dichloromethane (1 mL) was added 4 M HCl in 1,4-dioxane (3 mL). The resulting solution was stirred for 1 h at 25°C. The solvent was removed under vacuum to afford the title compound (100 mg, 100% yield) as a yellow solid. LCMS (ESI): [M+H]⁺ = 541.0.

### Step 6: (S)-N-(2,3-Difluoro-5-methyl-4-(2-(piperidin-3-ylamino)quinazolin-6-yl)phenyl)-1-phenylmethanesulfonamide

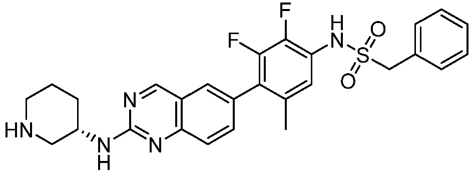

The title compound was prepared according to step 2 of Example 1005. This provides the title compound (21.9 mg, 27.2% yield) as an off-white solid.

### Example 1010: (S)-N-(2-Fluoro-4-(2-(piperidin-3-ylamino)quinazolin-6-yl)phenyl)-1-phenylmethanesulfonamide Compound 1010

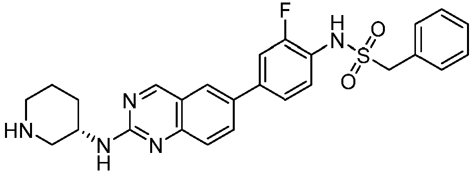

The title compound was prepared according to Example 1005. This provides the title compound (20.0 mg, 37.2% yield) as a yellow solid.

### Example 1011: (S)-N-(2,6-Difluoro-4-(2-(piperidin-3-ylamino)quinazolin-6-yl)phenyl)-1-phenylmethanesulfonamide Compound 1011

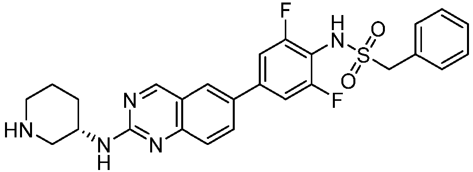

The title compound was prepared according to Example 1005. This provides the title compound (16.0 mg, 12.5% yield) as a yellow solid. LCMS (ESI): [M+H]⁺ = 510.1; ¹H NMR

### Example 1012: (S)-N-(2,3-Difluoro-4-(5-fluoro-2-(piperidin-3-ylamino)quinazolin-6-yl)phenyl)-1-phenylmethanesulfonamide Compound 1012

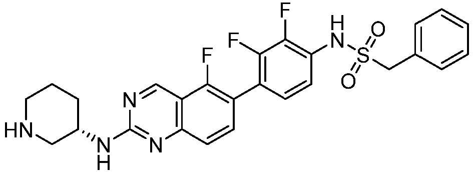

The title compound was prepared according to Example 1001. This provides the title compound (65.3 mg, 50.2% yield) as a white solid.

### Example 1013: N-(2,6-Difluoro-4-(2-(((3S,5S)-5-fluoropiperidin-3-yl)amino)quinazolin-6-yl)phenyl)-1-phenylmethanesulfonamide Compound 1013

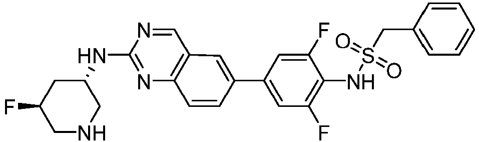

The title compound was prepared according to Example 1005. This provides the title compound (20.2 mg, 28% yield) as a yellow solid.

### Example 1014: (S)-1-Phenyl-N-(2,3,5-trifluoro-4-(2-(piperidin-3-ylamino)quinazolin-6-yl)phenyl)methanesulfonamide hydrochloride Compound 1014

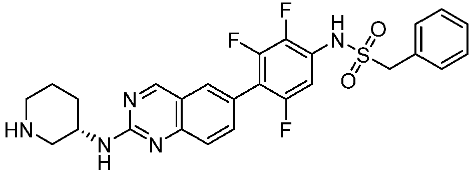

The title compound was prepared according to Example 1009. This provides the title compound (20.0 mg, 26.1% yield) as a white solid and as HCl salt.

### Example 1015: (S)-N-(2,3-Difluoro-4-(7-fluoro-2-(piperidin-3-ylamino)quinazolin-6-yl)phenyl)-1-phenylmethanesulfonamide Compound 1015

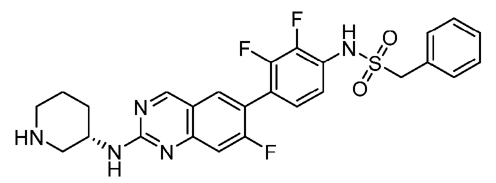

### Step 1: 5-Bromo-4-fluoro-2-nitro-benzaldehyde

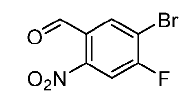

To a mixture of nitric acid (3 mL, 67 mmol) in sulfuric acid (20 mL) was added 3-bromo-4-fluorobenzaldehyde (5 g, 24 mmol) at 0 °C, the mixture was stirred for 2h at rt. The mixture was poured into ice water, extracted with ethyl acetate and concentrated. The residue was purified by silica flash chromatography eluting with ethyl acetate/petroleum ether (2%) to afford the title compound (5 g, 81.9% yield) as a yellow solid. ¹H NMR (400 MHz,CDCl₃) δ 10.40 (s, 1H), 8.24 (d, *J=* 6.7 Hz, 1H), 7.92 (d, *J=* 7.5 Hz, 1H).

### Step 2: 2-Amino-5-bromo-4-fluoro-benzaldehyde

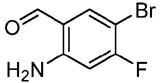

A mixture of 5-bromo-4-fluoro-2-nitro-benzaldehyde (5.0 g, 20 mmol), iron (3.5 g, 63 mmol), acetic Acid (20 mL, 349 mmol) in water (20 mL) and ethanol (100 mL) was stirred for 2h at 80 °C. The solids were filtered out. After filtration, the filtrate was concentrated under reduced pressure. The residue was purified by silica flash chromatography eluting with ethyl acetate/petroleum ether (1/4) to afford the title compound (3.5 g, 79.6% yield) as a yellow solid. LCMS (ESI): [M+H]⁺ = 218.0.

### Step 3: 6-Bromo-7-fluoroquinazolin-2(1H)-one

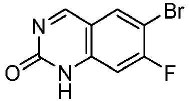

A mixture of 2-amino-5-bromo-4-fluoro-benzaldehyde (3.5 g, 16 mmol) and urea (14 g, 240 mmol) in 1-methyl-2-pyrrolidinone (60 mL) was stirred at 115 °C for overnight. The resulting solution was diluted with methanol and purified by reverse phase chromatography (acetonitrile /0.1% NH₄HCO₃ in water) to afford the title compound (730 mg, 18.7% yield) as a white solid. LCMS (ESI): [M+H]⁺ = 242.9.

### Step 4: 6-Bromo-2-chloro-7-fluoro-quinazoline

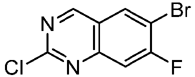

A mixture of 6-bromo-7-fluoroquinazolin-2(1*H*)-one (720 mg, 2.96 mmol) in phosphorus oxychloride (20 mL) was stirred at 105 °C for 2h. After most solvent was evaporated out, the resulting solution was poured into ice water, extracted with ethyl acetate and concentrated in vacuum. The residue was purified by silica flash chromatography eluting with ethyl acetate/petroleum ether (1/4) to afford the title compound (380 mg, 49.1% yield) as a white solid. LCMS (ESI): [M+H]⁺ = 260.9.

### Step 5: (S)-N-(2,3-Difluoro-4-(7-fluoro-2-(piperidin-3-ylamino)quinazolin-6-yl)phenyl)-1-phenylmethanesulfonamide

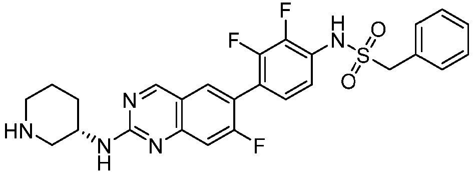

The title compound was prepared according to Example 1001. This provides the title compound (33.3 mg, 32.9% yield) as a yellow solid.

### Example 1016: N-(4-(8-Ethyl-2-(((3S,5S)-5-fluoropiperidin-3-yl)amino)quinazolin-6-yl)-3-fluorophenyl)-1-phenylmethanesulfonamide formate Compound 1016

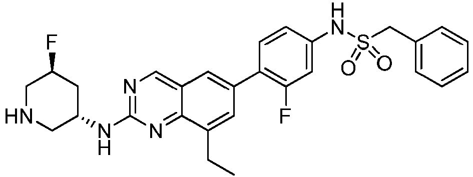

### Step 1: 4-Bromo-1-fluoro-2-vinylbenzene

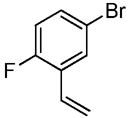

Methyltriphenylphosphonium bromide (17.1 g, 47.8 mmol) was suspended in diethylether (100 mL) and cooled to 0 °C. Potassium tert-butoxide (5.37 g, 47.8 mmol) was then added and the resulting yellow suspension was stirred at 0 °C for 15 min. To this was then added 5-bromo-2-fluoro-benzaldehyde (8.09 g, 39.9 mmol) dropwise and the cooling bath was removed. After stirring at rt for 30 min, the mixture was diluted with pentane (200 mL) and stirred at rt for 20 min then the solids were filtered off. The filtrate was concentrated under reduced pressure and to the crude residue was added pentane (200 mL) and the suspension stirred for 20 min then the solids were filtered off. The filtrate was concentrated under reduced pressure and the crude material was purified by silica flash chromatography (5% EtOAc/pentane) to provide the title compound (7.5 g mg, 93% yield). ¹H NMR (400 MHz, CDCl₃) δ 7.59 (dd, *J =* 6.6, 2.5 Hz, 1H), 7.32 - 7.28 (m, 1H), 6.92 (dd, *J =* 10.0, 8.8 Hz, 1H), 6.78 (dd, *J* = 17.7, 11.2 Hz, 1H), 5.81 (dd, *J* = 17.7, 0.6 Hz, 1H), 5.41 (dd, *J* = 11.2, 0.6 Hz, 1H).

### Step 2: 4-Bromo-2-ethyl-1-fluorobenzene

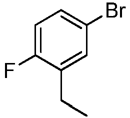

To a solution of 4-bromo-1-fluoro-2-vinylbenzene (14.0 g, 69.6 mmol) in methanol (40 mL) was added 10% w/w Pd/C (1.4 g) and the flask was sealed and purged with N₂ then purged with H₂ and stirred under a H₂ balloon (1 atm) overnight at rt. After 16 h, the flask was purged with N₂ and filtered through celite and the filtrate was concentrated under reduced pressure. The filtrate was then dissolved in pentane and filtered through a short pad of silica gel. The filtrate was then concentrated under reduced pressure to provide the title compound (12.3 g, 87 % yield). ¹H NMR (400 MHz, CDCl₃) 7.32 (dd, *J =* 6.7, 2.5 Hz, 1H), 7.29 - 7.21 (m, 1H), 6.93 - 6.84 (m, 1H), 2.64 (q, *J =* 7.6 Hz, 2H), 1.22 (t, *J =* 7.6 Hz, 3H).

### Step 3: 5-Bromo-3-ethyl-2-fluorobenzaldehyde

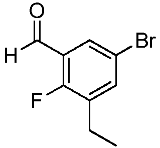

To a solution of 4-bromo-2-ethyl-1-fluorobenzene (12.3 g, 60.6 mmol) in THF (70 mL) at -78 °C was added 2M lithium diisopropylamide amide solution in THF/heptane (36.0 mL, 72.7 mmol). The mixture was stirred at -78 °C for 1 h then anhydrous DMF (7.03 mL, 90.9 mmol) was added dropwise. After the addition was complete, the cooling bath was removed and the mixture stirred at rt for 3 h. Ethyl acetate (200 mL) was then added and the solution was washed with 1N HCl, then with water, then saturated aqueous sodium chloride. The organic extract was dried over anhydrous Na₂SO₄, filtered and concentrated under reduced pressure. The crude material was purified by silica flash chromatography (0-50% EtOAc/heptanes) to provide the title compound (10.3 g, 73% yield). ¹H NMR (400 MHz, CDCl₃) 10.30 (s, 1H), 7.80 (dd, *J* = 5.6, 2.6 Hz, 1H), 7.58 (dd, *J =* 6.5, 2.6 Hz, 1H), 2.72 (q, J = 7.6 Hz, 2H), 1.28 - 1.24 (t, *J =* 7.6 Hz, 3H)

### Step 4: 6-Bromo-8-ethylquinazolin-2-amine

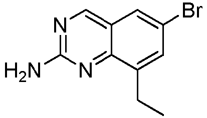

Guanidine carbonate salt (6.86 g, 76.2 mmol) and 5-bromo-3-ethyl-2-fluorobenzaldehyde (11.0 g, 47.6 mmol) were combined in dimethylacetamide (165 mL) and stirred in a 160 °C oil bath for 1 h. The mixture was then cooled to rt and diluted with water (250 mL) and ethyl acetate (200 mL) and the phases were separated. The organic extract was washed with water, then with saturated aqueous sodium chloride, dried (Na₂SO₄), filtered and concentrated under reduced pressure. The crude material was purified by flash chromatography through silica gel (10-100% EtOAc/heptanes) to provide the title compound (1.3 g, 11% yield). LCMS (ESI) [M+H]⁺= 252.0.

### Step 5: 6-Bromo-2-chloro-8-ethylquinazoline

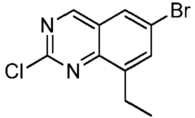

To a mixture of 6-bromo-8-ethylquinazolin-2-amine (1000 mg, 3.97 mmol), chlorotrimethylsilane (0.76 mL, 5.95 mmol), and tetrabutylammonium chloride hydrate (1.65 g, 5.95 mmol) in DMF (1.67 mL) and DCM (19.4 mL) was added *tert*-butyl nitrite (1.23 g, 11.9 mmol) and the mixture placed in a 50 °C oil bath overnight. After 16 h, the mixture was diluted with DCM and saturated aqueous sodium bicarbonate and the phases were separated. The aqueous phase was extracted twice with DCM and the combined organic extracts were washed with saturated aqueous sodium chloride, dried over anhydrous Na₂SO₄, filtered and concentrated under reduced pressure. The crude material was purified by silica flash chromatography (0-50% EtOAc/heptanes) to provide the title compound (430 mg, 40% yield). LCMS (ESI) [M+H]⁺= 272.9.

### Step 6: (3S,5S)-tert-Butyl 3-((6-bromo-8-ethylquinazolin-2-yl)amino)-5-fluoropiperidine-1-carboxylate

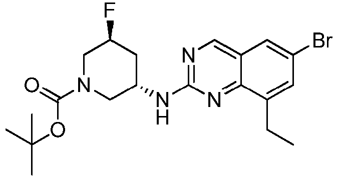

To a solution of 6-bromo-2-chloro-8-ethylquinazoline (210 mg, 0.77 mmol) in acetonitrile (3.9 mL) was added (3*S*,5*S*)-*tert*-butyl 3-amino-5-fluoropiperidine-1-carboxylate (202 mg, 0.93 mmol) and 1,8-diazabicyclo[5.4.0]undec-7-ene (353 mg, 2.32 mmol) and the mixture was placed in a 90 °C oil bath. After 4.5 h, the mixture was diluted with saturated aqueous ammonium chloride and ethyl acetate and the phases were separated. The aqueous phase was extracted twice with ethyl acetate and the combined organic extracts were washed with saturated aqueous sodium chloride, dried over anhydrous Na₂SO₄, filtered and concentrated under reduced pressure. The crude material was purified by silica flash chromatography (0-40% EtOAc/DCM) to provide the title compound (283 mg, 81% yield). LCMS (ESI) [M+H]⁺= 453.1.

### Step 7: (3S,5S)-tert-Butyl 3-((6-(4-amino-2-fluorophenyl)-8-ethylquinazolin-2-yl)amino)-5-fluoropiperidine-1-carboxylate

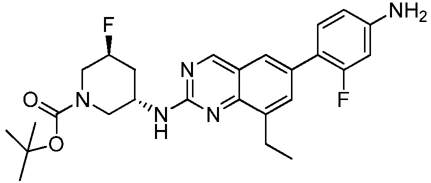

To a solution of (3*S*,5*S*)-*tert*-butyl 3-((6-bromo-8-ethylquinazolin-2-yl)amino)-5-fluoropiperidine-1-carboxylate (92 mg, 0.20 mmol) in a mixture of 1,2-dimethoxyethane (4 mL) and water (1 mL) was added 3-fluoro-4-(4,4,5,5-tetramethyl-1,3,2-dioxaborolan-2-yl)aniline (96 mg, 0.41 mmol), sodium carbonate (43 mg, 0.41 mmol), palladium(II)acetate (9.1 mg, 0.04 mmol), and tri-o-tolylphosphine (25 mg, 0.08 mmol) in that order. The reaction flask was sealed and purged with N₂ for 10 min then placed in a 85 °C oil bath. After 3 h, the mixture was cooled to rt and diluted with EtOAc and water. The phases were separated and the organic extract was washed with saturated aqueous sodium chloride, dried (Na₂SO₄), filtered and concentrated under reduced pressure. The crude material was purified by flash chromatography through silica gel (0-40% EtOAc/DCM) to provide the title product (62 mg, 63% yield). LCMS (ESI) [M+H]⁺= 484.3.

### Step 8: N-(4-(8-Ethyl-2-(((3S,5S)-5-fluoropiperidin-3-yl)amino)quinazolin-6-yl)-3-fluorophenyl)-1-phenylmethanesulfonamide formate

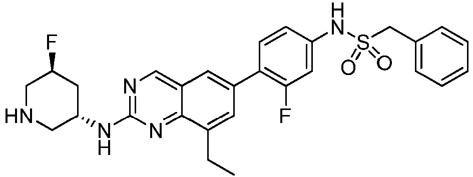

To a solution of (3*S*,5*S*)-*tert*-butyl 3-((6-(4-amino-2-fluorophenyl)-8-ethylquinazolin-2-yl)amino)-5-fluoropiperidine-1-carboxylate (62 mg, 0.13 mmol) in pyridine (0.7 mL) was added phenylmethanesulfonyl chloride (32 mg, 0.17 mmol) and the mixture stirred at rt. After 1 h, the mixture was diluted with methanol and silica gel was added and volatiles removed under reduced pressure to adsorb the crude product onto silica gel and purified by silica flash chromatography (10% EtOAc/heptanes) to provide the sulfonamide (3*S*,5*S*)-*tert*-butyl 3-((8-ethyl-6-(2-fluoro-4-(phenylmethylsulfonamido)phenyl)quinazolin-2-yl)amino)-5-fluoropiperidine-1-carboxylate (56 mg, 68% yield). *(3S,5S)-tert-Butyl* 3-((8-ethyl-6-(2-fluoro-4-(phenylmethylsulfonamido)phenyl)quinazolin-2-yl)amino)-5-fluoropiperidine-1-carboxylate (56 mg, 0.09 mmol) thus obtained was treated with 4N HCl in dioxanes (0.6 mL, 2.4 mmol) and stirred at rt. After 1 h, volatiles removed under reduced pressure and the crude residue was purified by C18 reverse phase flash chromatography (0 - 100% MeCN/10mM aqueous ammonium formate, pH = 3.8) to provide the title product (29 mg, 55% yield over two steps).

### Example 1017: N-(4-(8-Ethyl-2-(((3S,5S)-5-fluoropiperidin-3-yl)amino)quinazolin-6-yl)-2-fluorophenyl)-1-phenylmethanesulfonamide Compound 1017

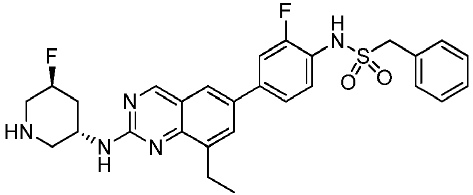

### Step 1: (3S,5S)-tert-Butyl 3-((6-(4-amino-3-fluorophenyl)-8-ethylquinazolin-2-yl)amino)-5-fluoropiperidine-1-carboxylate

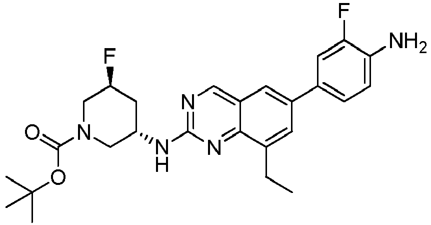

Prepared according to Example 1016 (step 7) to provide the title compound (93 mg, 95% yield). LCMS (ESI) [M+H]⁺= 484.0.

### Step 2: (3S,5S)-tert-Butyl 3-((8-ethyl-6-(3-fluoro-4-(phenylmethylsulfonamido)phenyl)quinazolin-2-yl)amino)-5-fluoropiperidine-1-carboxylate

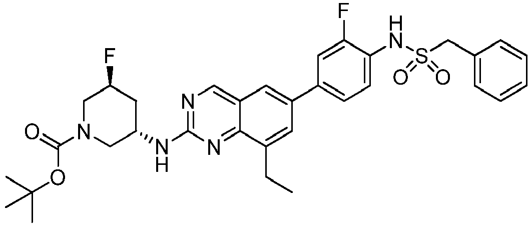

To a solution of (3*S*,5*S*)-*tert*-butyl 3-((6-(4-amino-3-fluorophenyl)-8-ethylquinazolin-2-yl)amino)-5-fluoropiperidine-1-carboxylate (93 mg, 0.19 mmol) in pyridine (1.0 mL) was added phenylmethanesulfonyl chloride (48 mg, 0.25 mmol) and the mixture stirred at rt. After 90 min, the mixture was diluted with methanol and silica gel was added and volatiles removed under reduced pressure to adsorb the crude product onto silica gel and purified by silica flash chromatography (0-100% EtOAc/heptanes) to provide the title compound (93 mg, 76% yield). LCMS (ESI) [M+H]⁺= 638.0.

### Step 3: N-(4-(8-Ethyl-2-(((3S,5S)-5-fluoropiperidin-3-yl)amino)quinazolin-6-yl)-2-fluorophenyl)-1-phenylmethanesulfonamide

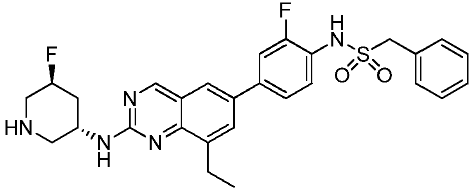

To a solution of (3*S*,5*S*)-*tert*-butyl 3-((8-ethyl-6-(3-fluoro-4-(phenylmethylsulfonamido)phenyl)quinazolin-2-yl)amino)-5-fluoropiperidine-1-carboxylate (93 mg, 0.15 mmol) in 1,4-dioxane (1.0 mL) was added 4N HCl in dioxanes (1 mL, 4 mmol) and the mixture was stirred at rt. After 90 min, volatiles removed under reduced pressure and the crude residue was purified by C18 reverse phase flash chromatography (0 - 50% MeCN/10mM aqueous ammonium formate, pH = 3.8) to provide the title product (44 mg, 56% yield).

### Example 1018: N-(4-(2-(((1,4-trans)-4-(Dimethylamino)cyclohexyl)amino)-8-ethylquinazolin-6-yl)-2-fluorophenyl)-3,3,3-trifluoropropane-1-sulfonamide Compound 1018

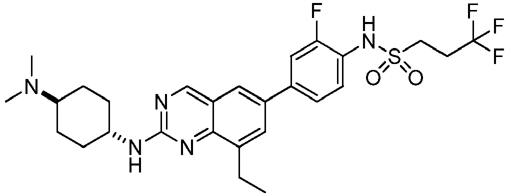

### Step 1: (1,4-trans)-N¹-(6-Bromo-8-ethylquinazolin-2-yl)-N⁴,N⁴-dimethylcyclohexane-1,4-diamine

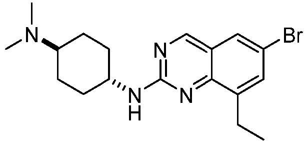

To a solution of 6-bromo-2-chloro-8-ethylquinazoline (100 mg, 0.37 mmol) in acetonitrile (1.8 mL) was added (1,4-*trans*)-*N*¹,*N*¹-dimethylcyclohexane-1,4-diamine (92 mg, 0.52 mmol) and 1,8-diazabicyclo[5.4.0]undec-7-ene (168 mg, 1.10 mmol) and the mixture was placed in a 60 °C oil bath. After 3 h, the mixture was diluted with saturated aqueous ammonium chloride and ethyl acetate and the phases were separated. The aqueous phase was extracted twice with ethyl acetate and the combined organic extracts were washed with saturated aqueous sodium chloride, dried over anhydrous Na₂SO₄, filtered and concentrated under reduced pressure. The crude material was purified by silica flash chromatography (0-100% EtOAc/DCM) to provide the title compound (61 mg, 44% yield). LCMS (ESI) [M+H]⁺= 377.1.

### Step 2: (1,4-trans)-N¹-(6-(4-Amino-3-fluorophenyl)-8-ethylquinazolin-2-yl)-N⁴,N⁴-dimethylcyclohexane-1,4-diamine

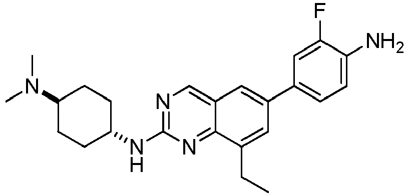

To a solution of (1,4-*trans*)-*N*¹-(6-bromo-8-ethylquinazolin-2-yl)-*N*⁴,*N*⁴-dimethylcyclohexane-1,4-diamine (63 mg, 0.17 mmol) in a mixture of 1,2-dimethoxyethane (4 mL) and water (1 mL) was added 2-fluoro-4-(4,4,5,5-tetramethyl-1,3,2-dioxaborolan-2-yl)aniline (79 mg, 0.33 mmol), sodium carbonate (35 mg, 0.33 mmol), palladium(II)acetate (3.8 mg, 0.02 mmol), and tri-o-tolylphosphine (10 mg, 0.03 mmol) in that order. The reaction flask was sealed and purged with N₂ for 10 min then placed in a 85 °C oil bath. After 3 h, the mixture was cooled to rt and diluted with EtOAc and water. The phases were separated and the organic extract was washed with saturated aqueous sodium chloride, dried over anhydrous Na₂SO₄, filtered and concentrated under reduced pressure. The crude material was purified by C18 reverse phase flash chromatography (10-70% acetoni/10 mM aqueous ammonium formate) to provide a yellow solid. This solid was partitioned between ethyl acetate and aqueous saturated sodium bicarbonate and the phases were separated. The organic extract was dried over anhydrous Na₂SO₄, filtered and concentrated under reduced pressure to provide the title compound (24 mg, 35% yield). LCMS (ESI) [M+H]⁺= 408.3.

### Step 3: N-(4-(2-(((1,4-trans)-4-(Dimethylamino)cyclohexyl)amino)-8-ethylquinazolin-6-yl)-2-fluorophenyl)-3,3,3-trifluoropropane-1-sulfonamide

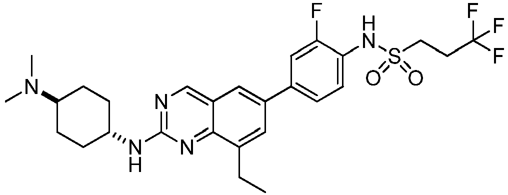

To a solution of (1,4-*trans*)-*N*¹-(6-(4-amino-3-fluorophenyl)-8-ethylquinazolin-2-yl)-*N*⁴,*N*⁴-dimethylcyclohexane-1,4-diamine (24 mg, 0.06 mmol) in a mixture of DCM (0.29 mL) and pyridine (0.095 mL, 1.18 mmol) was added 3,3,3-trifluoropropane-1-sulfonyl chloride (16 mg, 0.08 mmol) and the mixture stirred at rt. After 3 h, the mixture was diluted with MeOH and volatiles removed under reduced pressure. The crude material thus obtained was purified by C18 reverse phase chromatography (20-100% MeCN/10mM aqueous ammonium bicarbonate, pH = 10). Appropriate fractions combined and lyophilized to provide the title product (13.5 mg, 40% yield).

Example 1019. IRE1α TR-FRET Competition Binding Assay.

To determine the affinity of compound binding to the kinase domain of IRE1α, a time-resolved fluorescence resonance energy transfer (TR-FRET) competition assay was used.

A His-tagged IRE1α kinase dead construct containing the kinase and RNase domains (KR, AA G547-L977, D688N) was expressed in Sf9 insect cells. The purified protein (final concentration 0.25 nM) was pre-incubated with anti-His Europium labeled antibody (Life Technologies PV5596, final concentration 2 nM) for one hour at 4°C in TR-FRET Assay Buffer (50 mM HEPES, pH 7.5, 10 mM MgCl₂, 0.083 mM Brij 35, 1 mM DTT, and 0.1% bovine gamma globulin) prior to addition to test compounds. An Alexa fluor 647-labeled probe based on an ATP competitive inhibitor was added to a final concentration of 2 nM. Reactions were carried out for one hour at room temperature in a final volume of 20 µL in 384 well white ProxiPlates (Perkin Elmer 6008289). Binding of the probe to the IRE1α protein was detected in an Envision instrument (PerkinElmer) equipped with a TRF laser option and a LANCE/Delfia Dual/Bias D400/D630 mirror (Ex 347 nm, 1st Em 665 nm, 2nd Em 615 nm).

Example 1020. IRE1α RNase Activity Assay.

Inhibitors of the RNase activity of IRE1α were assessed by using a mini-XBP-1 stem-loop RNA as a substrate for the IRE1α RNase activity. A 5'-Carboxyfluorescein (FAM)-and 3'-Black Hole Quencher (BHQ)-labeled XBP1 single stem-loop mini-substrate (5'FAM-CAUGUCCGCAGCGCAUG-3'BHQ) is cleaved by IRE1α. When the oligo is intact, the fluorescence signal is quenched by BHQ. Upon cleavage, the fluorescence is no longer quenched and can be quantified.

An IRE1α construct corresponding to the linker, kinase and RNase domains (LKR, AA Q470-L977) was expressed in Sf9 insect cells. All reagent preparation and procedures were done under RNase free conditions. Test compounds and purified enzyme were combined in RNase Assay Buffer (20 mM HEPES, pH 7.5, 50 mM KAc, 1 mM MgAc, 1 mM DTT, and 0.05% Triton X-100) in a 384 well white ProxiPlate (Perkin Elmer 6008289). Upon addition of the RNA substrate (final assay volume 20 µL), the plates were placed into a Flexstation 3 instrument (Molecular Devices) for kinetic fluorescence reading at 2 minute intervals (Ex 485, Em 535). The velocity of the reaction, using the first 50 minutes, was used to calculate the RNase activity and inhibition of test compounds.

### Example 1021: IRE1α Ribonuclease Luciferase Reporter Assay

HEK293 cells expressing a pBABE.puro_HA-2xXBP1delta DBD firefly luciferase reporter, obtained from the University of California at San Francisco (UCSF, Walter lab), were cultured in DMEM high glucose media containing L-glutamine, 10% fetal bovine serum, 100 units/mL of penicillin and 100 µg/mL of streptomycin, plus 2 µg/ml puromycin to maintain selective pressure. Upon stimulation of IRE1 and activation of the endogenous RNase activity, a 26 nt intron is removed from XBP1 resulting in a frame shift allowing the transcription of the luciferase.

Cells were seeded without puromycin at 10,000/well in 384 well clear bottom white tissue culture plates (Corning 3707), 25 µL volume. The following morning, test compounds were added and incubated for one hour at 37°C prior to stimulation of the cells with thapsigargin at 50 µM final concentration for an additional 5 hours. After equilibration to room temperature, 25 µL of One-Glo luciferase detection reagent (Promega cat # E6120) was added, plates sealed and shaken for 5 minutes to lyse cells, then luciferase quantified by luminescence detection using an Envision instrument (PerkinElmer).

Reference for the XBP1s reporter cell line: Mendez AS, Alfaro J, Morales-Soto MA, Dar AC, McCullagh E, Gotthardt K, Li H, Acosta-Alvear D, Sidrauski C, Korennykh AV, Bemales S, Shokat KM, Walter P. 2015. Endoplasmic reticulum stress-independent activation of unfolded protein response kinases by a small molecule ATP-mimic. eLife 2015;4:e05434

All technical and scientific terms used herein have the same meaning. Efforts have been made to ensure accuracy with respect to numbers used (e.g. amounts, temperature, etc.) but some experimental errors and deviations should be accounted for.

Throughout this specification and the claims, the words "comprise," "comprises," and "comprising" are used in a non-exclusive sense, except where the context requires otherwise. It is understood that embodiments described herein include "consisting of" and/or "consisting essentially of" embodiments.

Where a range of values is provided, it is understood that each intervening value, to the tenth of the unit of the lower limit, unless the context clearly dictates otherwise, between the upper and lower limit of the range and any other stated or intervening value in that stated range, is encompassed herein. The upper and lower limits of these small ranges which can independently be included in the smaller rangers is also encompassed herein, subject to any specifically excluded limit in the stated range. Where the stated range includes one or both of the limits, ranges excluding either or both of those included limits are also included herein.

## Claims

1. A compound having formula (A): 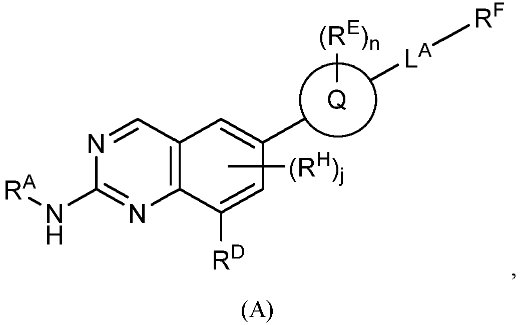 or a stereoisomer, tautomer, or pharmaceutically acceptable salt thereof, wherein:
Ring Q is R^{E}-substituted or unsubstituted C₅₋₇ aryl, or R^{E}-substituted or unsubstituted 6 membered heteroaryl comprising at least one nitrogen heteroatom;
L^{A} is -NHSO₂-, -SO₂NH-, or -NH-;
R^{A} is 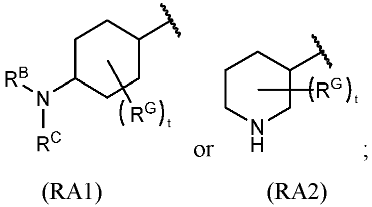
R^{B} and R^{C} are independently hydrogen or R^{N}-substituted or unsubstituted C₁₋₆ alkyl, R^{N}-substituted or unsubstituted C₃₋₇ cycloalkyl, or R^{N}-substituted or unsubstituted 3 to 7 membered heterocycloalkyl, or R^{B} and R^{C} are taken together with the nitrogen atom to which they are attached to form a R^{N}-substituted or unsubstituted 4 to 7 membered heterocycloalkyl;
R^{D} is hydrogen, halogen, -CN, -OR^{I}, -S(O)₂R^{I}, -NR^{J}R^{K}, R^{N}-substituted or unsubstituted C₁₋₆ alkyl, R^{N}-substituted or unsubstituted C₁₋₆ haloalkyl, R^{N}-substituted or unsubstituted C₃₋₇ cycloalkyl, R^{N}-substituted or unsubstituted 3 to 7 membered heterocycloalkyl, R^{N}-substituted or unsubstituted C₅₋₇ aryl, or R^{N}-substituted or unsubstituted 5 to 7 membered heteroaryl;
each R^{E} is hydrogen, halogen, -OR^{I}, -CN, -S(O)₂R^{I}, R^{M}-substituted or unsubstituted C₁₋₆ alkyl, R^{M}-substituted or unsubstituted C₁₋₆ haloalkyl, or R^{M}-substituted or unsubstituted C₃₋₇ cycloalkyl;
n is 0, 1, 2, 3, or 4;
R^{F} is R^{N}-substituted or unsubstituted C₁₋₆ alkyl, R^{N}-substituted or unsubstituted C₃₋₇ cycloalkyl, R^{N}-substituted or unsubstituted benzyl, or R^{N}-substituted or unsubstituted 3 to 7 membered heterocycloalkyl;
each R^{G} is independently halogen, -OR^{I}, R^{M}-substituted or unsubstituted C₁₋₆ alkyl, or R^{M}-substituted or unsubstituted C₁₋₆ haloalkyl;
t is 0, 1, 2, 3, 4, 5, or 6;
each R^{H} is independently halogen, C₁₋₃ alkyl, C₁₋₃ haloalkyl, or cyclopropyl;
j is 0, 1, or 2;
each R^{I}, R^{J}, and R^{K} is independently hydrogen, R^{M}-substituted or unsubstituted C₁₋₆ alkyl, R^{M}-substituted or unsubstituted C₁₋₆ haloalkyl, R^{M}-substituted or unsubstituted C₃₋₇ cycloalkyl, R^{M}-substituted or unsubstituted 3 to 7 membered heterocycloalkyl, R^{M}-substituted or unsubstituted C₅₋₇ membered aryl, or R^{M}-substituted or unsubstituted 5 to 7 membered heteroaryl;
each R^{M} is independently hydrogen, halogen, -CN, -OH, -OCH₃, -OCF₃, -S(O)₂H, - S(O)₂NH₂, -NH₂, -NH(CH₃), -N(CH₃)₂, -CF₃, -CHF₂, -CH₂F, -C(CH₃)₂F, -C(CH₃)F₂, unsubstituted C₁₋₆ alkoxy, unsubstituted C₁₋₆ alkyl, unsubstituted C₁₋₆ haloalkyl, unsubstituted C₃₋₇ cycloalkyl, unsubstituted 3 to 6 membered heterocycloalkyl, unsubstituted C₅₋₇ aryl, or unsubstituted 5 to 7 membered heteroaryl;
each R^{N} is independently hydrogen, halogen, -CN, -OR^{R}, -S(O)₂R^{R}, -NR^{S}R^{T}, R^{P}-substituted or unsubstituted C₁₋₆ alkyl, R^{P}-substituted or unsubstituted C₁₋₆ haloalkyl, R^{P}-substituted or unsubstituted C₁₋₆ alkoxy, R^{P}-substituted or unsubstituted C₃₋₇ cycloalkyl, R^{P}-substituted or unsubstituted 3 to 6 membered heterocycloalkyl, R^{P}-substituted or unsubstituted C₅₋₇ aryl, or R^{P}-substituted or unsubstituted 5 to 7 membered heteroaryl;
each R^{R}, R^{S} and R^{T} is independently hydrogen, R^{V}-substituted or unsubstituted C₁₋₆ alkyl, R^{V}-substituted or unsubstituted C₁₋₆ haloalkyl, R^{V}-substituted or unsubstituted C₃₋₇ cycloalkyl, or R^{V}-substituted or unsubstituted 3 to 7 membered heterocycloalkyl;
each R^{V} is independently hydrogen, halogen, -CN, -OH, -OCH₃, -OCF₃, -S(O)₂H, - S(O)₂NH₂, -NH₂, -NH(CH₃), -N(CH₃)₂, -CF₃, -CHF₂, -CH₂F, -C(CH₃)₂F, -C(CH₃)F₂, unsubstituted C₁₋₆ alkoxy, unsubstituted C₁₋₆ alkyl, unsubstituted C₁₋₆ haloalkyl, unsubstituted C₃₋₇ cycloalkyl, unsubstituted 3 to 6 membered heterocycloalkyl, unsubstituted C₅₋₇ aryl, or unsubstituted 5 to 7 membered heteroaryl;
each R^{P} is independently hydrogen, halogen, -CN, -OR^{W}, -S(O)₂R^{W}, -NR^{X}R^{Y}, R^{U}-substituted or unsubstituted C₁₋₆ alkyl, R^{U}-substituted or unsubstituted C₁₋₆ haloalkyl, R^{U}-substituted or unsubstituted C₃₋₇ cycloalkyl, R^{U}-substituted or unsubstituted 3 to 6 membered heterocycloalkyl, R^{V}-substituted or unsubstituted C₅₋₇ aryl, or R^{U}-substituted or unsubstituted 5 to 7 membered heteroaryl;
each R^{W}, R^{X} and R^{Y} is independently hydrogen, unsubstituted C₁₋₆ alkyl, unsubstituted C₁₋₆ haloalkyl, unsubstituted C₃₋₇ cycloalkyl, or unsubstituted 3 to 7 membered heterocycloalkyl, and
each R^{U} is independently hydrogen, halogen, -CN, -OH, -OCH₃, -OCF₃, -S(O)₂H, - S(O)₂NH₂, -NH₂, -NH(CH₃), -N(CH₃)₂, -CF₃, -CHF₂, -CH₂F, -C(CH₃)₂F, -C(CH₃)F₂, unsubstituted C₁₋₆ alkoxy, unsubstituted C₁₋₆ alkyl, unsubstituted C₁₋₆ haloalkyl, unsubstituted C₃₋₇ cycloalkyl, unsubstituted 3 to 6 membered heterocycloalkyl, unsubstituted C₅₋₇ aryl, or unsubstituted 5 to 7 membered heteroaryl;
wherein cycloalkyl refers to non-aromatic, saturated or unsaturated cyclic univalent hydrocarbon structures, which can consist of one ring or multiple rings, wherein a cycloalkyl comprising more than one ring can be fused, spiro, or bridged, or combinations thereof; and
wherein heterocycloalkyl refers to non-aromatic, saturated or unsaturated cyclic univalent hydrocarbon structures where one or more of the ring carbon atoms have been replaced by a heteroatom, which can consist of one ring or multiple rings, wherein a heterocycloalkyl comprising more than one ring can be fused, spiro, or bridged, or combinations thereof.

2. The compound or pharmaceutically acceptable salt thereof of claim 1, wherein the compound has the formula:

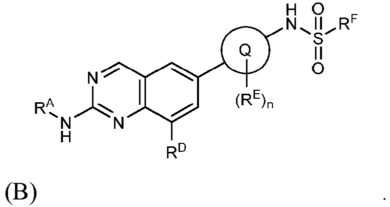

3. The compound or pharmaceutically acceptable salt thereof of claim 1 or 2, wherein R^{D} is halogen, -OR^{I}, -NR^{J}R^{K}, R^{N}-substituted or unsubstituted C₁₋₆ alkyl, R^{N}-substituted or unsubstituted C₁₋₆ haloalkyl, R^{N}-substituted or unsubstituted C₃₋₇ cycloalkyl, or R^{N}-substituted or unsubstituted 3 to 7 membered heterocycloalkyl.

4. The compound or pharmaceutically acceptable salt thereof of any one of claims 1 to 3, wherein R^{D} is R^{N}-substituted or unsubstituted C₁₋₆ alkyl or R^{N}-substituted or unsubstituted C₁₋₆ haloalkyl.

5. The compound or pharmaceutically acceptable salt thereof of claim 4, wherein R^{D} is methyl, ethyl, propyl, or isopropyl.

6. The compound or pharmaceutically acceptable salt thereof of any one of claims 1 to 5, wherein R^{E} is F and n is 1, 2, or 3.

7. The compound or pharmaceutically acceptable salt thereof of any one of claims 1 to 6, wherein L^{A} is -NHSO₂-.

8. The compound or pharmaceutically acceptable salt thereof of any one of claims 1 to 7, wherein R^{F} is R^{N}-substituted or unsubstituted C₁₋₆ alkyl or R^{N}-substituted or unsubstituted benzyl.

9. The compound or pharmaceutically acceptable salt thereof of claim 8, wherein R^{N} is hydrogen, halogen, -OH, -CN, -CF₃, -CHF₂, -CH₂F, -C(CH₃)₂F, -C(CH₃)F₂, methyl, ethyl, or propyl.

10. The compound or pharmaceutically acceptable salt thereof of claim 8, wherein R^{F} is: 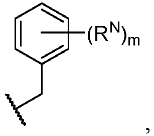
wherein R^{N} is halogen and m is 1 or 2; or
wherein R^{F} is: 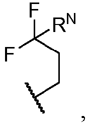
wherein R^{N} is F or methyl.

11. The compound or pharmaceutically acceptable salt thereof of any one of claims 1 to 10, wherein R^{A} is: 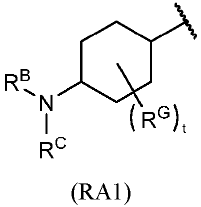 wherein:
R^{B} and R^{C} are each independently R^{N}-substituted or unsubstituted C₁₋₆ alkyl; or
R^{B} is 4-membered heterocycloalkyl and R^{C} is hydrogen or R^{N}-substituted or unsubstituted C₁₋₆ alkyl.

12. The compound or pharmaceutically acceptable salt thereof of any one of claims 1 to 10, wherein R^{A} is:

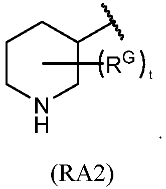

13. The compound or pharmaceutically acceptable salt thereof of any one of claims 1 to 12, wherein R^{G} is independently halogen or R^{M}-substituted or unsubstituted C₁₋₆ alkyl.

14. The compound or pharmaceutically acceptable salt thereof of any one of claims 1 to 13, wherein R^{G} is independently fluoro and t is 1 or 2.

15. The compound or pharmaceutically acceptable salt thereof of any one of claims 1 to 14, wherein R^{H} is halogen and j is 1.

16. The compound or pharmaceutically acceptable salt thereof of any one of claims 1 to 15, wherein Ring Q has formula: 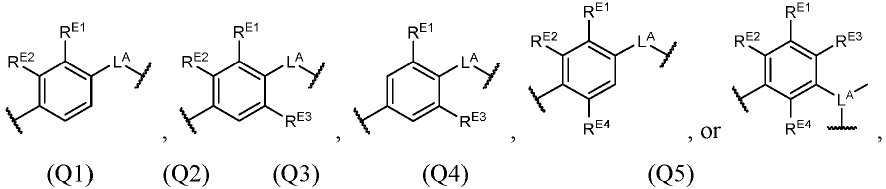 wherein R^{E1}, R^{E2}, R^{E3}, and R^{E4} are each independently hydrogen, halogen, or, R^{M}-substituted or unsubstituted C₁₋₆ alkyl.

17. The compound or pharmaceutically acceptable salt thereof of claim 1, selected from the group consisting of
| Cmpd No. | Structure |
|---|---|
| 1001 | 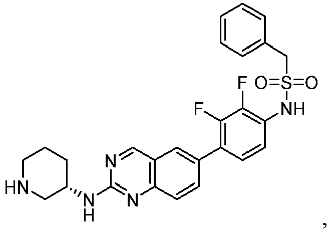 |
| 1002 | 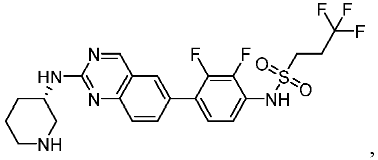 |
| 1003 | 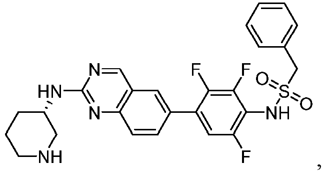 |
| 1004 | 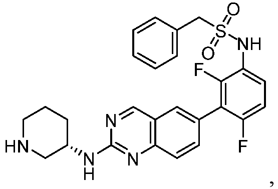 |
| 1005 | 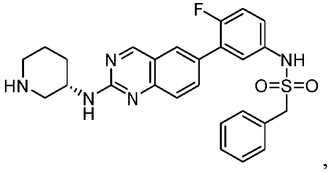 |
| 1006 | 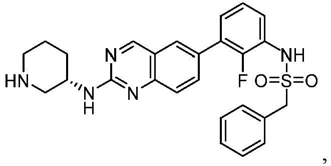 |
| 1007 | 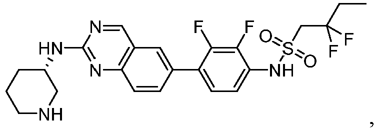 |
| 1008 | 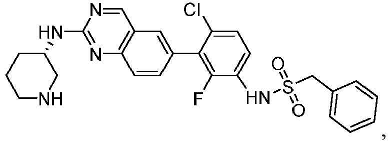 |
| 1009 | 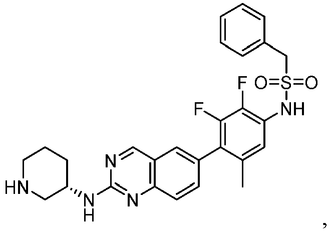 |
| 1010 | 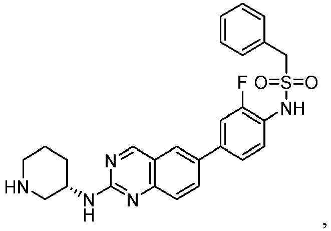 |
| 1011 | 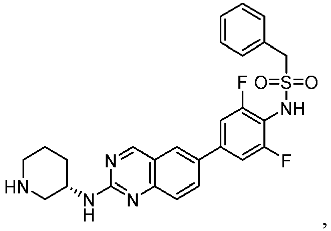 |
| 1012 | 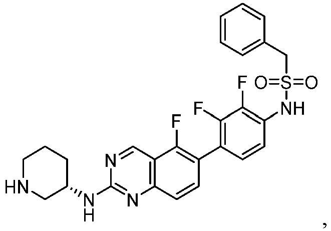 |
| 1013 | 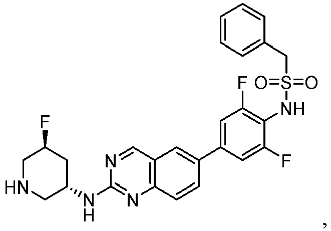 |
| 1014 | 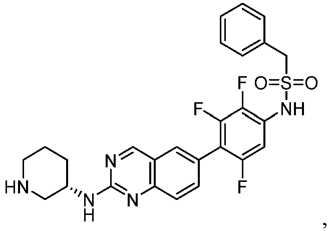 |
| 1015 | 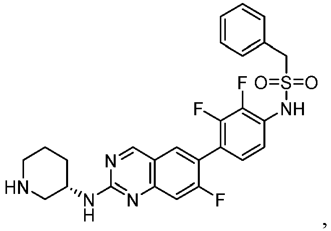 |
| 1016 | 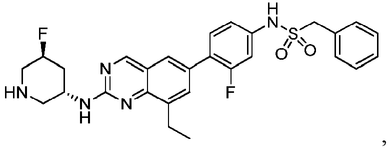 |
| 1017 | 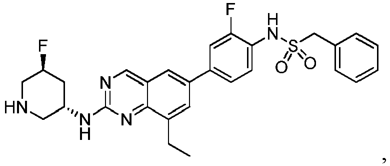 |
| 1018 | 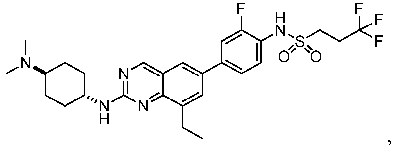 |
| 600 | 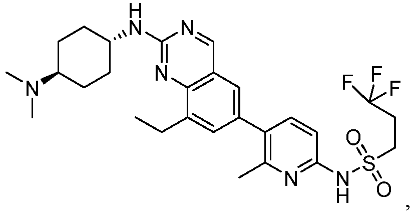 |
| 601 | 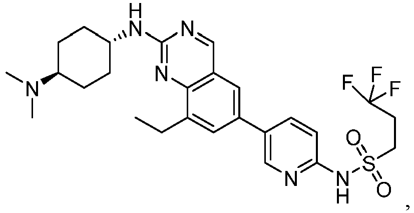 |
| 602 | 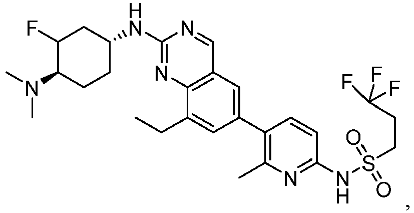 |
| 603 | 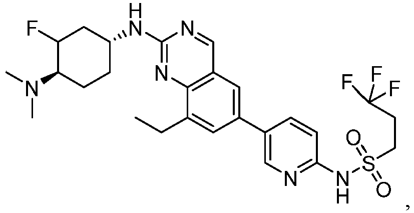 |
| 604 | 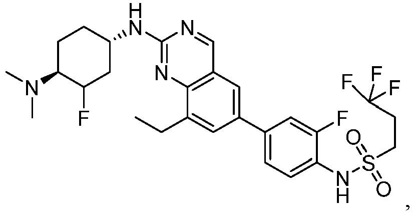 |
| 605 | 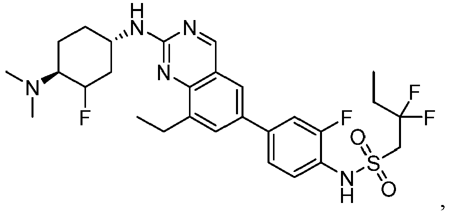 |
| 606 | 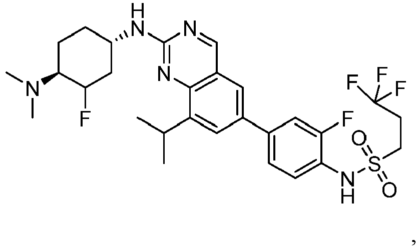 |
| 607 | 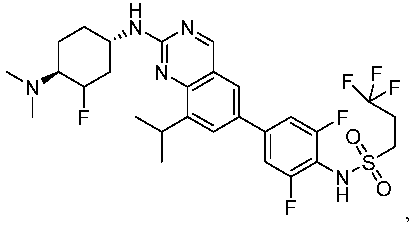 |
| 608 | 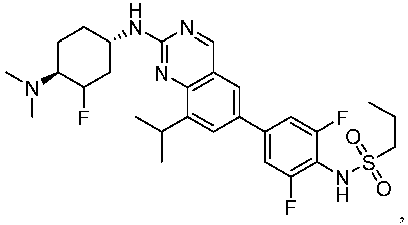 |
| 609 | 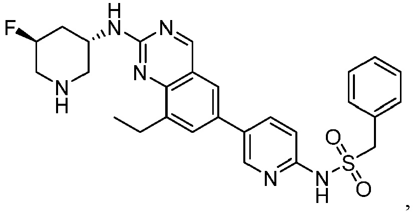 |
| 610 | 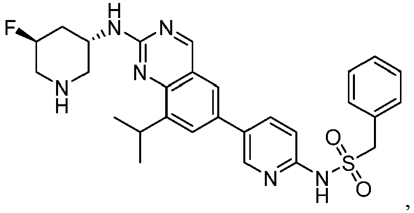 |
| 611 | 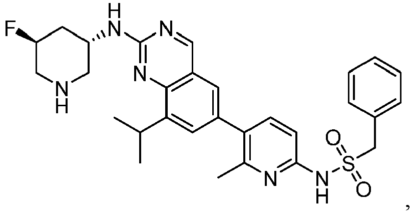 |
| 612 | 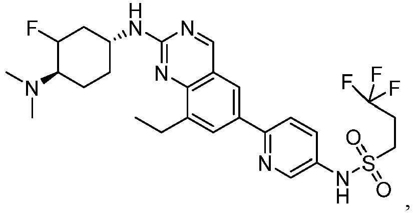 |
| 613 | 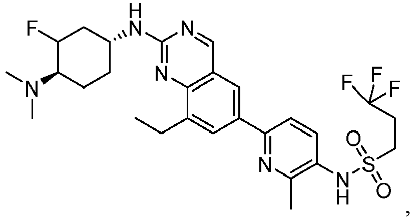 |
| 614 | 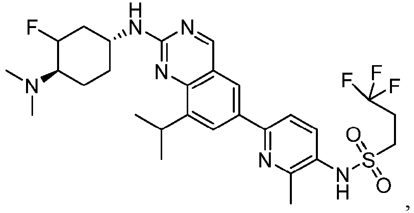 |
| 615 | 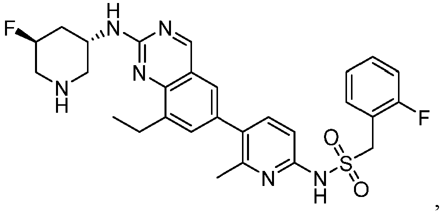 |
| 616 | 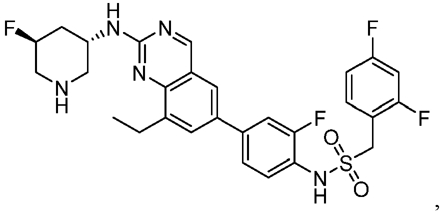 |
| 617 | 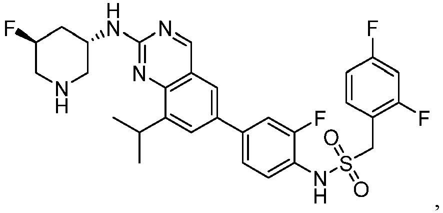 |
| 618 | 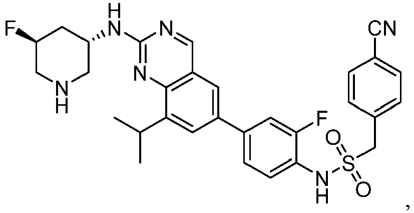 |
| 619 | 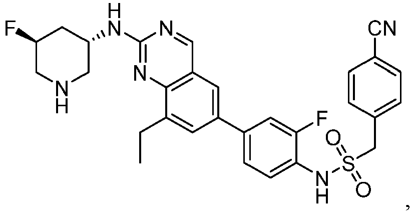 |
| 620 | 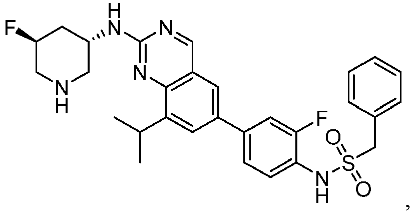 |
| 621 | 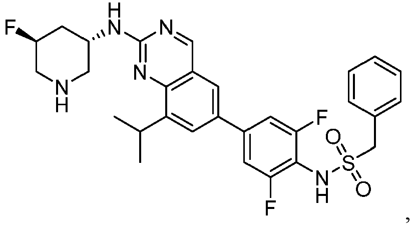 |
| 622 | 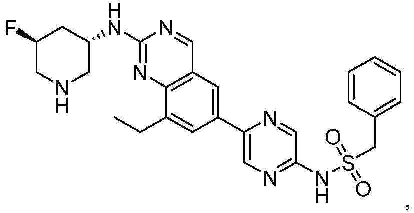 |
| 623 | 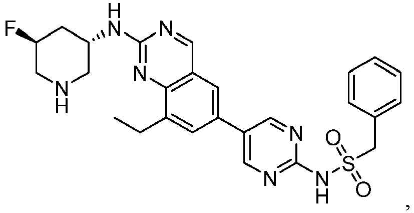 |
| 624 | 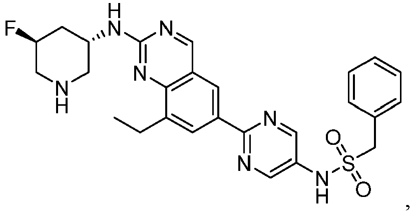 |
| 625 | 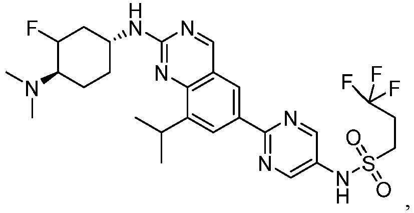 |
| 626 | 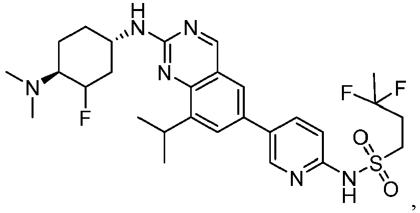 |
| 627 | 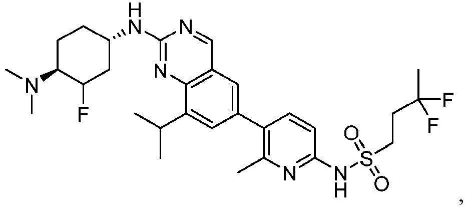 |
| 631 | 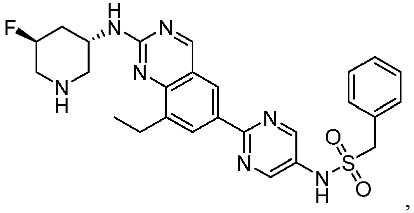 |
| 632 | 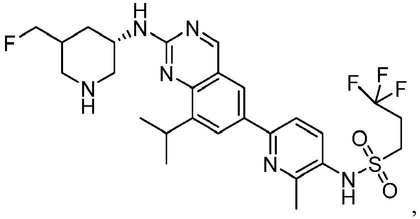 |
| 633 | 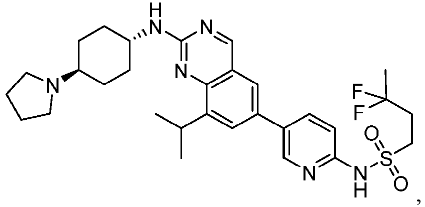 |
| 634 | 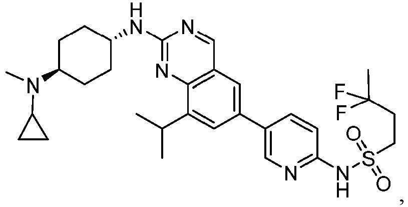 |
| 635 | 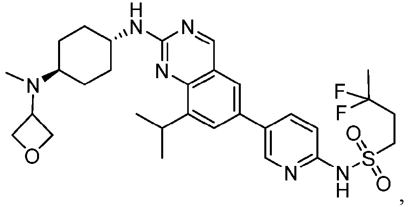 |
| 636 | 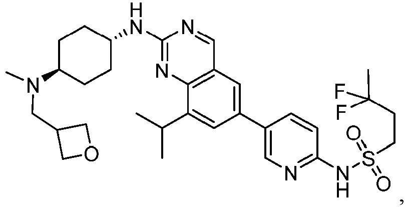 and |
| 639 | 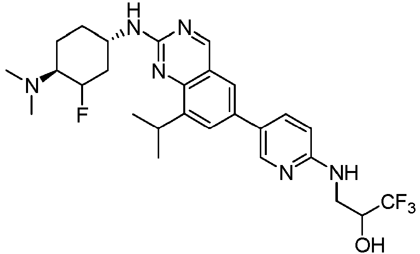 |

18. A pharmaceutical composition comprising a compound or pharmaceutically acceptable salt thereof of any one of claims 1 to 17 and one or more pharmaceutically acceptable excipients.

19. A compound according to any one of claims 1 to 17 or a pharmaceutically acceptable salt thereof, or the pharmaceutical composition of claim 18, for use in a method for treating an IRE1-related disease or disorder, wherein the IRE1-related disease or disorder is cancer.

## Patentansprüche

1. Verbindung mit der Formel (A): 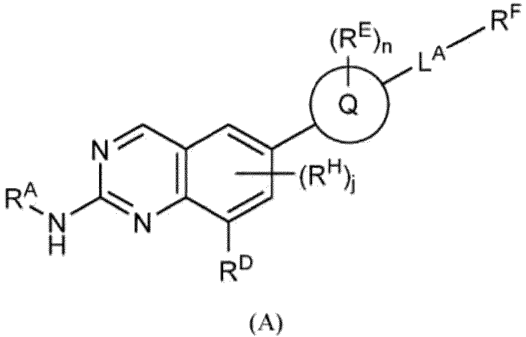 oder ein Stereoisomer, Tautomer oder pharmazeutisch annehmbares Salz davon, wobei:
Ring Q R^{E}-substituiertes oder unsubstituiertes C₅₋₇-Aryl oder R^{E}-substituiertes oder unsubstituiertes 6-gliedriges Heteroaryl ist, umfassend mindestens ein Stickstoff-Heteroatom;
L^{A} -NHSO₂-, -SO₂NH- oder -NH- ist;
R^{A} 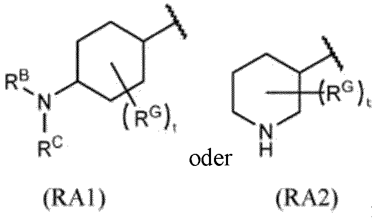 ist;
R^{B} und R^{C} unabhängig Stickstoff oder R^{N}-substituiertes oder unsubstituiertes C₁₋₆-Alkyl, R^{N}-substituiertes oder unsubstituiertes C₃₋₇-Cycloalkyl oder R^{N}-substituiertes oder unsubstituiertes 3- bis 7-gliedriges Heterocycloalkyl ist, oder R^{B} und R^{C} mit dem Stickstoffatom zusammengenommen sind, an dem sie befestigt sind, um ein R^{N}-substituiertes oder unsubstituiertes 4- bis 7-gliedriges Heterocycloalkyl zu bilden;
R^{D} Wasserstoff, Halogen, -CN, -OR^{I}, -S(O)₂R^{I}, -NR^{J}R^{K}, R^{N}-substituiertes oder unsubstituiertes C₁₋₆-Alkyl, R^{N}-substituiertes oder unsubstituiertes C₁₋₆-Haloalkyl, R^{N}-substituiertes oder unsubstituiertes C₃₋₇-Cycloalkyl, R^{N}-substituiertes oder unsubstituiertes 3-bis 7-gliedriges Heterocycloalkyl, R^{N}-substituiertes oder unsubstituiertes C₅₋₇-Aryl oder R^{N}-substituiertes oder unsubstituiertes 5- bis 7-gliedriges Heteroaryl ist;
jedes R^{E} Wasserstoff, Halogen, -OR^{I}, -CN, -S(O)₂R^{I}, R^{M}-substituiertes oder unsubstituiertes C₁₋₆-Alkyl, R^{M}-substituiertes oder unsubstituiertes C₁₋₆-Haloalkyl oder R^{M}-substituiertes oder unsubstituiertes C₃₋₇-Cycloalkyl ist;
N 0, 1, 2, 3 oder 4 ist;
R^{F} R^{N}-substituiertes oder unsubstituiertes C₁₋₆-Alkyl, R^{N}-substituiertes oder unsubstituiertes C₃₋₇-Cycloalkyl, R^{N}-substituiertes oder unsubstituiertes Benzyl oder R^{N}-substituiertes oder unsubstituiertes 3- bis 7-gliedriges Heterocycloalkyl ist;
jedes R^{G} unabhängig Halogen, -OR^{I}, R^{M}-substituiertes oder unsubstituiertes C₁₋₆-Alkyl oder R^{M}-substituiertes oder unsubstituiertes C₁₋₆-Haloalkyl ist;
t 0, 1, 2, 3, 4, 5 oder 6 ist;
jedes R^{H} unabhängig Halogen, C₁₋₃-Alkyl, C₁₋₃-Haloalkyl oder Cyclopropyl ist;
j 0, 1 oder 2 ist;
jedes R^{I}, R^{J} und R^{K} unabhängig Wasserstoff, R^{M}-substituiertes oder unsubstituiertes C₁₋₆-Alkyl, R^{M}-substituiertes oder unsubstituiertes C₁₋₆-Haloalkyl, R^{M}-substituiertes oder unsubstituiertes C₃₋₇-Cycloalkyl, R^{M}-substituiertes oder unsubstituiertes 3- bis 7-gliedriges Heterocycloalkyl, R^{M}-substituiertes oder unsubstituiertes C₅₋₇-gliedriges Aryl oder R^{M}-substituiertes oder unsubstituiertes 5- bis 7-gliedriges Heteroaryl ist;
jedes R^{M} unabhängig Wasserstoff, Halogen, -CN, -OH, -OCH₃, -OCF₃, -S(O)₂H, - S(O)₂NH₂, -NH₂, -NH(CH₃), -N(CH₃)₂, -CF₃, -CHF₂, -CH₂F, -C(CH₃)₂F, -C(CH₃)F₂, unsubstituiertes C₁₋₆-Alkoxy, unsubstituiertes C₁₋₆-Alkyl, unsubstituiertes C₁₋₆-Haloalkyl, unsubstituiertes C₃₋₇-Cycloalkyl, unsubstituiertes 3- bis 6-gliedriges Heterocycloalkyl, unsubstituiertes C₅₋₇-Aryl oder unsubstituiertes 5- bis 7-gliedriges Heteroaryl ist;
jedes R^{N} unabhängig Wasserstoff, Halogen, -CN, -OR^{R}, -S(O)₂R^{R}, -NR^{S}R^{T}, R^{P}-substituiertes oder unsubstituiertes C₁₋₆-Alkyl, R^{P}-substituiertes oder unsubstituiertes C₁₋₆-Haloalkyl, R^{P}-substituiertes oder unsubstituiertes C₁₋₆-Alkoxy, R^{P}-substituiertes oder unsubstituiertes C₃₋₇-Cycloalkyl, R^{P}-substituiertes oder unsubstituiertes 3- bis 6-gliedriges Heterocycloalkyl, R^{P}-substituiertes oder unsubstituiertes C₅₋₇-Aryl oder R^{P}-substituiertes oder unsubstituiertes 5- bis 7-gliedriges Heteroaryl ist;
jedes R^{R}, R^{S} und R^{T} unabhängig Wasserstoff, R^{V}-substituiertes oder unsubstituiertes C₁₋₆-Alkyl, R^{V}-substituiertes oder unsubstituiertes C₁₋₆-Haloalkyl, R^{V}-substituiertes oder unsubstituiertes C₃₋₇-Cycloalkyl oder R^{V}-substituiertes oder unsubstituiertes 3- bis 7-gliedriges Heterocycloalkyl ist;
jedes R^{V} unabhängig Wasserstoff, Halogen, -CN, -OH, -OCH₃, -OCF₃, -S(O)₂H, - S(O)₂NH₂, -NH₂, -NH(CH₃), -N(CH₃)₂, -CF₃, -CHF₂, -CH₂F, -C(CH₃)₂F, -C(CH₃)F₂, unsubstituiertes C₁₋₆-Alkoxy, unsubstituiertes C₁₋₆-Alkyl, unsubstituiertes C₁₋₆-Haloalkyl, unsubstituiertes C₃₋₇-Cycloalkyl, unsubstituiertes 3- bis 6-gliedriges Heterocycloalkyl, unsubstituiertes C₅₋₇-Aryl oder unsubstituiertes 5- bis 7-gliedriges Heteroaryl ist;
jedes R^{P} unabhängig Wasserstoff, Halogen, -CN, -OR^{W}, -S(O)₂R^{W}, -NR^{X}R^{Y}, R^{U}-substituiertes oder unsubstituiertes C₁₋₆-Alkyl, R^{U}-substituiertes oder unsubstituiertes C₁₋₆-Haloalkyl, R^{U}-substituiertes oder unsubstituiertes C₃₋₇-Cycloalkyl, R^{U}-substituiertes oder unsubstituiertes 3- bis 6-gliedriges Heterocycloalkyl, R^{U}-substituiertes oder unsubstituiertes C₅₋₇-Aryl oder R^{U}-substituiertes oder unsubstituiertes 5- bis 7-gliedriges Heteroaryl ist;
jedes R^{W}, R^{X} und R^{Y} unabhängig Wasserstoff, unsubstituiertes C₁₋₆-Alkyl, unsubstituiertes C₁₋₆-Haloalkyl, unsubstituiertes C₃₋₇-Cycloalkyl oder unsubstituiertes 3- bis 7-gliedriges Heterocycloalkyl ist, und
jedes R^{U} unabhängig Wasserstoff, Halogen, -CN, -OH, -OCH₃, -OCF₃, -S(O)₂H, - S(O)₂NH₂, -NH₂, -NH(CH₃), -N(CH₃)₂, -CF₃, -CHF₂, -CH₂F, -C(CH₃)₂F, -C(CH₃)F₂, unsubstituiertes C₁₋₆-Alkoxy, unsubstituiertes C₁₋₆-Alkyl, unsubstituiertes C₁₋₆-Haloalkyl, unsubstituiertes C₃₋₇-Cycloalkyl, unsubstituiertes 3- bis 6-gliedriges Heterocycloalkyl, unsubstituiertes C₅₋₇-Aryl oder unsubstituiertes 5- bis 7-gliedriges Heteroaryl ist;
wobei sich Cycloalkyl auf nicht-aromatische, gesättigte oder ungesättigte, zyklische, einwertige Kohlenwasserstoffstrukturen bezieht, die aus einem Ring oder mehreren Ringen bestehen können, wobei ein Cycloalkyl, das mehr als einen Ring umfasst, verschmolzenes, Spiro- oder verbrücktes Cycloalkyl oder Kombinationen davon sein kann; und
wobei sich Heterocycloalkyl auf nicht-aromatische, gesättigte oder ungesättigte, zyklische, einwertige Kohlenwasserstoffstrukturen bezieht, wobei ein oder mehrere aus den Ring-Kohlenstoffatomen durch ein Heteroatom ersetzt wurden, die aus einem Ring oder mehreren Ringen bestehen können, wobei ein Heterocycloalkyl, das mehr als einen Ring umfasst, verschmolzenes, Spiro- oder verbrücktes Heterocycloalkyl oder Kombinationen davon sein kann.

2. Verbindung oder pharmazeutisch annehmbares Salz davon nach Anspruch 1, wobei die Verbindung die folgende Formel aufweist:

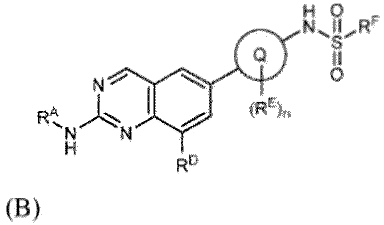

3. Verbindung oder pharmazeutisch annehmbares Salz davon nach Anspruch 1 oder 2, wobei R^{D} Halogen, -OR^{I}, -NR^{J}R^{K}, R^{N}-substituiertes oder unsubstituiertes C₁₋₆-Alkyl, R^{N}-substituiertes oder unsubstituiertes C₁₋₆-Haloalkyl, R^{N}-substituiertes oder unsubstituiertes C₃₋₇-Cycloalkyl oder R^{N}-substituiertes oder unsubstituiertes 3- bis 7-gliedriges Heterocycloalkyl ist.

4. Verbindung oder pharmazeutisch annehmbares Salz davon nach einem der Ansprüche 1 bis 3, wobei R^{D} R^{N}-substituiertes oder unsubstituiertes C₁₋₆-Alkyl oder R^{N}-substituiertes oder unsubstituiertes C₁₋₆-Haloalkyl ist.

5. Verbindung oder pharmazeutisch annehmbares Salz davon nach Anspruch 4, wobei R^{D} Methyl, Ethyl, Propyl oder Isopropyl ist.

6. Verbindung oder pharmazeutisch annehmbares Salz davon nach einem der Ansprüche 1 bis 5, wobei R^{E} F ist und n 1, 2 oder 3 ist.

7. Verbindung oder pharmazeutisch annehmbares Salz davon nach einem der Ansprüche 1 bis 6, wobei L^{A} -NHSO₂- ist.

8. Verbindung oder pharmazeutisch annehmbares Salz davon nach einem der Ansprüche 1 bis 7, wobei R^{F} R^{N}-substituiertes oder unsubstituiertes C₁₋₆-Alkyl oder R^{N}-substituiertes oder unsubstituiertes Benzyl ist.

9. Verbindung oder pharmazeutisch annehmbares Salz davon nach Anspruch 8, wobei R^{N} Wasserstoff, Halogen, -OH, -CN, -CF₃, -CHF₂, -CH₂F, -C(CH₃)₂F, -C(CH₃)F₂, Methyl, Ethyl oder Propyl ist.

10. Verbindung oder pharmazeutisch annehmbares Salz davon nach Anspruch 8, wobei R^{F} Folgendes ist: 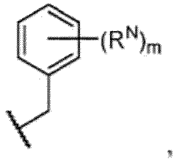
wobei R^{N} Halogen ist und m 1 oder 2 ist; oder
wobei R^{F} Folgendes ist: 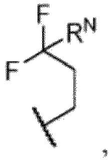
wobei R^{N} F oder Methyl ist.

11. Verbindung oder pharmazeutisch annehmbares Salz davon nach einem der Ansprüche 1 bis 10, wobei R^{A} Folgendes ist: 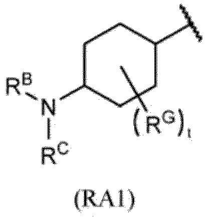 wobei
R^{B} und R^{C} jeweils unabhängig R^{N}-substituiertes oder unsubstituiertes C₁₋₆-Alkyl sind; oder
R^{B} 4-gliedriges Heterocycloalkyl ist und R^{C} Wasserstoff oder R^{N}-substituiertes oder unsubstituiertes C₁₋₆-Alkyl ist.

12. Verbindung oder pharmazeutisch annehmbares Salz davon nach einem der Ansprüche 1 bis 10, wobei R^{A} Folgendes ist:

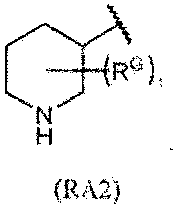

13. Verbindung oder pharmazeutisch annehmbares Salz davon nach einem der Ansprüche 1 bis 12, wobei R^{G} unabhängig Halogen oder R^{M}-substituiertes oder unsubstituiertes C₁₋₆-Alkyl ist.

14. Verbindung oder pharmazeutisch annehmbares Salz davon nach einem der Ansprüche 1 bis 13, wobei R^{G} unabhängig Fluor ist und t 1 oder 2 ist.

15. Verbindung oder pharmazeutisch annehmbares Salz davon nach einem der Ansprüche 1 bis 14, wobei R^{H} Halogen ist und j 1 ist.

16. Verbindung oder pharmazeutisch annehmbares Salz davon nach einem der Ansprüche 1 bis 15, wobei Ring Q die folgende Formel aufweist: 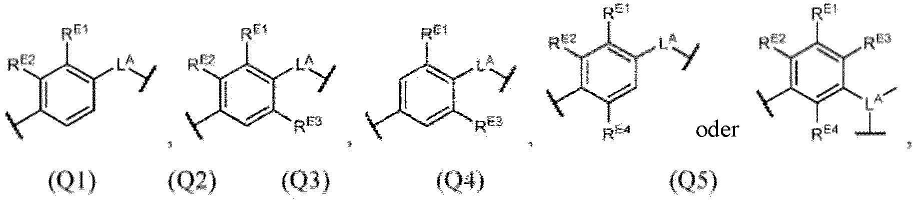 wobei R^{E1}, R^{E2}, R^{E3} und R^{E4} jeweils unabhängig Wasserstoff, Halogen oder R^{M}-substituiertes oder unsubstituiertes C₁₋₆-Alkyl sind.

17. Verbindung oder pharmazeutisch annehmbares Salz davon nach Anspruch 1, ausgewählt aus der Gruppe bestehend aus
| Verbindung Nr. | Struktur |
|---|---|
| 1001 | 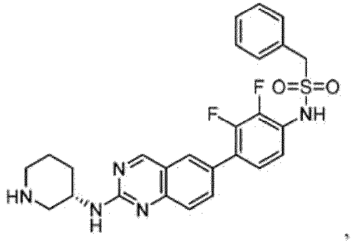 |
| 1002 | 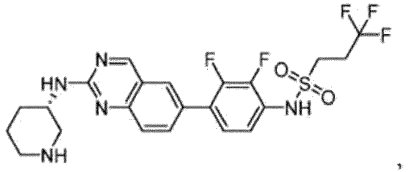 |
| 1003 | 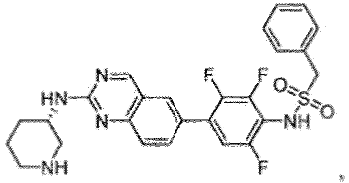 |
| 1004 | 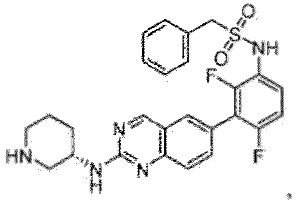 |
| 1005 | 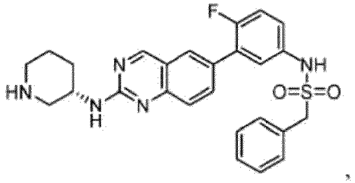 |
| 1006 | 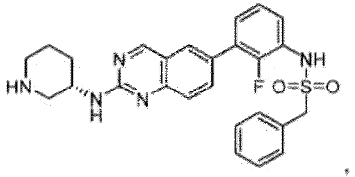 |
| 1007 | 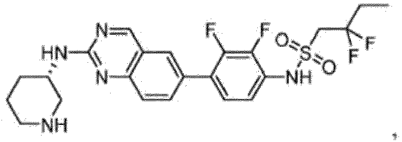 |
| 1008 | 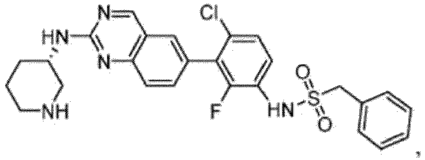 |
| 1009 | 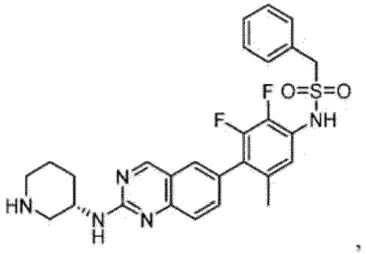 |
| 1010 | 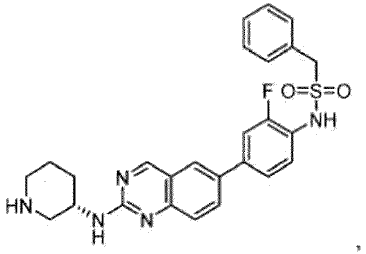 |
| 1011 | 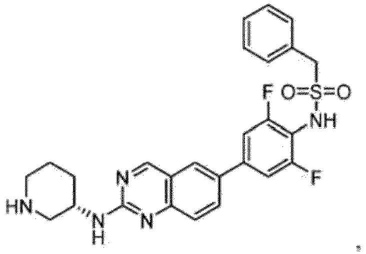 |
| 1012 | 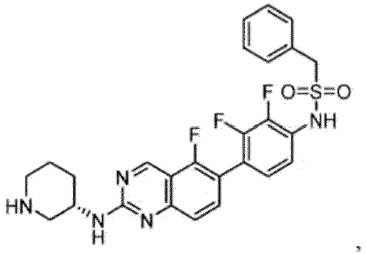 |
| 1013 | 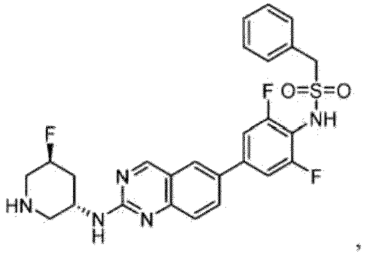 |
| 1014 | 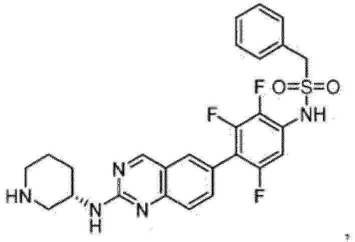 |
| 1015 | 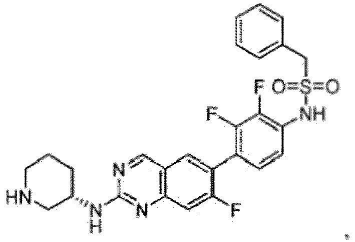 |
| 1016 | 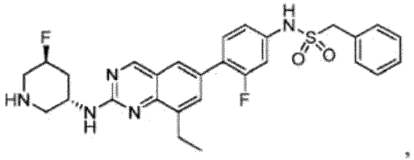 |
| 1017 | 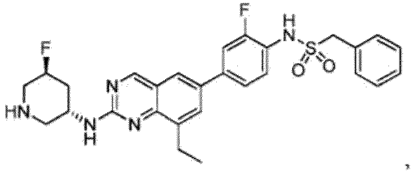 |
| 1018 | 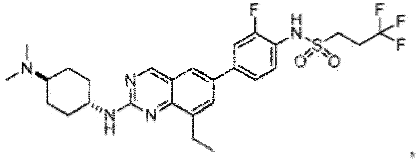 |
| 600 | 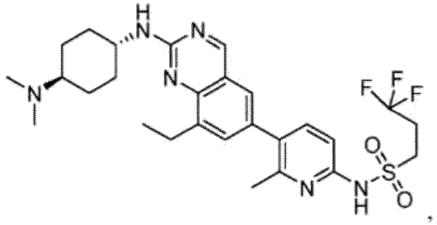 |
| 601 | 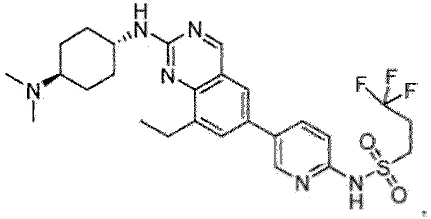 |
| 602 | 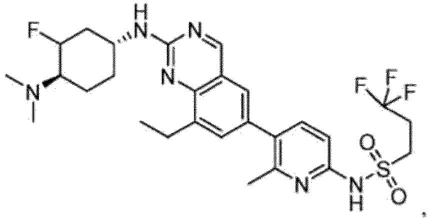 |
| 603 | 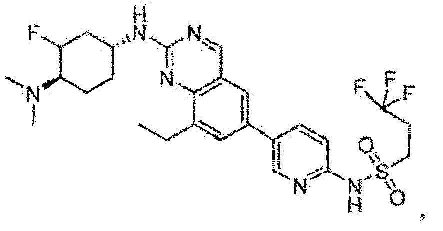 |
| 604 | 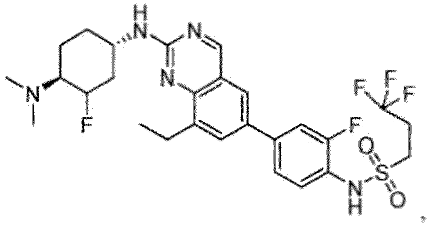 |
| 605 | 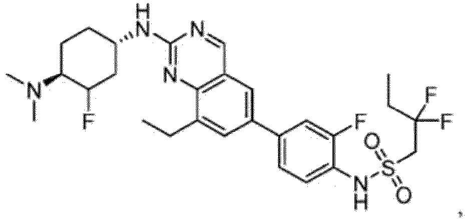 |
| 606 | 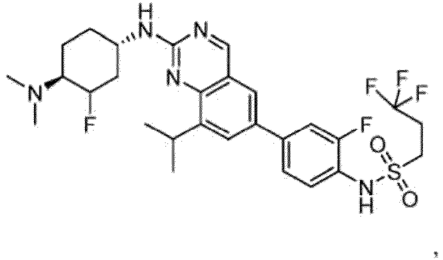 |
| 607 | 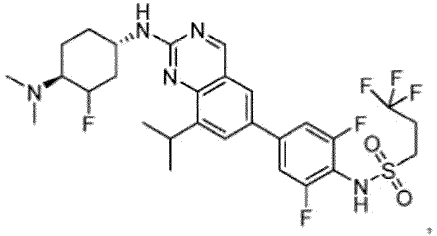 |
| 608 | 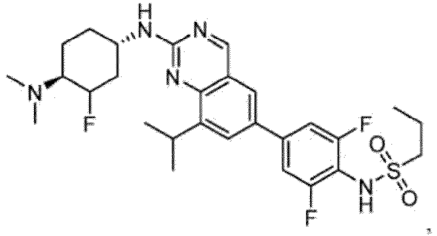 |
| 609 | 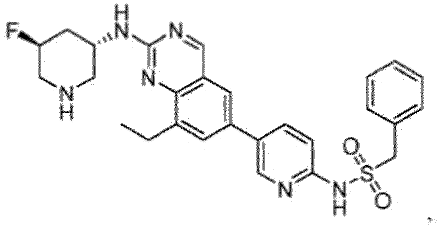 |
| 610 | 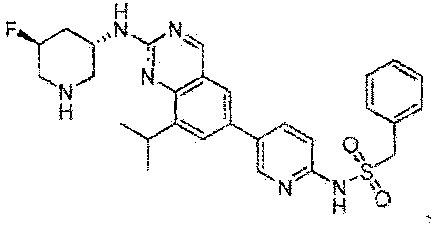 |
| 611 | 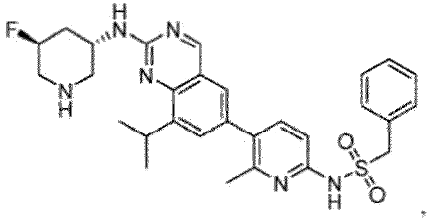 |
| 612 | 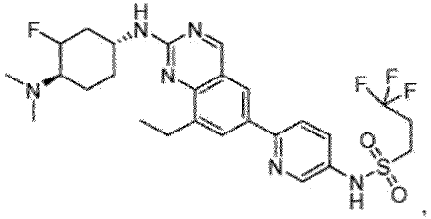 |
| 613 | 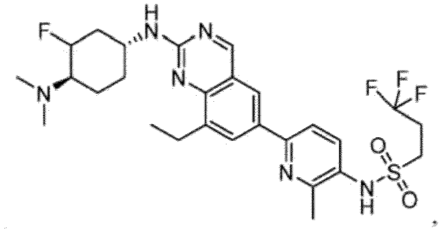 |
| 614 | 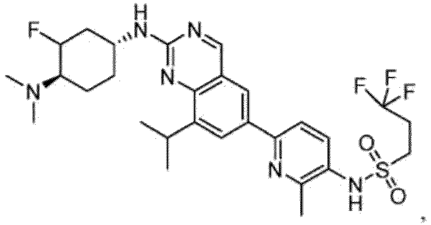 |
| 615 | 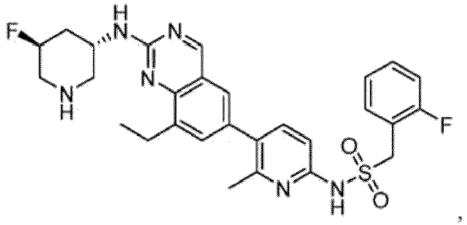 |
| 616 | 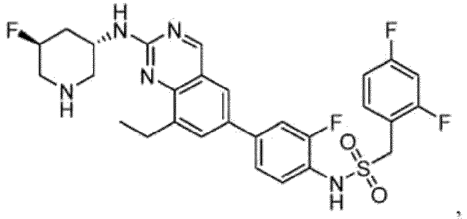 |
| 617 | 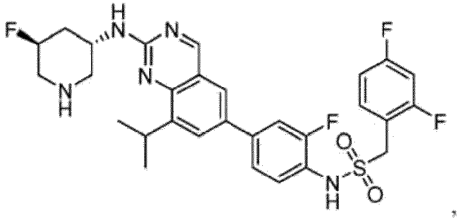 |
| 618 | 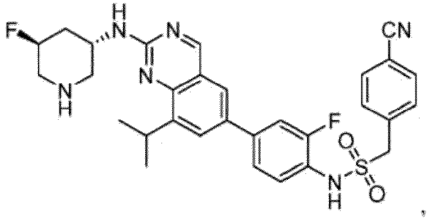 |
| 619 | 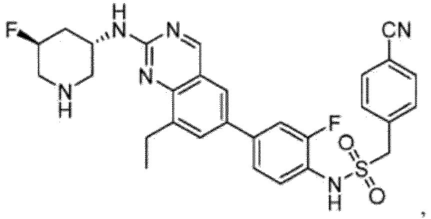 |
| 620 | 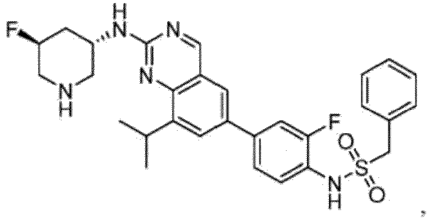 |
| 621 | 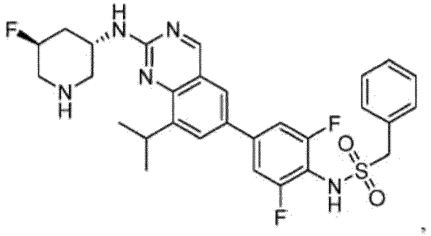 |
| 622 | 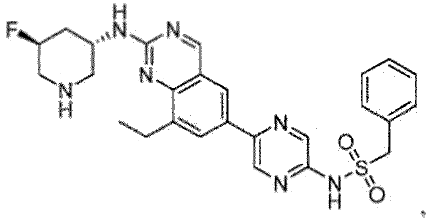 |
| 623 | 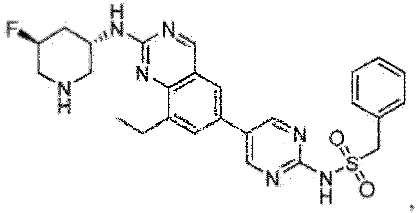 |
| 624 | 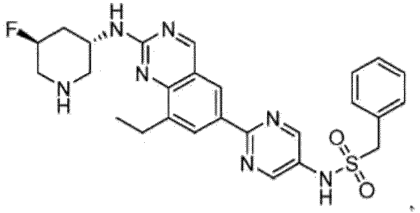 |
| 62 5 | 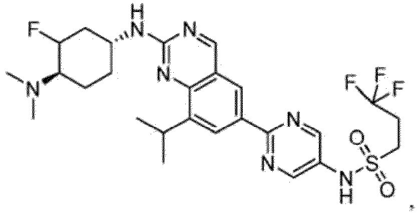 |
| 626 | 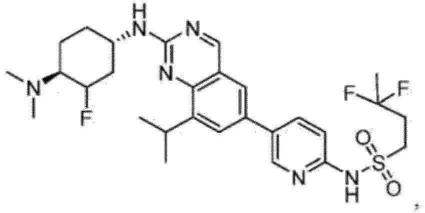 |
| 627 | 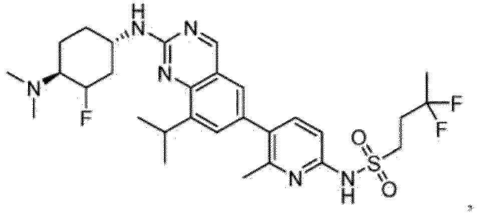 |
| 631 | 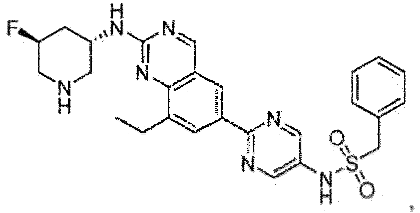 |
| 632 | 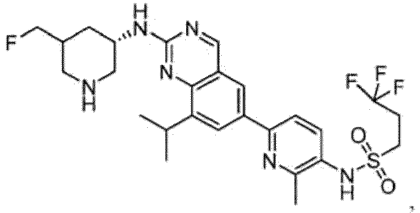 |
| 633 | 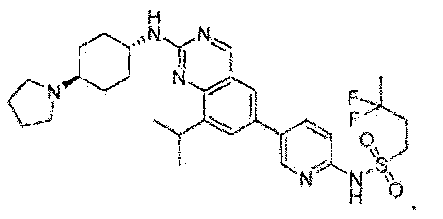 |
| 634 | 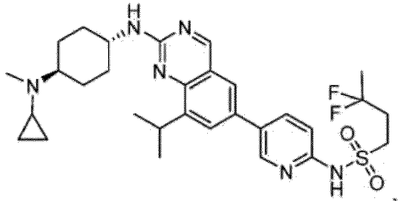 |
| 63 5 | 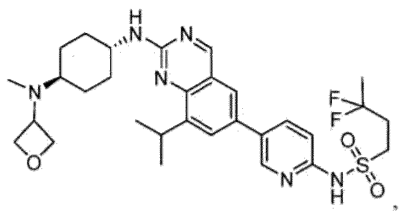 |
| 636 | 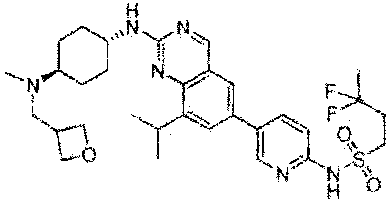 und |
| 639 | 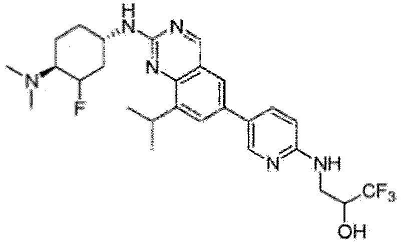 |

18. Pharmazeutische Zusammensetzung, umfassend eine Verbindung oder ein pharmazeutisch annehmbares Salz davon nach einem der Ansprüche 1 bis 17 und einen oder mehrere pharmazeutisch annehmbare Hilfsstoffe.

19. Verbindung nach einem der Ansprüche 1 bis 17 oder ein pharmazeutisch annehmbares Salz davon oder die pharmazeutische Zusammensetzung nach Anspruch 18, zur Verwendung in einem Verfahren zum Behandeln einer IRE1-bedingten Erkrankung oder Störung, wobei die IRE1-bedingte Erkrankung oder Störung Krebs ist.

## Revendications

1. Composé, ayant la formule (A) : 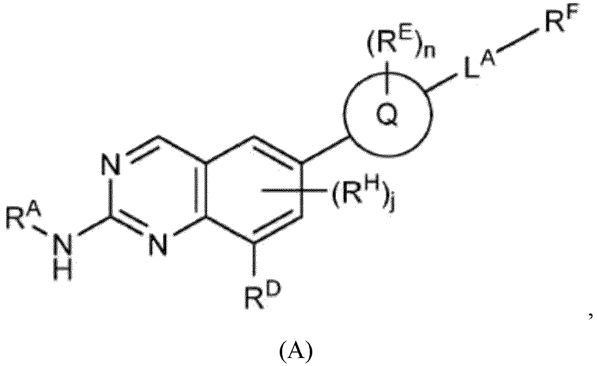 ou un stéréoisomère, tautomère, ou sel pharmaceutiquement acceptable de celui-ci, dans lequel :
l'anneau Q est aryle en C₅₋₇ à R^{E} substitué ou non substitué, ou hétéroaryle, à 6 membres, à R^{E} substitué ou non substitué comprenant au moins un hétéroatome d'azote ;
L^{A} est -NHSO₂-, -SO₂NH-, ou -NH- ;
R^{A} est 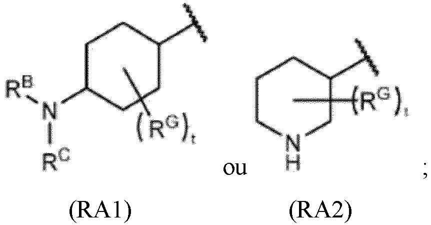
R^{B} et R^{C} sont indépendamment hydrogène ou alkyle en C₁₋₆ à R^{N} substitué ou non substitué, cycloalkyle en C₃₋₇ à R^{N} substitué ou non substitué, ou hétérocycloalkyle, à 3 à 7 membres, à R^{N} substitué ou non substitué, ou R^{B} et R^{C} sont pris conjointement avec l'atome d'azote auquel ils sont attachés pour former un hétérocycloalkyle, à 4 à 7 membres, à R^{N} substitué ou non substitué ;
R^{D} est hydrogène, halogène, -CN, -OR^{I}, -S(O)₂R^{I}, -NR^{J}R^{K}, alkyle en C₁₋₆ à R^{N} substitué ou non substitué, haloalkyle en C₁₋₆ à R^{N} substitué ou non substitué, cycloalkyle en C₃₋₇ à R^{N} substitué ou non substitué, hétérocycloalkyle, à 3 à 7 membres, à R^{N} substitué ou non substitué, aryle en C₅₋₇ à R^{N} substitué ou non substitué, ou hétéroaryle, à 5 à 7 membres, à R^{N} substitué ou non substitué ;
chaque R^{E} est hydrogène, halogène, -OR^{I}, -CN, -S(O)₂R^{I}, alkyle en C₁₋₆ à R^{M} substitué ou non substitué, haloalkyle en C₁₋₆ à R^{M} substitué ou non substitué, ou cycloalkyle en C₃₋₇ à R^{M} substitué ou non substitué ;
n est 0, 1, 2, 3, ou 4 ;
R^{F} est alkyle en C₁₋₆ à R^{N} substitué ou non substitué, cycloalkyle en C₃₋₇ à R^{N} substitué ou non substitué, benzyle à R^{N} substitué ou non substitué, ou hétérocycloalkyle, à 3 à 7 membres, à R^{N} substitué ou non substitué ;
chaque R^{G} est indépendamment halogène, -OR^{I}, alkyle en C₁₋₆ à R^{M} substitué ou non substitué, ou haloalkyle en C₁₋₆ à R^{M} substitué ou non substitué ;
t est 0, 1, 2, 3, 4, 5, ou 6 ;
chaque R^{H} est indépendamment halogène, alkyle en C₁₋₃, haloalkyle en C₁₋₃, ou cyclopropyle ;
j est 0, 1, ou 2 ;
chaque R^{I}, R^{J}, et R^{K} est indépendamment hydrogène, alkyle en C₁₋₆ à R^{M} substitué ou non substitué, haloalkyle en C₁₋₆ à R^{M} substitué ou non substitué, cycloalkyle en C₃₋₇ à R^{M} substitué ou non substitué, hétérocycloalkyle, à 3 à 7 membres, à R^{M} substitué ou non substitué, aryle à membres en C₅₋₇ à R^{M} substitué ou non substitué, ou hétéroaryle, à 5 à 7 membres, à R^{M} substitué ou non substitué ;
chaque R^{M} est indépendamment hydrogène, halogène, -CN, -OH, -OCH₃, -OCF₃, -S(O)₂H, - S(O)₂NH₂, -NH₂, -NH(CH₃), -N(CH₃)₂, -CF₃, -CHF₂, -CH₂F, -C(CH₃)₂F, -C(CH₃)F₂, alkoxy en C₁₋₆ non substitué, alkyle en C₁₋₆ non substitué, haloalkyle en C₁₋₆ non substitué, cycloalkyle en C₃₋₇ non substitué, hétérocycloalkyle, à 3 à 6 membres, non substitué, aryle en C₅₋₇ non substitué, ou hétéroaryle, à 5 à 7 membres, non substitué ;
chaque R^{N} est indépendamment hydrogène, halogène, -CN, -OR^{R}, -S(O)₂R^{R}, -NR^{S}R^{T}, alkyle en C₁₋₆ à R^{P} substitué ou non substitué, haloalkyle en C₁₋₆ à R^{P} substitué ou non substitué, alkoxy en C₁₋₆ à R^{P} substitué ou non substitué, cycloalkyle en C₃₋₇ à R^{P} substitué ou non substitué, hétérocycloalkyle, à 3 à 6 membres, à R^{P} substitué ou non substitué, aryle en C₅₋₇ à R^{P} substitué ou non substitué, ou hétéroaryle, à 5 à 7 membres, à R^{P} substitué ou non substitué ;
chaque R^{R}, R^{S}, et R^{T} est indépendamment hydrogène, alkyle en C₁₋₆ à R^{V} substitué ou non substitué, haloalkyle en C₁₋₆ à R^{V} substitué ou non substitué, cycloalkyle en C₃₋₇ à R^{V} substitué ou non substitué, ou hétérocycloalkyle, à 3 à 7 membres, à R^{V} substitué ou non substitué ;
chaque R^{V} est indépendamment hydrogène, halogène, -CN, -OH, -OCH₃, -OCF₃, -S(O)₂H, -S(O)₂NH₂, -NH₂, -NH(CH₃), -N(CH₃)₂, -CF₃, -CHF₂, -CH₂F, -C(CH₃)₂F, -C(CH₃)F₂, alkoxy en C₁₋₆ non substitué, alkyle en C₁₋₆ non substitué, haloalkyle en C₁₋₆ non substitué, cycloalkyle en C₃₋₇ non substitué, hétérocycloalkyle, à 3 à 6 membres non substitué, aryle en C₅₋₇ non substitué, ou hétéroaryle, à 5 à 7 membres, non substitué ;
chaque R^{P} est indépendamment hydrogène, halogène, -CN, -OR^{W}, -S(O)₂R^{W}, -NR^{X}R^{Y}, alkyle en C₁₋₆ à R^{U} substitué ou non substitué, haloalkyle en C₁₋₆ à R^{U} substitué ou non substitué, cycloalkyle en C₃₋₇ à R^{U} substitué ou non substitué, hétérocycloalkyle, à 3 à 6 membres à R^{U} substitué ou non substitué, aryle en C₅₋₇ à R^{U} substitué ou non substitué, ou hétéroaryle, à 5 à 7 membres, à R^{U} substitué ou non substitué ;
chaque R^{W}, R^{X}, et R^{Y} est indépendamment hydrogène, alkyle en C₁₋₆ non substitué, haloalkyle en C₁₋₆ non substitué, cycloalkyle en C₃₋₇ non substitué, ou hétérocycloalkyle, à 3 à 7 membres, non substitué, et
chaque R^{U} est indépendamment hydrogène, halogène, -CN, -OH, -OCH₃, -OCF3, -S(O)₂H, - S(O)₂NH₂, -NH₂, -NH(CH₃), -N(CH₃)₂, -CF₃, -CHF₂, -CH₂F, -C(CH₃)₂F, -C(CH₃)F₂, alkoxy en C₁₋₆ non substitué, alkyle en C₁₋₆ non substitué, haloalkyle en C₁₋₆ non substitué, cycloalkyle en C₃₋₇ non substitué, hétérocycloalkyle, à 3 à 6 membres, non substitué, aryle en C₅₋₇ non substitué, ou hétéroaryle, à 5 à 7 membres, non substitué ;
dans lequel cycloalkyle fait référence à des structures hydrocarbure univalentes cycliques non aromatiques saturées ou non saturées, qui peuvent être constituées d'un anneau ou de multiples anneaux, dans lequel un cycloalkyle comprenant plus d'un anneau peut être fusionné, spiro, ou ponté, ou des combinaisons de ceux-ci ; et
dans lequel hétérocycloalkyle fait référence à des structures hydrocarbure univalentes cycliques non aromatiques saturées ou non saturées où un ou plusieurs des atomes de carbone d'anneau ont été remplacés par un hétéroatome, qui peuvent être constituées d'un anneau ou de multiples anneaux, dans lequel un hétérocycloalkyle comprenant plus d'un anneau peut être fusionné, spiro, ou ponté, ou des combinaisons de ceux-ci.

2. Composé ou sel pharmaceutiquement acceptable de celui-ci de la revendication 1, dans lequel le composé a la formule :

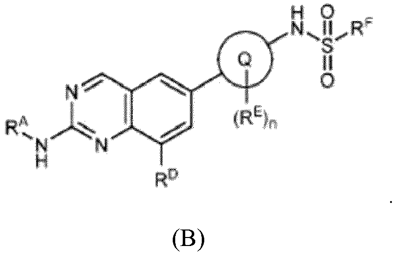

3. Composé ou sel pharmaceutiquement acceptable de celui-ci de la revendication 1 ou 2,
dans lequel R^{D} est halogène, -OR^{I}, -NR^{J}R^{K}, alkyle en C₁₋₆ à R^{N} substitué ou non substitué, haloalkyle en C₁₋₆ à R^{N} substitué ou non substitué, cycloalkyle en C₃₋₇ à R^{N} substitué ou non substitué, ou hétérocycloalkyle, à 3 à 7 membres, à R^{N} substitué ou non substitué.

4. Composé ou sel pharmaceutiquement acceptable de celui-ci de l'une quelconque des revendications 1 à 3, dans lequel R^{D} est alkyle en C₁₋₆ à R^{N} substitué ou non substitué ou haloalkyle en C₁₋₆ à R^{N} substitué ou non substitué.

5. Composé ou sel pharmaceutiquement acceptable de celui-ci de la revendication 4, dans lequel R^{D} est méthyle, éthyle, propyle, ou isopropyle.

6. Composé ou sel pharmaceutiquement acceptable de celui-ci de l'une quelconque des revendications 1 à 5, dans lequel R^{E} est F et n est 1, 2, ou 3.

7. Composé ou sel pharmaceutiquement acceptable de celui-ci de l'une quelconque des revendications 1 à 6, dans lequel L^{A} est -NHSO₂-.

8. Composé ou sel pharmaceutiquement acceptable de celui-ci de l'une quelconque des revendications 1 à 7, dans lequel R^{F} est alkyle en C₁₋₆ à R^{N} substitué ou non substitué ou benzyle à R^{N} substitué ou non substitué.

9. Composé ou sel pharmaceutiquement acceptable de celui-ci de la revendication 8, dans lequel R^{N} est hydrogène, halogène, -OH, -CN, -CF₃, -CHF₂, -CH₂F, -C(CH₃)₂F, -C(CH₃)F₂, méthyle, éthyle, ou propyle.

10. Composé ou sel pharmaceutiquement acceptable de celui-ci de la revendication 8, dans lequel R^{F} est : 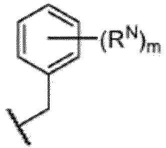
dans lequel R^{N} est halogène et m est 1 ou 2 ; ou
dans lequel R^{F} est : 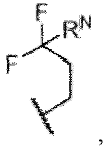
dans lequel R^{N} est F ou méthyle.

11. Composé ou sel pharmaceutiquement acceptable de celui-ci de l'une quelconque des revendications 1 à 10, dans lequel R^{A} est: 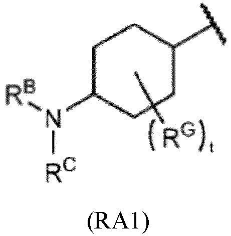 dans lequel :
R^{B} et R^{C} sont chacun indépendamment alkyle en C₁₋₆ à R^{N} substitué ou non substitué ; ou
R^{B} est hétérocycloalkyle à 4 membres et R^{C} est hydrogène ou alkyle en C₁₋₆ à R^{N} substitué ou non substitué.

12. Composé ou sel pharmaceutiquement acceptable de celui-ci de l'une quelconque des revendications 1 à 10, dans lequel R^{A} est :

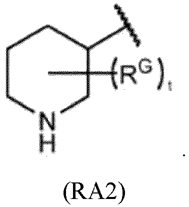

13. Composé ou sel pharmaceutiquement acceptable de celui-ci de l'une quelconque des revendications 1 à 12, dans lequel R^{G} est indépendamment halogène ou alkyle en C₁₋₆ à R^{M} substitué ou non substitué.

14. Composé ou sel pharmaceutiquement acceptable de celui-ci de l'une quelconque des revendications 1 à 13, dans lequel R^{G} est indépendamment fluoro et t est 1 ou 2.

15. Composé ou sel pharmaceutiquement acceptable de celui-ci de l'une quelconque des revendications 1 à 14, dans lequel R^{H} est halogène et j est 1.

16. Composé ou sel pharmaceutiquement acceptable de celui-ci de l'une quelconque des revendications 1 à 15, dans lequel l'anneau Q a la formule : 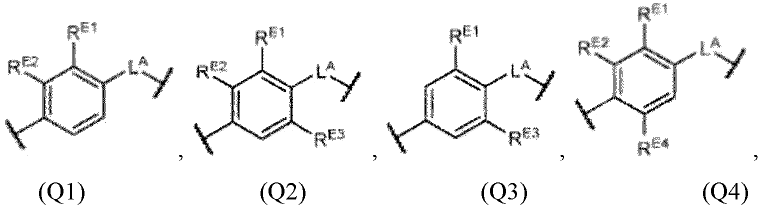 ou 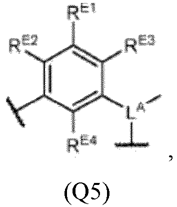 dans lequel R^{E1}, R^{E2}, R^{E3}, et R^{E4} sont chacun indépendamment hydrogène, halogène, ou alkyle en C₁₋₆ à R^{M} substitué ou non substitué.

17. Composé ou sel pharmaceutiquement acceptable de celui-ci de la revendication 1, sélectionné parmi le groupe constitué de
| N° de composé | Structure |
|---|---|
| 1001 | 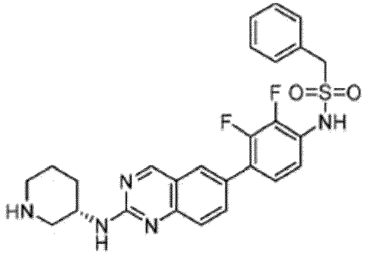 |
| 1002 | 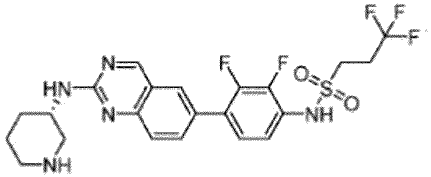 |
| 1003 | 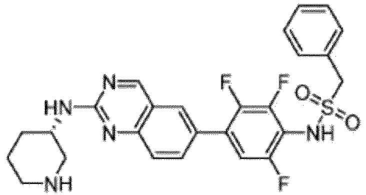 |
| 1004 | 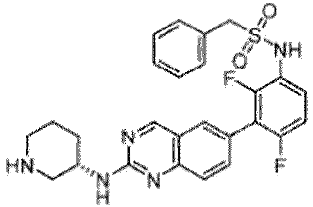 |
| 1005 | 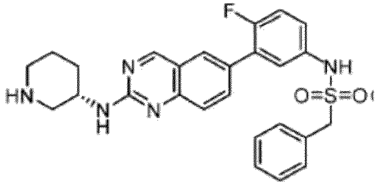 |
| 1006 | 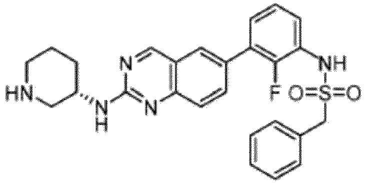 |
| 1007 | 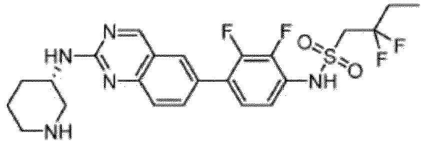 |
| 1008 | 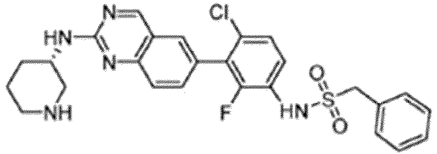 |
| 1009 | 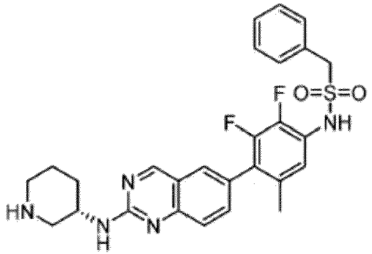 |
| 1010 | 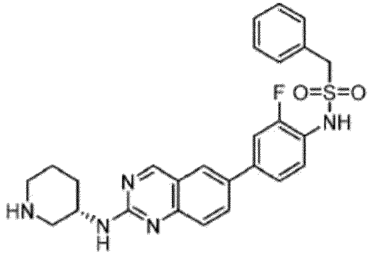 |
| 1011 | 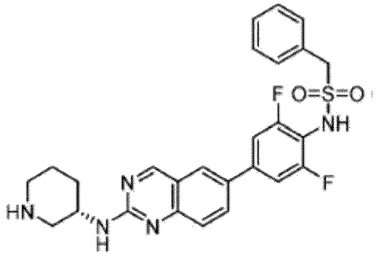 |
| 1012 | 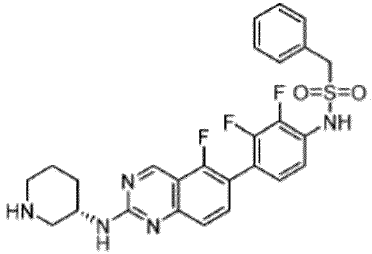 |
| 1013 | 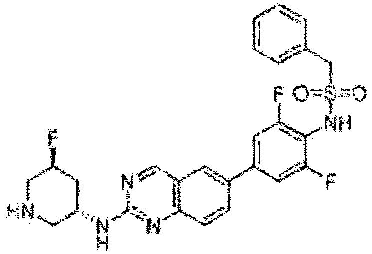 |
| 1014 | 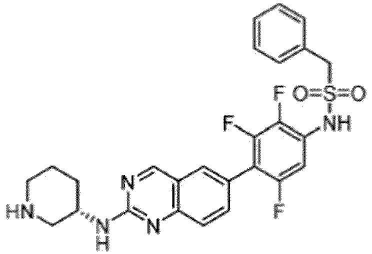 |
| 1015 | 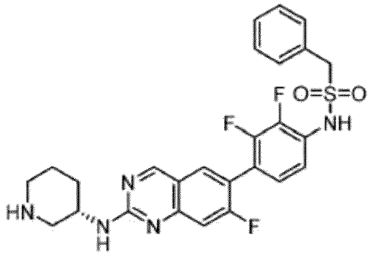 |
| 1016 | 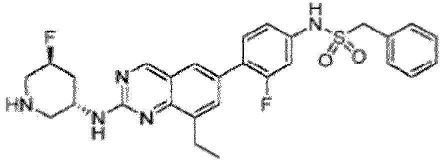 |
| 1017 | 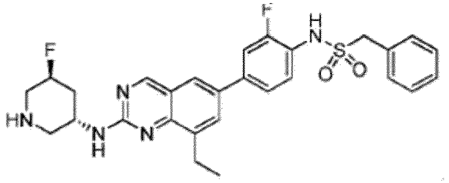 |
| 1018 | 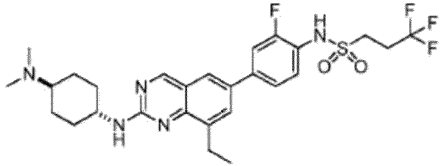 |
| 600 | 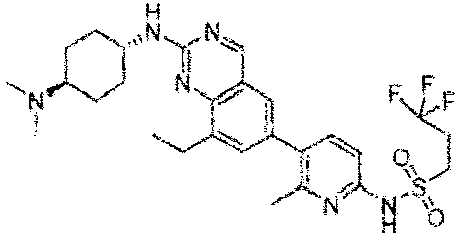 |
| 601 | 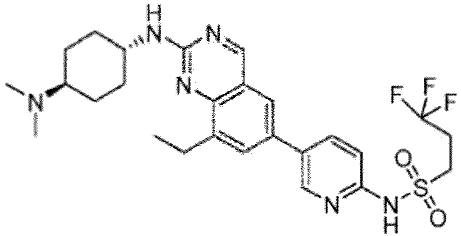 |
| 602 | 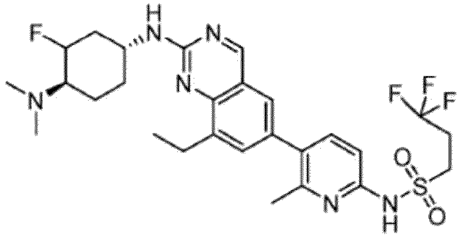 |
| 603 | 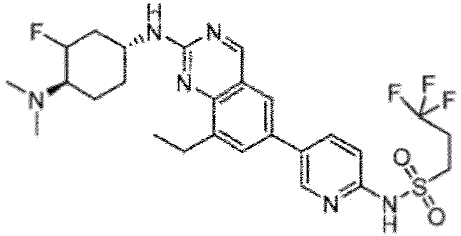 |
| 604 | 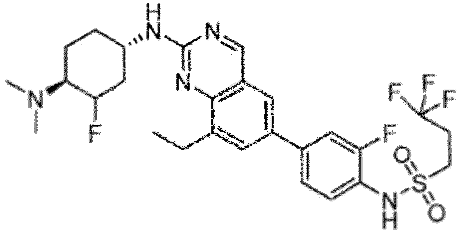 |
| 605 | 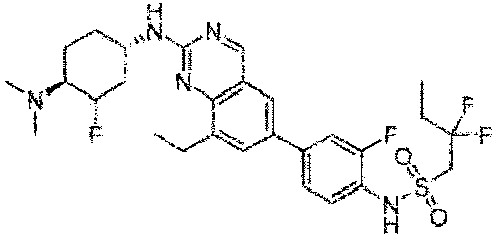 |
| 606 | 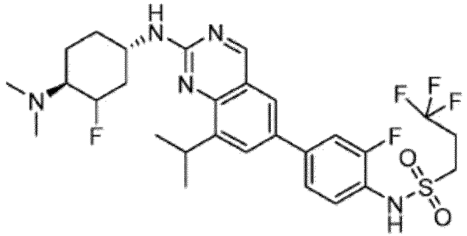 |
| 607 | 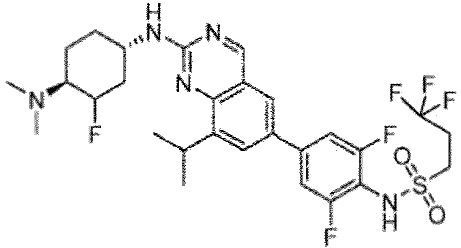 |
| 608 | 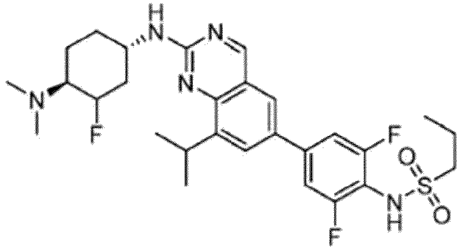 |
| 609 | 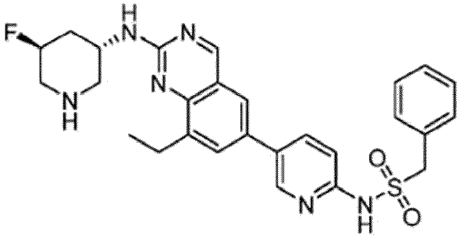 |
| 610 | 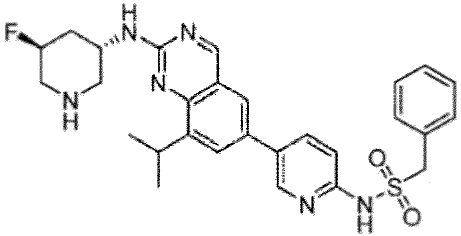 |
| 611 | 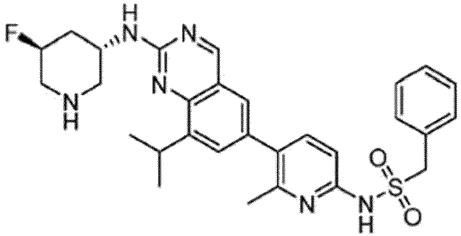 |
| 612 | 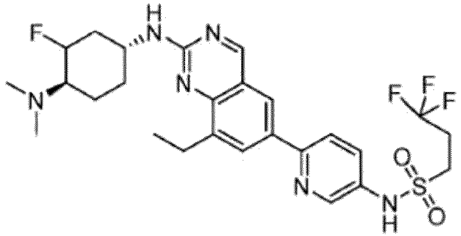 |
| 613 | 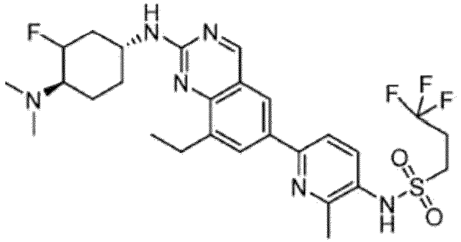 |
| 614 | 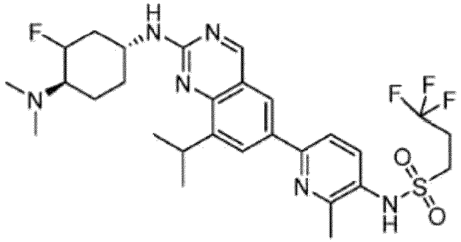 |
| 615 | 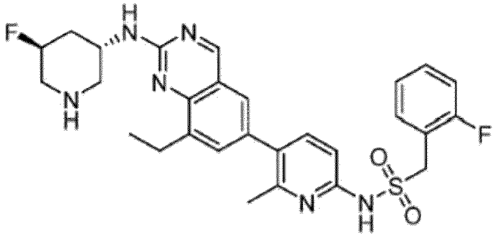 |
| 616 | 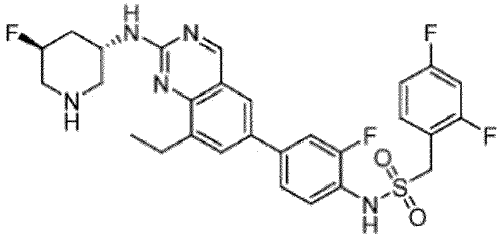 |
| 617 | 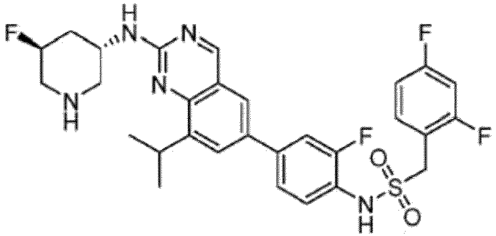 |
| 618 | 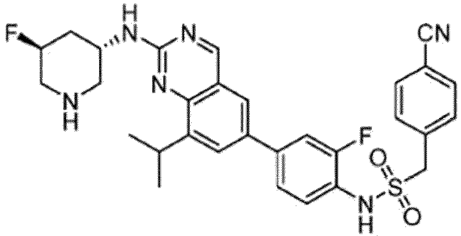 |
| 619 | 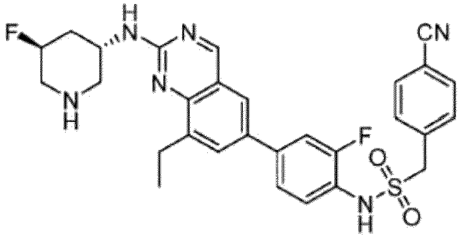 |
| 620 | 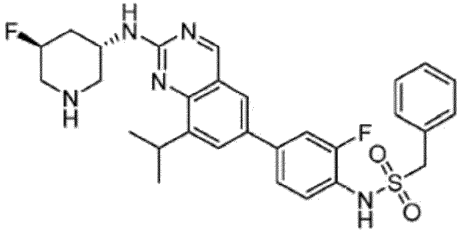 |
| 621 | 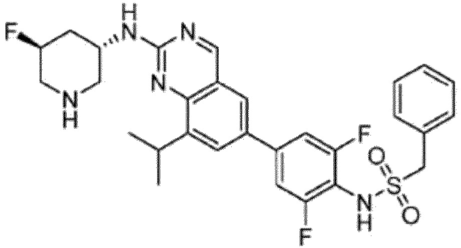 |
| 622 | 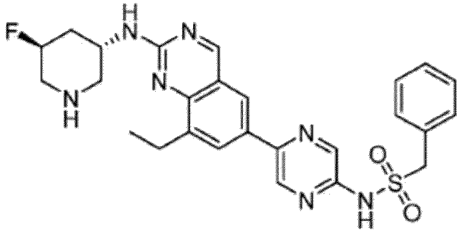 |
| 623 | 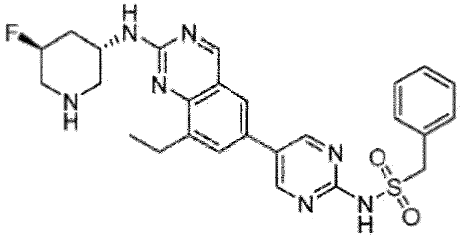 |
| 624 | 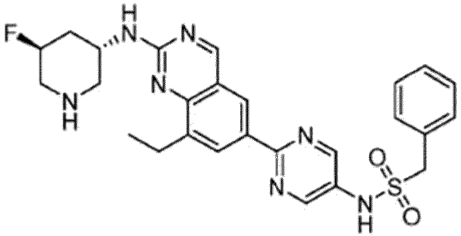 |
| 625 | 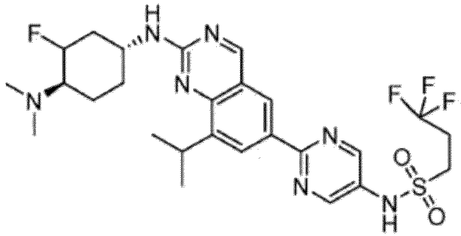 |
| 626 | 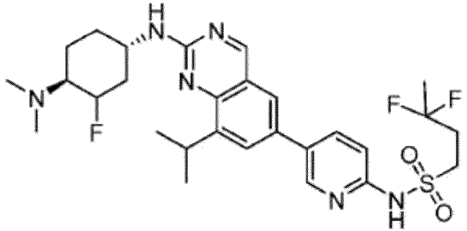 |
| 627 | 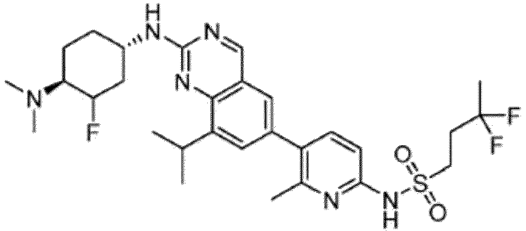 |
| 631 | 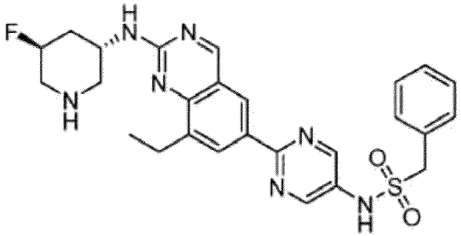 |
| 632 | 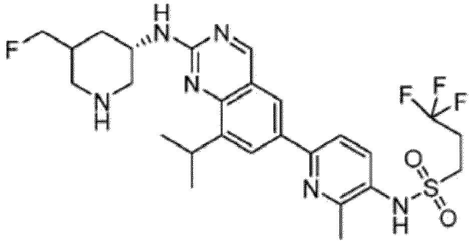 |
| 633 | 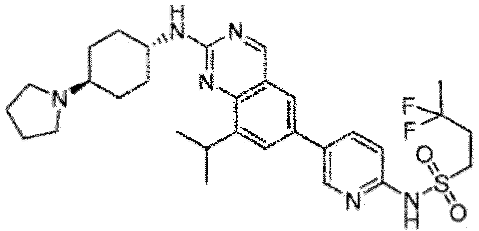 |
| 634 | 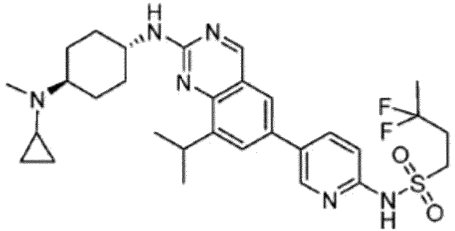 |
| 635 | 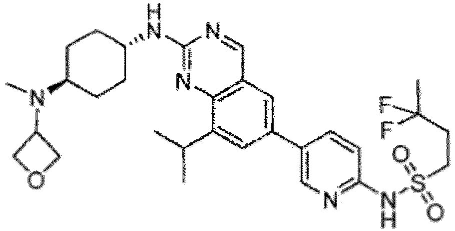 |
| 636 | 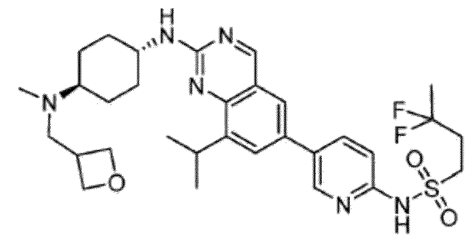 et |
| 639 | 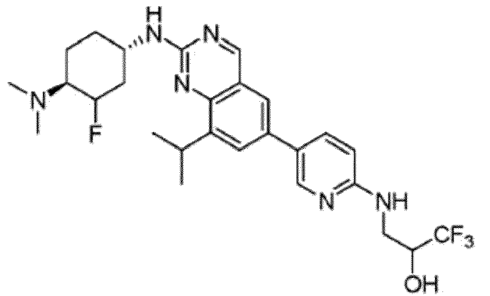 |

18. Composition pharmaceutique, comprenant un composé ou sel pharmaceutiquement acceptable de celui-ci de l'une quelconque des revendications 1 à 17 et un ou plusieurs excipients pharmaceutiquement acceptables.

19. Composé selon l'une quelconque des revendications 1 à 17 ou sel pharmaceutiquement acceptable de celui-ci, ou composition pharmaceutique de la revendication 18, pour utilisation dans un procédé pour traiter une maladie ou un trouble associé(e) à IRE1, dans lequel la maladie ou le trouble associé(e) à IRE1 est le cancer.
